# FASCICULE DE BREVET EUROPEEN

(11) **EP 2 185 561 B1**
(45) Date de publication et mention de la délivrance du brevet: **15.06.2011**
(21) Numéro de dépôt: 08836692.7
(22) Date de dépôt: 24.07.2008
(51) Int. Cl.: C07D 487/04, A61K 31/551, A61P 25/00, C07D 243/08, C07D 207/36, A61K 31/4985, C07D 241/06, C07D 241/08

(54) **Dérivés de 1,2,3,4-tétrahydropyrrolo(1,2-a)pyrazine-6-carboxamides et de 2,3,4,5-tétrahydropyrrolo(1,2-a)-diazépine-7-carboxamides, leur préparation et leur application en thérapeutique**
1,2,3,4-Tetrahydropyrrolo(1,2-a)pyrazin-6-carboxamid und 2,3,4,5-Tetrahydropyrrolo(1,2-a)-diazepin-7-carboxamid- Derivate, ihre Herstellung und therapeutische Verwendung
1,2,3,4-tetrahydropyrrolo(1,2-a)pyrazine-6-carboxamide and 2,3,4,5-tetrahydropyrrolo(1,2-a)-diazepine-7-carboxamide derivatives, preparation and therapeutic use thereof

(30) Priorité: 27.07.2007 FR 0705497; 27.07.2007 FR 0705498
(43) Date de publication de la demande: 19.05.2010
(73) Titulaire: Sanofi-Aventis, 75013 Paris (FR)
(72) Inventeur: BAUDOIN, Bernard, F-75013 Paris (FR); EVERS, Michel, F-75013 Paris (FR); GENEVOIS-BORELLA, Arielle, F-75013 Paris (FR); KARLSSON, Andreas, F-75013 Paris (FR); MALLERON, Jean-Luc, F-75013 Paris (FR); MATHIEU, Magali, F-75013 Paris (FR)
(74) Mandataire: Le Coupanec, Pascale A.M.P.
(86) Numéro de dépôt international: PCT/FR2008/001096
(87) Numéro de publication internationale: WO 2009/044007

(56) Documents cités:
- EP-A- 1 477 490
- WO-A-2004/094430
- WO-A-2005/058915
- WO-A-2006/103088

## Description

La présente invention se rapporte à des dérivés de 1,2,3,4-tétrahydropyrrolo-[1,2*-a*]-pyrazine-6-carboxamides et de 2,3,4,5-tétrahydropyrrolo[1,2*-a*][1,4]-diazépino-7-carboxamides, à leur préparation et à leur application en thérapeutique.

La présente de multiples plaques séniles dans le tissu cérébral est l'une des principales altérations histo-pathologiques observées dans la maladie d'Alzheimer; ces plaques se forment par dépôt d'agrégats fibrillaires d'un peptide de 4 kDa (40-42 amino-acides), appelé peptide amyloïde β (Aβ). La production et l'accumulation progressive de ce peptide pourraient jouer un rôle crucial dans le déclenchement et la progression de la pathologie d'Alzheimer, selon l'hypothèse d'une cascade amyloïde (D. Selko et al. Nature 399A (1999) 23 ; S. Roggo et al. Top. Med. Chem 2 (2002) 359 ; A. Ghosh et al. Curr. Med. Chem. 9 (2002) 1135).

Le peptide Aβ provient de la protéine APP (Amyloid Precursor Protein), qui peut être clivée par au moins trois activités protéolytiques différentes: 1) clivage dans la région Aβ par une activité α-secrétase (empêchant ainsi la formation de Aβ) ; 2) clivage à l'extrémité N-terminale de Aβ par une activité β-secrétase : 3) clivage à l'extrémité C-terminale de Aβ par une activité γ-secrétase. Le clivage consécutif de la protéine APP aux sites β et γ conduit à la formation du peptide Aβ (M. Citron Nat. Rev. Neurosci. 5 (2004) 677-685 ; D. Beher et al. Expert Opin. Invest. Drugs 14 (2005) 1385-1409).

Il existe donc un réel intérêt à trouver des composés inhibiteurs de la production du peptide Aβ (T. B. Durham et al. CuiT. Opin. Drug Disc. Dev. 9 (2006) 776-791).

WO 20041094430, WO 2006/103088 et WO 2005/058915 décrivent des dérivés hydroxyéthylamine utiles dans le traitement de la maladie d'Alzheimer.

EP 1 477 490 décrit des dérivés pyrrolopyrimidine utiles comme intermédiaires de synthèse de composés proposés pour le traitement de la maladie d'Alzheimer.

Il a maintenant été trouvé que des composés; dérivés de 1,2,3,4 tétrahydmpyrrolo-[1,2*-a*]-pyrazine-6-carbommides et de 2,3,4,5-tétrahydropyrrolo[1,2-*a*][1,4]diazépine-7-carboxamides possèdent une forte activité inhibitrice vis-à-vis de l'activité β-secrétase.

La présente invention a pour objet les composés répondant à la formule (I) 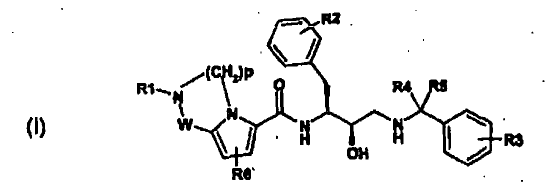 dans laquelle :
R1 représente un atome d'hydrogène, un groupe (C₁-C₁₀)alkyle, (C₃-C₇)cycloalkyle, (CH₂)ₙ-(C₁-C₆)alcényle, (CH₂)ₙ-(C₁-C₆)alcynyle, (C₁-C₆)alkyle-Z-(C₁-C₆)alkyle, dans lequel Z représente un hétéroatome choisi parmi O, N et S(O)ₘ, ou bien R1 représente un groupe COOR, S(O)ₘR, un aryle ou un aralkyle ; les groupes (C₁-C₁₀)alkyle, (C₃-C₇)cycloalkyle, (CH₂)ₙ-(C₁-C₆)alcényle, (CH₂)ₙ-(C₁-C₆)alcynyle, (C₁-C₆)alkyle-Z-(C₁-C₆)alkyle, aryle ou aralkyle étant éventuellement substitués par un ou plusieurs groupes choisis parmi un atome d'halogène, un groupe (C₁-C₆)alkyle, (C₃-C₇)cycloalkyle, halo(C₁-C₆)alkyle, (C₁-C₆)alcoxy, halo(C₁-C₆)alcoxy, NR7R8, nitro, cyano, OR, COOR, C(O)NR7R8, S(O)ₘNR7R8,
R2 représente un ou plusieurs groupes choisis parmi un atome d'hydrogène, un atome d'halogène, un groupe (C₁-C₆)alkyle, (C₃-C₇)cycloalkyle, (C₁-C₆)alcényle, (C₁-C₆)alcynyle, (C₁-C₆)alkyle-Z-(C₁-C₆)alkyle, dans lequel Z représente un hétéroatome choisi parmi O, N et S(O)ₘ, ou bien R2 représente un groupe halo(C₁-C₆)alkyle, halo(C₁-C₆)alcoxy, hydroxy, (C₁-C₆)alcoxy, nitro, cyano, amino, un groupe NR7R8, COOR, C(O)NR7R8, O-C(O)(C₁-C₆)alkyle, S(O)ₘ-NR7R8, un groupe aryle, le groupe aryle pouvant être éventuellement substitué par un ou plusieurs groupes choisis parmi un atome d'halogène, un groupe (C₁-C₆)alkyle, (C₃-C₇)cycloalkyle, halo(C₁-C₆)alkyle, (C₁-C₆)alcoxy, halo(C₁-C₆)alcoxy, NR7R8, OR, nitro, cyano, COOR, C(O)NR7R8, S(O)ₘNR7R8,
R3 représente un groupe trifluorométhyle,
R4 et R5 représentent un atome d'hydrogène, ou bien R4 et R5 forment avec l'atome de carbone qui les porte un cycle saturé contenant de 3 à 6 atomes de carbone et contenant éventuellement de 0 à 1 hétéroatome choisi parmi O, N ou S,
R6 représente un groupe choisi parmi un atome d'hydrogène, un atome d'halogène, un groupe (C₁-C₆)alkyle, (C₃-C₇)cycloalkyle, (C₃-C₇)cycloalkyle(C₁-C₆)alkyle, nitro, amino, un groupe NR7R8, COOR, un groupe aryle, un groupe NR7(SO₂)R8 ou C(O)NR7R8,
R, R7 et R8 représentent, indépendamment l'un de l'autre, un ou plusieurs groupes choisis parmi un atome d'hydrogène, un groupe (C₁-C₆)alkyle, (C₃-C₇)cycloalkyle, (C₃-C₇)cycloalkyle(C₁-C₆)alkyle, un groupe aryle, aryle(C₁-C₆)alkylène, ou bien R7 et R8 peuvent former avec l'atome qui les porte un cycle saturé, partiellement insaturé ou insaturé, contenant de 5 à 7 atomes de carbone et contenant éventuellement en plus un hétéroatome choisi parmi O, N ou S(O)ₘ,
W représente un groupe méthylène ou C(O),
m représente un nombre entier pouvant prendre les valeurs 0, 1 ou 2,
n représente un nombre entier pouvant prendre les valeurs 1, 2, 3, 4, 5 ou 6,
p représente un nombre entier pouvant prendre les valeurs 2 ou 3,
le carbone portant le groupe benzyle substitué par R2 est de configuration absolue S,
le carbone portant le groupe hydroxyle est de configuration absolue R, à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par :
- Cₜ₋C_{z}, où t et z peuvent prendre les valeurs de 1 à 10, une chaîne ou un cycle carboné pouvant avoir de t à z atomes de carbone, par exemple C₁-C₃ peut caractériser une chaîne carbonée ayant de 1 à 3 atomes de carbone;
- un atome d'halogène : un fluor, un chlore, un brome ou un iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe cycloalkyle : un groupe alkyle cyclique. A titre d'exemples, on peut citer les groupes cyclopropyle, méthylcyclopropyle, cyclobutyle, cyclopentyle, cyclohexyle, etc ;
- un groupe alkylène : un groupe aliphatique divalent saturé, linéaire ou ramifié. A titre d'exemple un groupe C₁₋₃-alkylène représente une chaîne carbonée divalente de 1 à 3 atomes de carbone, linéaire ou ramifiée, telle que un méthylènyle (-CH₂-), un éthylènyle (-CH₂CH₂-), un 1-méthyléthylènyle (-CH(CH₃)CH₂-), un propylènyle (-CH₂CH₂CH₂-);
- un groupe haloalkyle : un groupe alkyle dont un ou plusieurs atomes d'hydrogène ont été substitués par un atome d'halogène. A titre d'exemples, on peut citer les groupes CF₃, CH₂CF₃, CHF₂, CCl₃ ;
- un groupe haloalcoxy : un groupe alcoxy dont un ou plusieurs atomes d'hydrogène ont été substitués par un atome d'halogène. A titre d'exemples, on peut citer les groupes OCF₃, OCHF₂, OCCl₃;
- un groupe alcényle : un groupe aliphatique mono- ou poly-insaturé, linéaire ou ramifié, comprenant par exemple une ou deux insaturations éthyléniques;
- un groupe alcynyle : un groupe aliphatique mono- ou poly-insaturé, linéaire ou ramifié, comprenant par exemple une ou deux insaturations acétyléniques;
- un groupe alcoxy : un radical -O-alkyle où le groupe alkyle est tel que précédemment défini ;
- un groupe aryle : un groupe aromatique cyclique comprenant entre 6 et 14 atomes de carbone. A titre d'exemple de groupe aryle, on peut citer phényle ou naphthyle;
- les atomes de soufre et d'azote peuvent être présents à l'état oxydé (N-oxide, sulfoxide, sulfone).
- Lorsque p représente 2, les composés répondent à la formule suivante: 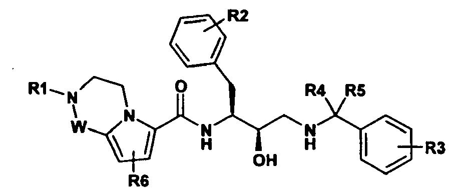
- Lorsque p représente 3, les composés repondent à la formule suivante:

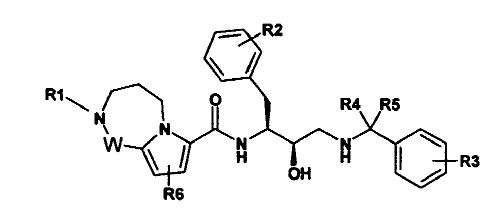

Dans les différents groupes tels que définis ci-dessous, les groupes R1, R2, R3, R4, R5, R6, R, R7, R8, W et p, lorsqu'ils ne sont pas définis, ont les mêmes définitions que celles mentionnées ci-dessus.

Parmi les composés de formule (I) objets de l'invention, un premier groupe de composés est constitué par les composés pour lesquels :
W représente un groupe méthylène.

Parmi les composés de formule (I) objets de l'invention, un second groupe de composés est constitué par les composés pour lesquels :
W représente un groupe C(O).

Parmi les composés de formule (I) objets de l'invention, un troisième groupe de composés est constitué par les composés pour lesquels :
p représente 2.

Parmi les composés de formule (I) objets de l'invention, un quatrième groupe de composés est constitué par les composés pour lesquels :
p représente 3.

Parmi les composés de formule (I) objets de l'invention, un cinquième groupe de composés est constitué par les composés pour lesquels :
R6 représente un groupe choisi parmi un atome d'hydrogène, un groupe COOR ou un groupe C(O)NR7R8.

Parmi ce sous-groupe de composés de formule (I) objets de l'invention, un sixième groupe de composés est constitué par les composés pour lesquels :
R6 représente un groupe choisi parmi un atome d'hydrogène, un groupe COOH, COOMe ou un groupe C(O)N(Et)₂.

Parmi les composés de formule (I) objets de l'invention, un septième groupe de composés est constitué par les composés pour lesquels :
W représente un groupe méthylène ou C(O),
p représente 2 ou 3,
R1 représente un atome d'hydrogène, un groupe (C₁-C₁₀)alkyle, COOR ou S(O)ₘR, le groupe (C₁-C₁₀)alkyle étant éventuellement substitué par un ou plusieurs groupes (C₁-C₆)alkyle,
R2 représente un ou plusieurs groupes choisis parmi un atome d'hydrogène ou un atome d'halogène,
R4 et R5 représentent un atome d'hydrogène ou forment avec l'atome de carbone qui les porte un groupe cyclopropyle,
R6 représente un groupe choisi parmi un atome d'hydrogène, un groupe COOR ou un groupe C(O)NR7R8,
R, R7 et R8 représentent, indépendamment l'un de l'autre, un atome d'hydrogène ou un ou plusieurs groupes (C₁-C₆)alkyle.

Les combinaisons des groupes un à sept tels que définis ci dessus font également partie de l'invention.

Parmi les composés de formule (I) objets de l'invention, on peut notamment citer les composés suivants :
- 6-{[(1*S*,2*R*)-1-benzyl-2-hydroxy-3-({1-[3-(trifluorométhyl) phényl]cyclopropyl}amino) propyl]carbamoyl}-3,4-dihydropyrrolo[1,2*-a*]pyrazine-2,8(1*H*)-dicarboxylate de 2-*tert-*butyle et de 8-méthyle
- Acide 6-{[(1*S*,2*R*)-1-benzyl-2-hydroxy-3-({1-[3-(trifluorométhyl)phényl]cyclopropyl} amino)propyl]carbamoyl}-2-(*tert*-butoxycarbonyl)-1,2, 3,4-tétrahydropyrrolo[1,2*-a*] pyrazine-8-carboxylique
- Chlorhydrate (2:1) d'acide 6-{[(1*S*,2*R*)-1-benzyl-2-hydroxy-3-({1-[3-(trifluorométhyl) phényl]cyclopropyl}amino)propyl]carbamoyl}-1,2,3,4-tétrahydropyrrolo [1,2*-a*]pyrazine-8-carboxylique
- 6-{[(1*S*,2*R*)-1-benzyl-2-hydroxy-3-({1-[3-(trifluorométhyl)phényl] cyclopropyl}amino) propyl]carbamoyl}-8-(diéthylcarbamoyl)-3,4-dihydropyrrolo[1,2*-a*]pyrazine-2(1*H*)-carboxylate de *tert*-butyle
- 6-{(1*S*,2*R*)-1-benzyl-2-hydroxy-3-[1-(3-trifluorométhylphényl)-cyclopropylamino]-propyl}-*N*-8,*N*-8-diéthyl-1 ,2,3,4-tétrahydropyrrolo[1,2*-a*]pyrazine-6,8-dicarboxamide et son chlorhydrate (2 :1)
- 6-{(1*S*,2*R*)-1-benzyl-2-hydroxy-3-[1-(3-trifluorométhylphényl)-cyclopropylamino]-propyl}-*N*-8,*N*-8-diéthyl-2-(méthylsulfonyl)-1,2,3,4-tétrahydropyrrolo[1,2*-a*]pyrazine-6,8-dicarboxamide
- *N*-[(1*S*,2*R*)-1-(3,5-difluorobenzyl)-2-hydroxy-3-({1-[3-(trifluorométhyl)phényl]cyclopropyl} amino)propyl]-1-oxo-2-(1-propylbutyl)-1,2,3,4-tétrahydropyrrolo[1,2*-a*]pyrazine-6-carboxamide et son chlorhydrate (1 :1)
- *N*-[(1*S*,2R)-1-benzyl-2-hydroxy-3-({1-[3-(trifluorométhyl)phényl]cyclopropyl}amino) propyl]-1-oxo-2-(1-propylbutyl)-1,2,3,4-tétrahydropyrrolo[1,2*-a*]pyrazine-6-carboxamide et son chlorhydrate (1 :1)
- *N*-[(1*S*,2*R*)-1-benzyl-2-hydroxy-3-{[3-(trifluorométhyl)benzyl]amino}propyl]-1-oxo-2-(1-propylbuty()-1,2,3,4-tétrahydropyrrolo[1,2*-a*]pyrazine-6-carboxamide et son chlorhydrate (1 : 1)
- *N*-[(1*S*,2*R*)-1-benzyl-2-hydroxy-3-{[3-(trifluorométhyl)benzyl]amino}propyl]-2-(1-éthyl propyl)-1-oxo-1,2,3,4-tétrahydropyrrolo[1,2*-a*]pyrazine-6-carboxamide et son chlorhydrate (1 :1).
- *N*-[(1*S*,2*R*)-1-benzyl-2-hydroxy-3-{[3-(trifluorométhyl)benzyl]amino}propyl]-1-oxo-2-(1-propylbutyl)-2,3,4,5-tétrahydro-1H-pyrrolo[1,2*-a*][1,4]diazépine-7-carboxamide et son chlorhydrate (1 :1).

L'invention a également pour objet un procédé de préparation des composés de l'invention de formule (I).

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Dans les schémas qui suivent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les abréviations et symboles utilisés pour la description des procédés de synthèse et pour la description des composés sont les suivants:
- BOC pour *tert*-butoxycarboxylate,
- DCC pour dicyclohexylcarbodiimide,
- DMF pour diméthylformamide,
- EDCI pour (1-ethyl-3,3-dimethylaminopropyl)carbodiimide,
- NMP pour N-méthyl-2-pyrrolidone,
- PyBOP pour hexafluorophosphate de benzotriazol-1-yloxytripyrrolidinophosphonium,
- THF pour tétrahydrofurane.

Conformément à l'invention, on peut préparer les composés de formule générale (I) selon le procédé illustré par le schéma 1 qui suit.

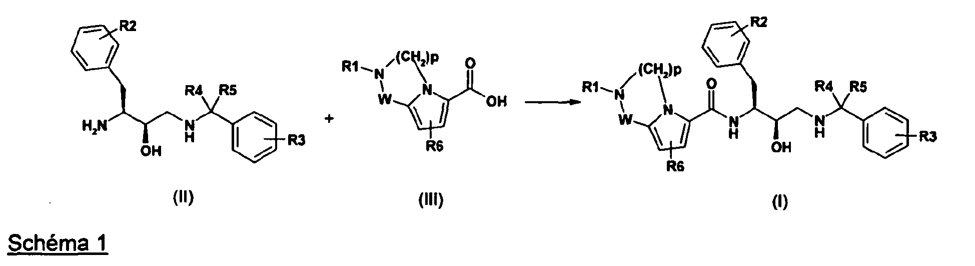

Le composé de formule générale (I) peut être préparé par condensation de la fonction amine du composé de formule générale (II), dans laquelle R2, R3, R4 et R5 sont tels que définis dans la formule générale (I), sur la fonction acide carboxylique du composé de formule générale (III), dans laquelle p, R1 et R6 sont tels que définis dans la formule générale (I). Cette réaction est opérée en milieu anhydre, de préférence inerte (azote ou argon par exemple) et en utilisant des agents classiques de couplage d'une fonction acide avec une fonction amine tels que la DCC, le PyBOP, l'EDCI, dans des solvants tels que le dichlorométhane, le THF, l'éther ou le chloroforme à une température comprise entre 20°C et la température de reflux du solvant.

Le composé de formule générale (II), dans laquelle R2, R3, R4 et R5 sont tels que définis dans la formule générale (I), peut être préparé à partir du composé de formule générale (IV), dans laquelle R2, R3, R4 et R5 sont tels que définis dans la formule générale (I), par déprotection de l'amine primaire par action d'un acide (acide chlorhydrique par exemple) en solution dans un solvant ou un mélange de solvant éthéré (diéthyléther par exemple) et/ou chloré (dichlorométhane par exemple), selon le procédé illustré par le schéma 2 qui suit.

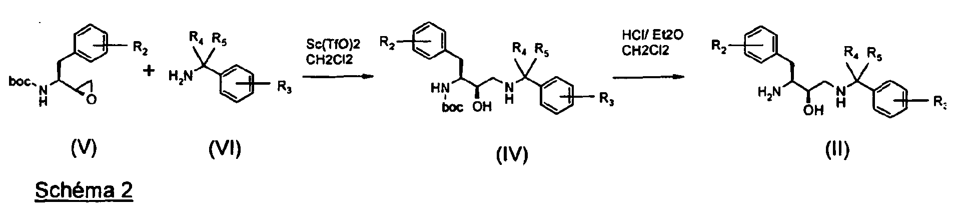

Le composé de formule générale (IV), dans laquelle R2, R3, R4 et R5 sont tels que précédemment décrits, peut être préparé par mise en réaction d'un dérivé de benzylamine de formule générale (VI), dans laquelle R3, R4 et R5 sont tels que définis dans la formule générale (I) avec un oxirane de formule générale (V), dans laquelle R2 est tel que défini dans la formule générale (I) en opérant en milieu anhydre, de préférence inerte (azote ou argon par exemple), dans un solvant chloré (dichlorométhane par exemple) et en présence d'un acide de Lewis comme par exemple le triflate de scandium.

Les composés de formule générale (I) dans laquelle W représente C(O) et p représente 2, peuvent être obtenus par condensation de la fonction amine du composé de formule générale (II) sur la fonction acide carboxylique du composé de formule générale (IIIa), dans laquelle R1 et R6 sont tels que précédemment décrits.

Les composés de formule générale (IIIa) peuvent être préparés selon le procédé du schéma 3 qui suit, à partir du composé de formule générale (VII), dans laquelle R1 et R6 sont tels que définis précédemment, par mise en réaction du composé de formule générale générale (VII) avec du monoxyde de carbone (sous pression de 1 à 20 atmosphères), en présence d'ions acétate (de potassium ou de sodium), et un iodure alcalin (iodure de sodium ou de potassium par exemple), en présence d'un catalyseur au palladium (acétate de palladium par exemple), d'une phosphine (triphénylphosphine par exemple) en solution dans un solvant organique (diméthylformamide par exemple) et en présence d'eau. La réaction a lieu à une température comprise entre 20°C et celle du reflux du solvant.

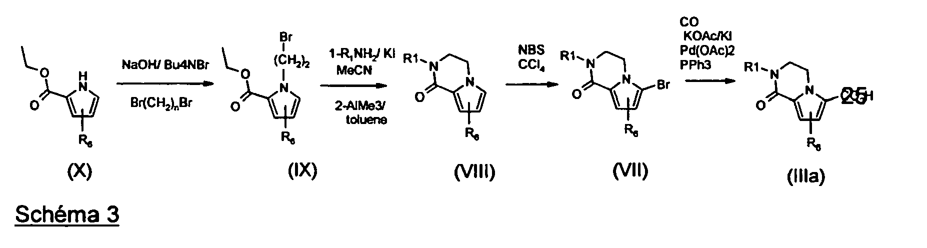

Le dérivé halogéné de formule générale (VII), dans laquelle R1 et R6 sont tels que précédemment décrits, peut être préparé à partir du dérivé bicyclique de formule générale (VIII), par réaction avec un agent d'halogénation telle la *N*-bromosuccinimide dans un solvant chloré (tétrachlorure de carbone par exemple).

Le dérivé bicyclique de formule générale (VIII) peut être préparé par aminolyse du composé de formule générale de formule (IX) avec une amine de formule générale R₁NH₂. Cette réaction est réalisée en présence ou non d'ions iodures (iodure de potassium ou de sodium par exemple) et en solution dans un solvant organique (acétonitrile, dimethylformamide, éthanol, NMP, éther, THF, dioxane, toluène par exemple), à une température comprise entre 20°C et la température de reflux du solvant. Le dérivé aminé obtenu intermédiairement est ensuite cyclisé en présence d'un agent de cyclisation (le triméthylaluminium par exemple) en solution dans un solvant organique (toluène par exemple) à une température comprise entre 20°C et la température de reflux du solvant.

Le composé de formule générale (IX) peut être préparé à partir du dérivé du pyrrole-2-carboxylate d'éthyle de formule générale (X), par une réaction de monoalkylation, par action du 1,2-dibromoéthane. Cette alkylation est réalisée avec une base telle que le carbonate de césium, le carbonate de potassium, le phosphate de potassium, le t-butylate de potassium, la soude, l'hydrure de sodium et dans des solvants tels que l'éthanol, le DMF, la NMP, l'éther, le THF, le dioxane, l'acétonitrile, le toluène ou par transfert de phase en présence d'un agent tel que le chlorure de tétrabutyl ammonium dans le dichlorométhane ou le dichloroéthane en présence d'eau, ou encore dans le benzène.

Les composés de formule (I) dans laquelle W représente un groupe méthylène et p représente 2, peuvent être obtenus par condensation de la fonction amine du composé de formule générale (II) sur la fonction acide carboxylique du composé de formule générale (IIIb), selon le procédé du schéma 4 qui suit.

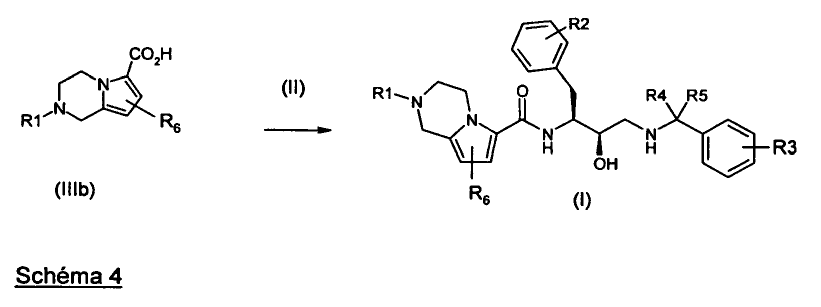

Parmi les composés de formule générale (IIIb), le composé de formule (IIIb'), dans laquelle R6 représente COOR, R étant tel que défini précédemment, est particulièrement intéressant pour la synthèse des composés de formule (I).

Ce composé de formule (IIIb') (acide 2-(*tert*-butoxycarbonyl)-8-(méthoxycarbonyl)-1,2,3,4-tétrahydropyrrolo[1,2*-a*]pyrazine-6-carboxylique) peut être synthétisé suivant le procédé du schéma 5 qui suit, à partir du 6-bromo-3,4-dihydropyrrolo[1,2a]pyrazine2,8(1H)-dicarboxylate de 2-*tert*-butyle et de 8-méthyle, dans les mêmes conditions que celles utilisées pour la préparation du composé de formule générale (IIIa).

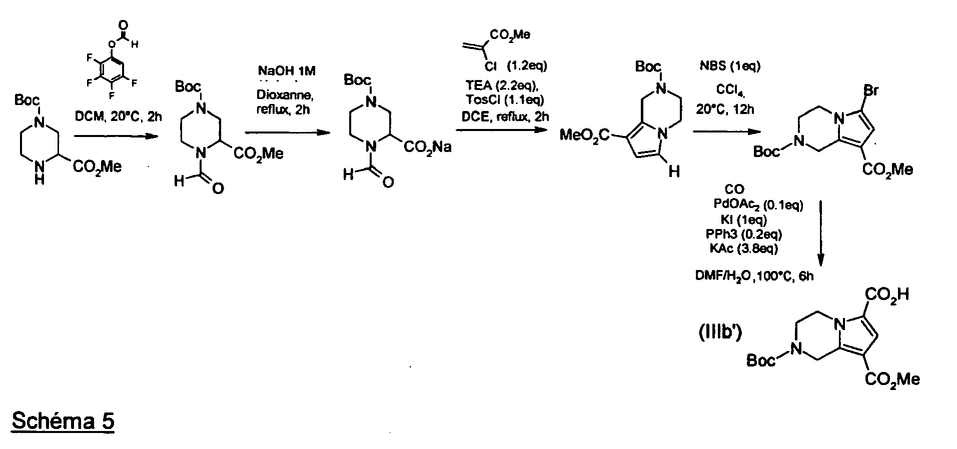

Le 6-bromo-3,4-dihydropyrrolo[1,2*a*]pyrazine2,8(1H)-dicarboxylate de 2-*tert*-butyle et de 8-méthyle peut être préparé par une réaction d'halogénation du 3,4-dihydropyrrolo[1,2*a*]pyrazine-2,8-(1H)-dicarboxylate de 2-*tert* butyle et de 8-méthyle, avec un agent d'halogénation telle que la N-bromosuccinimide, dans un solvant chloré (tétrachlorure de carbone par exemple) à une température comprise entre 0°C et la température de reflux du solvant.

Le 3,4-dihydropyrrolo[1,2*-a*]pyrazine-2,8-(1H)-dicarboxylate de 2-*tert-*butyle et de 8-méthyle peut être préparé à partir du 4-(*tert*-butoxycarbonyl)-1-formylpiperazine-2-carboxylate de sodium, mis en réaction avec un mélange de chlorure de tosyle, et d'α-chloroacrylate de méthyle en présence d'une base organique telle que la triéthylamine ou la pyridine, dans un solvant chloré tel le dichlorométhane ou le dichloroéthane à une température comprise entre 20°C et la température de reflux du solvant.

Le 4-(*tert*-butoxycarbonyl)-1-formylpiperazine-2-carboxylate de sodium peut être préparé à partir de la 2-méthoxycarbonyl-4-N-*tert*-butyle piperazine commerciale, par une réaction de formylation par le formiate de pentafluorophényle (qui est préparé extemporanément selon L. Kisfaludy et al. Synthesis (1987) 5, 510) en opérant en milieu anhydre, de préférence inerte (azote ou argon par exemple) dans un solvant organique chloré (dichlorométhane par exemple) à une température proche de 20°C, suivie d'une réaction de salification par action d'une base telle que la soude, le carbonate de potassium, le *tert*-butylate de potassium dans des solvants tels que le THF ou le dioxanne, à une température proche de 20°C.

Les composés de formule générale (I) dans laquelle W représente un groupe CO et p représente 3, peuvent être obtenus par condensation de la fonction amine du composé de formule générale (II) sur la fonction acide carboxylique du composé de formule générale (IIIc), celui-ci étant obtenu selon le procédé du schéma 6 qui suit.

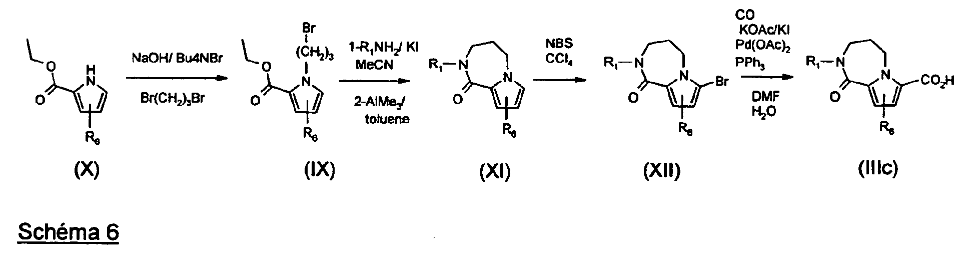

Les composés de formule générale (IIIc) peuvent être préparés par mise en réaction du composé de formule générale (XII) avec du monoxyde de carbone, en présence d'ions acétate (de potassium ou de sodium), et d'un iodure alcalin (iodure de sodium ou de potassium par exemple), en présence d'un catalyseur au palladium (acétate de palladium par exemple), d'une phosphine (triphénylphosphine par exemple) en solution dans un solvant organique (diméthylformamide par exemple) et en présence d'eau. La réaction a lieu à une température comprise entre 20°C et celle du reflux du solvant.

Le dérivé halogéné de formule générale (XII), dans laquelle R1 et R6 sont tels que précédemment décrits, peut être préparé à partir du dérivé de formule générale (XI) par réaction avec un agent d'halogénation telle la *N*-bromosuccinimide dans un solvant chloré (tétrachlorure de carbone par exemple).

Le dérivé bicyclique de formule générale (XI) peut être préparé par aminolyse du composé de formule générale de formule (IX) avec une amine de formule générale R1-NH₂. Cette réaction est réalisée en présence ou non d'ions iodures (iodure de potassium ou de sodium par exemple) et en solution dans un solvant organique (acétonitrile, dimethylformamide, éthanol, NMP, éther, THF, dioxane, toluène par exemple), à une température comprise entre 20°C et la température de reflux du solvant. Le dérivé aminé obtenu intermédiairement est ensuite cyclisé en présence d'un agent de cyclisation (le triméthylaluminium par exemple) en solution dans un solvant organique (toluène par exemple) à une température comprise entre 20°C et la température de reflux du solvant. Le composé de formule générale (IX) peut être préparé à partir du dérivé du pyrrole-2-carboxylate d'éthyle de formule générale (X), par une réaction de monoalkylation, par action du 1,3-dibromopropane. Cette alkylation est réalisée avec une base telle que le carbonate de césium, le carbonate de potassium, le phosphate de potassium, le t-butylate de potassium, la soude, l'hydrure de sodium et dans des solvants tels que l'éthanol, le DMF, la NMP, l'éther, le THF, le dioxane, l'acétonitrile, le toluène ou par transfert de phase en présence d'un agent tel que le chlorure de tétrabutyl ammonium dans le dichlorométhane ou le dichloroéthane en présence d'eau, ou encore dans le benzène.

Les produits de formule (I), peuvent être soumis, si désiré et si nécessaire, pour obtenir des produits de formule (I) ou être transformés en d'autres produits de formule (I), à l'une ou plusieurs des réactions de transformations suivantes, dans un ordre quelconque :
a) une réaction d'estérification ou d'amidification de fonction acide,
b) une réaction d'hydrolyse de fonction ester en fonction acide,
c) une réaction de transformation de fonction hydroxyle en fonction alcoxy,
d) une réaction d'oxydation de fonction alcool en fonction aldéhyde, cétone ou acide,
e) une réaction de réduction de fonction acide, aldéhyde ou cétone en fonction alcool,
f) une réaction d'amination réductrice d'une fonction aldéhyde ou cétone,
g) une réaction d'oxydation de groupe alcényle en fonction aldéhyde ou cétone,
h) une réaction d'oxydation d'un thioéther en sulfone ou sulfoxyde,
i) une réaction d'alkylation d'un sulfonamide,
j) une réaction de déshydratation de groupe hydroxyalkyle en groupe alcényle,
k) une réaction de déshydrohalogénation d'un dérivé halogéné,
l) une réaction d'hydrogénation totale ou partielle de groupe alcényle ou alcynyle en groupe alcényle ou alkyle,
m) une réaction de couplage catalytique d'un dérivé halogéné et d'un dérivé organométallique tel que stannique ou boronique pour introduire un substituant alkyle, alcènyle, alcynyle ou aryle,
n) une réaction de protection des fonctions réactives,
o) une réaction d'élimination des groupements protecteurs que peuvent porter les fonctions réactives protégées,
p) une réaction de salification par un acide minéral ou organique ou par une base pour obtenir le sel correspondant,
q) une réaction de dédoublement des formes racémiques en énantiomères, lesdits produits de formule (I) ainsi obtenus étant le cas échéant sous toutes les formes isomères possibles racémiques, énantiomères et diastéréoisomères,
r) une réaction de réduction de dérivés nitro en dérivés nitroso ou amino,
s) une réaction de mono- ou de di-alkylation d'une fonction amine,
t) une réaction de sulfonylation d'une amine primaire ou secondaire,
u) une réaction d'acylation d'une fonction amine.

L'invention, selon un autre de ses aspects, a également pour objet les composés de formules (IIIa), (IIIb), (IIIb') et (IIIc). Ces composés sont utiles comme intermédiaires de synthèse des composés de formule (I).

Les composés de formule (I) peuvent être purifiés par les méthodes connues de l'homme du métier, par exemple par cristallisation, chromatographie ou extraction.

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés selon l'invention.

Les spectres de résonnance magnétique nucléaire du proton (RMN ¹H) ont été effectués à 250 MHz, 300 MHz, 400 MHz ou 500 MHz sur des appareils Brüker (déplacements chimiques (δ en ppm) - dans le solvant diméthylsulfoxyde - d6 (DMSO-d6) référencé à 2,50 ppm à la température de 303K). Les abréviations utilisées pour caractériser les signaux sont les suivantes: s = singulet, m = multiplet, d = doublet, t = triplet, q = quadruplet.

La nomenclature des composés ci-après exemplifiés a été établie à l'aide du logiciel ACDLabs^{®} version 10.0.

### Exemple 1 :

### 6-{[(1S,2R)-1-Benzyl-2-hydroxy-3-({1-[3-(trifluorométhyl)phényl]cyclopropyl}amino) propyl]carbamoyl}-3,4-dihydropyrrolo[1,2-a]pyrazine-2,8(1H)-dicarboxylate de 2-tert-butyle et de 8-méthyle

La préparation du formiate de pentafluorophényle est décrite dans la littérature (Lajos Kisfaludy et al, Synthesis 1987, 5, 510).

### 1.1 : 4-Formylpipérazine-1,3-dicarboxylate de 1-tert butyle et de 3-méthyle

10 g de 2-méthoxycarbonyl-4-*N*-*tert*-butyle-pipérazine sont dissous dans 25 cm³ de dichlorométhane sous atmosphère inerte à une température proche de 20°C. La solution de formiate de pentafluorophényle obtenue dans l'étape précédente est ajoutée goutte à goutte à une température proche de 20°C. L'agitation est maintenue 1 h30 après la fin de l'addition. Le mélange réactionnel est concentré à l'évaporateur rotatif sous pression réduite (5 kPa). L'huile jaune-orangée obtenue est purifiée par chromatographie flash sur une cartouche de silice (colonne : 700g ; granulométrie : 40-60µm ; débit : 80 cm³/min ; éluant : cyclohexane 30%- acétate d'éthyle 70%). Après concentration des fractions sous pression réduite (5 kPa), on obtient 10,5 g de 4-formylpipérazine-1,3-dicarboxylate de 1-*tert*-butyle et de 3-méthyle sous forme d'une huile jaune pâle.
- RMN : pour ce lot, nous observons un dédoublement 50 % -50 % de rotamères avec : 1,38 (s, 4,5H) ; 1,39 (s, 4,5H) ; de 2,62 à 2,93 (m, 1,5H) ; 3,08 (m, 1H) ; 3,26 (m partiellement masqué, 0,5H) ; 3,64 (m, partiellement masqué, 0,5H) ; 3,68 (s, 1,5H) ; 3,69 (s, 1,5H) ; 3,90 (m, 1H) ; 4,02 (m, 0,5H) ; 4,36 (d large, J = 13,5 Hz, 0,5H) ; 4,42 (d large, J = 13,5 Hz, 0,5H) ; 4,71 (d large, J = 4,5 Hz, 0,5H) ; 4,89 (d large, J = 4,5 Hz, 0,5H) ; 8,09 (s, 0,5H) ; 8,16 (s, 0,5H).

### 1.2 : 4-(tert-Butoxycarbonyl)-1-formylpipérazine-2-carboxylate de sodium

10,5 g de 4-formylpipérazine-1,3-dicarboxylate de 1-*tert*-butyle et de 3-méthyle sont dissous dans 200 cm³ de dioxanne à une température proche de 20°C. 42 cm³ d'une solution de soude 1 M sont ajoutés, puis le mélange réactionnel est chauffé au reflux du solvant pendant 1 h30. Le mélange réactionnel est concentré à l'évaporateur rotatif sous pression réduite (5 kPa). Le résidu solide beige obtenu est trituré dans l'acétate d'éthyle, puis filtré, rincé et séché à l'air. On obtient 10,5 g de 4-(*tert*-butoxycarbonyl)-1-formylpipérazine-2-carboxylate de sodium sous forme d'un solide blanc amorphe.
- LC-MS-DAD-ELSD: 259⁽⁺⁾ = (M+H)⁽⁺⁾
- RMN : 8.08 (d, 1H) ; 4.74 (d, 0.5H) ; 4.55 (m, 1H) ; 4.39 (d, 0.5H) ; 4.00 (dd, 1H) ; 3.62 (d, 0.5H) ; 3.43 (t, 0.5H) ; 3.00 (m, 3H) ; 1.40 (s, 9H).

### 1.3: 3,4-dihydropyrrolo[1,2-a]pyrazine-2,8(1H)-dicarboxylate de 2-tert-butyle et de 8-méthyle

10,5 g de 4-(*tert*-butoxycarbonyl)-1-formylpipérazine-2-carboxylate de sodium sont dissous dans 300 cm³ de dichloroéthane à une température proche de 20°C. 8,5 g de chlorure de tosyle, 5,3 cm³ de α-chloroacrylate de méthyle et 12,6 cm³ de triéthylamine sont ajoutés. Le mélange réactionnel est chauffé au reflux du solvant pendant 1 h. Le mélange réactionnel est refroidi à une température proche de 20°C. Il est ensuite lavé successivement avec 2 fois 100 cm³ d'eau et avec 2 fois 100 cm³ d'une solution aqueuse saturée en chlorure de sodium. Les phases aqueuses sont réextraites au dichlorométhane. Les phases organiques sont réunies, séchées sur filtre séparateur de phases, puis concentrées à l'évaporateur rotatif sous pression réduite (5 kPa). L'huile brune-orangée obtenue est purifiée par chromatographie flash sur une cartouche de silice (colonne : 700 g ; granulométrie : 40-60 µm; débit: 80 cm³/min ; éluant : cyclohexane 80%- acétate d'éthyle 20%). Après concentration des fractions sous pression réduite (5 kPa), on obtient 9,5 g de 3,4-dihydropyrrolo[1,2*-a*]pyrazine-2,8(1*H*)-dicarboxylate de 2-*tert*-butyle et de 8-méthyle sous forme d'un solide jaune pâle.
- RMN (ppm) : 6.77 (d, 1H) ; 6.42 (d, 1H) ; 4.73 (s, 2H) ; 3.98 (t, 2H) ; 3.72 (t, 2H) ; 3.70 (s, 3H) ; 1.43 (s, 9H)
- LC-MS-DAD-ELSD: 281 (+) = (M+H)⁽⁺⁾
- PF: 100°C

### 1.4: 6-Bromo-3,4-dihydropyrrolo[1,2-a]pyrazine-2,8(1H)-dicarboxylate de 2-tert butyle et de 8-méthyle

9,5 g de 3,4-dihydropyrrolo[1,2*-a*]pyrazine-2,8(1*H*)-dicarboxylate de 2-*tert*-butyle et de 8-méthyle sont dissous dans 1 dm³ de tétrachlorure de carbone à une température proche de 20°C. Le mélange réactionnel est refroidi à 4°C à l'aide d'un bain eau-glace et 6 g de *N*-bromosuccinimide sont ajoutés en deux fois à 5 mn d'intervalle. L'agitation est maintenue 18 h pendant lesquelles le milieu réactionnel remonte progressivement à une température proche de 20°C. 6 g d'hydrogénocarbonate de sodium et 6 g de sulfate de sodium dissous dans 150 cm³ d'eau sont ajoutés au mélange réactionnel (pH 9). La phase organique est lavée successivement avec 150 cm³ d'eau puis avec 150 cm³ d'une solution aqueuse saturée en chlorure de sodium. Les phases aqueuses sont réextraites au tétrachlorure de carbone. Les phases organiques sont réunies, séchées sur filtre séparateur de phases puis concentrées à l'évaporateur rotatif sous pression réduite (5 kPa). L'huile jaune obtenue est triturée dans le cyclohexane. Le dérivé bromé qui a précipité est filtré et rincé au cyclohexane. On obtient 8,6 g de 6-bromo-3,4-dihydropyrrolo[1,2*-a*]pyrazine-2,8(1*H*)-dicarboxytate de 2-*tert*-butyle et de 8-méthyle sous forme d'un solide jaune pâle.
- RMN: 6.57 (s, 1H) ; 4.72 (s, 2H) ; 3.88 (t, 2H) ; 3.76 (t, 2H) ; 3.71 (s, 3H) ; 1.43 (s, 9H)
- LC-MS-DAD-ELSD: 359⁽⁺⁾⁷⁹Br = (M+H)⁽⁺⁾, 381⁽⁺⁾⁷⁹Br = (M+Na)⁽⁺⁾
- PF: 109°C

### 1.5: Acide 2-(tert-butoxycarbonyl)-8-(méthoxycarbonyl)-1,2,3,4-tétrahydropyrrolo[1,2-a]pyrazine-6-carboxylique

8,6 g de 6-bromo-3,4-dihydropyrrolo[1,2*-a*]pyrazine-2,8(1*H*)-dicarboxylate de 2-*tert* butyle et de 8-méthyle sont dissous dans 320 cm³ de diméthylformamide à une température proche de 20°C. 9 g d'acétate de potassium et 4,2 g d'iodure de potassium sont ajoutés, puis !e mélange réactionnel est purgé au monoxyde de carbone. Sous atmosphère de monoxyde de carbone, 20 cm³ d'eau sont ajoutés ainsi que 538 mg de d'acétate de palladium et 1,3 g de triphénylphosphine. Le mélange réactionnel est soumis à un bullage de monoxyde de carbone puis est chauffé à 100°C pendant 6 h. Il est ensuite concentré à l'évaporateur rotatif sous pression réduite (5 kPa). Le résidu solide obtenu est repris dans 300 cm³ d'acétate d'éthyle et 220 cm³ de soude sont ajoutés, respectivement 200 cm³ de 1 M et 20 cm³ de 5 M (pH 12). Après filtration du palladium et décantation du filtrat, l'acide est précipité par addition de 70 cm³ d'acide chlorhydrique 5 M à la phase aqueuse. Le solide est filtré et séché à l'air. On obtient 6,9 g d'acide 2-(*tert* butoxycarbonyl)-8-(méthoxycarbonyl)-1,2,3,4-tétrahydropyrrolo[1,2*-a*]pyrazine-6-carboxylique sous forme d'un solide blanc.
- RMN: 7.09 (s, 1H) ; 4.79 (s, 2H) ; 4,32 (t, 2H) ; 3.73 (m, 5H) ; 1.44 (s, 9H)
- LC-MS-DAD-ELSD: 325⁽⁺⁾ = (M+H)⁽⁺⁾; 225⁽⁺⁾ = (M+H)⁽⁺⁾ t-BOC + H
- PF: 177°C

La préparation du 1-(3-trifluorométhyl-phényl)-cyclopropylamine est décrite dans la littérature (Armin de Meijere et al, Organic Letters 2003, 5(5),753-755).

### 1.6: [(1S,2R)-1-Benzyl-2-hydroxy-3-({1-[3-(trifluorométhyl)phényl]cyclopropyl}amino) propyl] carbamate de tert-butyle

4,6 g de 1-(3-trifluorométhyl-phényl)-cyclopropylamine sont dissous dans 12 cm³ de dichlorométhane à une température proche de 20°C. 7,9 g de *tert*-butyl [*S*-(*R,R*)]-(-)-(1-oxiranyl-2-phényléthyl)carbamate et 2,3 g de triflate de scandium sont ajoutés. Le mélange réactionnel est maintenu sous agitation à 20°C pendant 12 h. Il est ensuite dilué dans 100 cm³ de dichlorométhane et lavé successivement avec 2 fois 15 cm³ d'eau, 20 cm³ d'une solution aqueuse saturée en hydrogénocarbonate de sodium et avec 50 cm³ d'une solution aqueuse saturée en chlorure de sodium. Les phases aqueuses sont extraites au dichlorométhane, et les phases organiques sont réunies, séchées sur filtre séparateur de phases et concentrées à l'évaporateur rotatif sous pression réduite (5 kPa). Le solide blanc obtenu est purifié par chromatographie flash sur une cartouche de silice (colonne : 600 g ; granulométrie : 40-60 µm; débit : 80 cm³/min ; éluant : éther de diisopropyle 80%-acétate d'éthyle 20%). On obtient 6,8 g de [(1*S*,2*R*)-1-benzyl-2-hydroxy-3-({1-[3-(trifluorométhyl)phényl]cyclopropyl}amino)propyl]carbamate de test-butyle sous forme d'un solide blanc.
- RMN: 7.64 (s, 1H) ; 7.53 (m, 3H) ; 7.23 (m, 2H) . 7.15 (m, 3H) ; 6.56 (d, 1H) ; 6.12 (d, 1H) ; 4.70 (d, 1H) ; 3.51 (m, 1H) ; 3.38 (m, 1H) ; 3.18 (m, 1H) ; 2.98 (dd, 1H) ; 2.50 (m, 2H) ; 1.21 (s, 9H) ; 0.98 (m, 4H)
- LC-MS-DAD-ELSD: 465⁽⁺⁾ = (M+H)⁽⁺⁾
- PF: 124°C

### 1.7: Chlorhydrate (2:1) de (2R,3S)-3-amino-4-phényl-l-({1-[3-(trifluorométhyl)phényl] cyclopropyl}amino)butan-2-ol

6,8 g de [(1*S*,2*R*)-1-benzyl-2-hydroxy-3-({1-[3-(trifluorométhyl)phényl]cyclopropyl} amino)propyl]carbamate de *tert*-butyle sont dissous dans 250 cm³ de dichlorométhane à une température proche de 20°C. 36,6 cm³ d'une solution d'acide chlorhydrique 4 M dans du dioxanne sont ajoutés. Le mélange réactionnel est maintenu sous agitation 1 h30 à une température proche de 20°C. Le mélange réactionnel est concentré à l'évaporateur rotatif sous pression réduite (5 kPa). Le solide beige obtenu est trituré dans l'éther de diisopropyle puis filtré. On obtient 5,4 g de chlorhydrate (2:1) de (2*R*,3*S*)-3-amino-4-phényl-1-({1-[3-(trifluorométhyl)phényl] cyclopropyl}amino)butan-2-ol.
- RMN : 1,16 (m, 1H) ; 1,27 (m, 1H) ; 1,60 (m, 2H) ; 2,62 (m, 1H) ; 2,85 (d, J = 7,0 Hz, 2H) ; 2,95 (m, 1H) ; 3,52 (m, 1H) ; 4,15 (m, 1H) ; 6,17 (m, 1H) ; 7,24 (m, 5H) ; 7,65 (t, J = 7,5 Hz, 1H) ; 7,77 (d, J = 7,5 Hz, 1H) ; 7,81 (d, J = 7,5 Hz, 1H) ; 7,94 (s, 1H) ; 8,23 (m large, 3H) ; 9,72 (m étalé, 1H) ; 10,35 (m étalé, 1H).
- LC-MS-DAD-ELSD: 409⁽⁺⁾ = (M+Ac Formique-H)⁽⁻⁾; 365⁽⁺⁾ = (M⁺)

### 1.8: 6-{[(1S,2R)-1-Benzyl-2-hydroxy-3-({1-[3-(trifluorométhyl)phényl]cyclopropyl}amino) propyl]carbamoyl}-3,4-dihydropyrrolo[1,2-a]pyrazine-2,8(1H)-dicarboxylate de 2-tert-butyle et de 8-méthyle

3,9 g d'acide 2-(*tert*-butoxycarbonyl)-8-(méthoxycarbonyl)-1,2,3,4-tétrahydropyrrolo[1,2-a]pyrazine-6-carboxylique et 5,4 g de chlorhydrate (2 :1) de (2*R*,3*S*)-3-amino-4-phényl-1-({1-[3-(trifluorométhyl)phényl]cyclopropyl}amino)butan-2-ol sont dissous dans 270 cm³ de dichlorométhane sous atmosphère inerte à une température proche de 20°C. 184 mg d'hydroxybenzotriazole, 7,1 cm³ de *N,N*-diisopropyléthylamine sont ajoutés, suivis de 3,1 g de chlorhydrate de 1-(3-diméthylaminopropyl)-3-éthylcarbodümide et de 166 mg de 4-diméthylaminopyridine. Le mélange réactionnel est maintenu sous agitation 3 h à une température proche de 20°C. Le mélange réactionnel est lavé successivement avec 50 cm³ d'eau, 2 fois 50 cm³ d'une solution aqueuse saturée en dihydrogénophosphate de potassium, encore avec 50 cm³ d'eau et avec 100 cm³ d'une solution aqueuse saturée en chlorure de sodium. Les phases aqueuses sont extraites au dichlorométhane. Les phases organiques sont réunies, séchées sur filtre séparateur de phases et concentrées à l'évaporateur rotatif sous pression réduite (5 kPa). La meringue beige obtenue est purifiée par reprécipitation dans l'éther de diisopropyle à une température proche de 20°C. Le précipité est filtré, rincé à l'éther de diisopropyle et séché à l'air. On obtient 6,8 g de 6-{[(1*S*,2*R*)-1-benzyl-2-hydroxy-3-({1-[3-(triftuorométhyl)phényl]cyctopropyl}amino) propyl]carbamoyl}-3,4-dihydropyrrolo[1,2*-a*]pyrazine-2,8(1*H*)-dicarboxylate de 2-*tert-*butyle et de 8-méthyle sous forme d'un solide blanc.
- RMN : 7.93 (d, 1H) ; 7.72 (s, 1H) ; 7.53 (m, 1H) ; 7.46 (m, 2H) ; 7.18 (m, 4H) ; 7.13 (s, 1H) ; 7.11 (m, 1H) ; 4.80 (d, 1H) ; 4.74 (q, 2H) ; 4.09 (m, 2H) ; 4.01 (m, 1H) ; 3.73 (s, 3H) ; 3.62 (m, 2H) ; 3.50 (m, 1H) ; 3.03 (dd, 1H) ; 2.69 (dd, 1H) ; 2.50 (m, 2H) ; 1.42 (s, 9H) ; 0.80 (m, 4H)
- LC-MS-DAD-ELSD: 669⁽⁻⁾ = (M-H)⁽⁻⁾; 671⁽⁺⁾ = (M+H)⁽⁺⁾
- PF: 136°C

### Exemple 2:

Acide 6-{[(1S,2R)-1-benzyl-2-hydroxy-3-({1-[3-(trifluorométhyl)phényl]cyclopropyl}amino) propyl]carbamoyl}-2-(*tert*-butoxycarbonyl)-1,2,3,4-tétrahydropyrrolo[1,2*-a*] pyrazine-8-carboxylique

2 g de 6-{[(1*S*,2*R*)-1-benzyl-2-hydroxy-3-((1-[3-(trifluorométhyl)phényl]cydopropyl} amino)propyl]carbamoyl}-3,4-dihydropyrrolo[1,2*-a*]pyrazine-2,8(1*H*)-dicarboxylate de 2-*tert*-butyle et de 8-méthyle (préparé à l'exemple 1.8) sont dissous dans 20 cm³ de méthanol à une température proche de 20°C. 6,8 cm³ de soude 1 M sont ajoutés et le mélange réactionnel est chauffé pendant 18 h au reflux du solvant. Le méthanol du mélange réactionnel est évaporé sous pression réduite (5 kPa), puis le résidu aqueux obtenu est acidifié par l'addition de 6,8 cm³ d'acide chlorhydrique 1 M. Le résidu gommeux obtenu est concentré à sec à l'évaporateur rotatif, puis repris dans l'acétate d'éthyle. Les sels de chlorure de sodium sont filtrés, puis le filtrat est concentré à l'évaporateur rotatif sous pression réduite (5 kPa). La meringue beige obtenue est triturée dans l'éther de diisopropyle. Le solide obtenu est filtré, rincé à l'éther de diisopropyle et séché à l'air. On obtient 1,8 g d'acide 6-{[(1*S*,2*R*)-1-benzyl-2-hydroxy-3-({1-[3-(trifluorométhyl)phényl]cyclopropyl}amino)propyl]carbamoyl}-2-(*tert*-butoxycarbonyl)-1,2,3,4-tétrahydropyrrolo[1,2*-a*]pyrazine-8-carboxylique sous forme d'une poudre blanche.
- RMN : de 0,87 à 1,06 (m, 4H) ; 1,42 (s, 9H) ; 2,46 (dd, J = 7,5 et 12,0 Hz, 1H) ; 2,55 (dd, J = 3,5 et 12,0 Hz, 1H) ; 2,69 (dd, J = 10,5 et 14,0 Hz, 1H) ; 3,04 (dd, J = 3,5 et 14,0 Hz, 1H) ; 3,49 (m, 1H) ; 3,62 (m, 2H) ; de 3,96 à 4,15 (m, 3H) ; 4,73 (m, 2H) ; 4,80 (m, 1H) ; 7,09 (s, 1H) ; 7,11 (m, 1H) ; 7,19 (m, 4H) ; 7,42 (m, 2H) ; 7,54 (m, 1H) ; 7,61 (s large, 1H) ; 7,92 (d, J = 9,0 Hz, 1H).
- LC-MS-DAD-ELSD: 655⁽⁻⁾ = (M-H)⁽⁻⁾; 657⁽⁺⁾ = (M+H)⁽⁺⁾
- PF: 134,5°C

### Exemple 3 :

### Chlorhydrate (2:1) d'acide 6-{[(1S,2R)-1-benzyl-2-hydroxy-3-({1-[3-(trifluorométhyl) phényl] cyclopropyl}amino)propyl]carbamoyl}-1,2,3,4-tétrahydropyrrolo[1,2-a]pyrazine-8-carboxylique

200 mg d'acide 6-{[(1*S*,2*R*)-1-benzyl-2-hydroxy-3-({1-[3-(trifluorométhyl)phényl] cyclopropyl}amino)propyl]carbamoyl}-2-(*tert*-butoxycarbonyl)-1,2,3,4-tétrahydropyrrolo [1,2*-a*]pyrazine-8-carboxylique (préparé comme à l'exemple 2) sont dissous dans 5 cm³ de dichlorométhane à une température proche de 20°C. 0,76 cm³ d'une solution d'acide chlorhydrique 4 M dans le dioxanne sont ajoutés au mélange réactionnel qui est maintenu sous agitation 18 h à une température proche de 20°C. Il est ensuite concentré à l'évaporateur rotatif sous pression réduite (5 kPa). Le résidu est trituré dans l'éther de diisopropyle puis le solide est filtré, rincé à l'éther de diisopropyle et séché à l'air. On obtient 194 mg de chlorhydrate (2:1) d'acide 6-{[(1S,2R)-1-benzyl-2-hydroxy-3-({1-[3-(trifluorométhyl)phényl]cyclopropyl}amino)propyl]carbamoyl}-1,2,3,4-tétrahydropyrrolo [1,2*-a*]pyrazine-8-carboxylique sous forme d'un solide jaune.
- RMN: 1,19 (m, 1H) ; 1,28 (m, 1H) ; 1,58 (m, 2H) ; 2,71 (m, 2H) ; 2,94 (m, 1H) ; 3,05 (dd, J = 3,0 et 14,0 Hz, 1H) ; 3,48 (m, 2H) ; 3,85 (m, 1H) ; 3,95 (m, 1H) ; 4,32 (m, 2H) ; 4,50 (s, 2H) ; 5,80 (m large, 1H) ; 7,11 (m, 1H) ; 7,16 (s, 1H) ; 7,18 (m, 4H) ; 7,60 (t, J = 7,5 Hz, 1H) ; 7,73 (d, J = 7,5 Hz, 1H) ; 7,84 (d, J = 7,5 Hz, 1H) ; 7,97 (s, 1H) ; 8,13 (d, J = 9,0 Hz, 1H) ; de 9,26 à 10,0 (m étalé, 4H) ; 12,4 (m étalé, 1H) .
- LC-MS-DAD-ELSD: 557⁽⁺⁾= (M+H)⁽⁺⁾
- PF: 196°C

### Exemple 4:

### 6-{[(1S,2R)-1-Benzyl-2-hydroxy-3-({1-[3-(trifluorométhyl)phényl]cyclopropyl}amino) propyl]carbamoyl}-8-(diéthylcarbamoyl)-3,4-dihydropyrrolo[1,2-a]pyrazine-2(1H)-carboxylate de tert-butyle

0,6 g d'acide 6-{[(1*S*,2*R*)-1-benzyl-2-hydroxy-3-({1-[3-(trifluorométhyl)phényl] cyclopropyl} amino)propyl]carbamoyl}-2-(*tert*-butoxycarbonyl)-1,2,3,4-tétrahydropyrrolo[1,2*-a*]pyrazine-8-carboxylique (préparé à l'exemple 2) et 0,094 cm³ de diéthylamine sont dissous dans 25 cm³ de dichlorométhane à une température proche de 20°C. 12 mg d'hydroxybenzotriazole, 0,5 cm³ de *N,N*-diisopropyléthylamine sont ajoutés, suivis de 210 mg de chlorhydrate de 1-(3-diméthy)aminopropyl)-3-éthy)carbodiimide et de 11 mg de 4-diméthylaminopyridine. Le mélange réactionnel est maintenu sous agitation 18 h à une température proche de 20°C. Il est lavé successivement avec 10 cm³ d'une solution d'acide chlorhydrique 1M, avec 20 cm³ d'eau, avec 10 cm³ d'une solution aqueuse saturée en hydrogénocarbonate de sodium et avec 15 cm³ d'une solution aqueuse saturée en chlorure de sodium. La phase organique est séchée sur filtre séparateur de phases et concentrée à l'évaporateur rotatif sous pression réduite (5 kPa). La meringue obtenue est purifiée par chromatographie flash sur une cartouche de silice (colonne : 70 g ; granulométrie : 15-40 µm ; débit : 30 cm³/min ; éluant : cyclohexane 20%- acétate d'éthyle 80%). Après concentration des fractions sous pression réduite (5 kPa), on obtient 310 mg de 6-{[(1*S*,2*R*)-1-benzyl-2-hydroxy-3-({1-[3-(trifluorométhyl) phényl]cyclopropyl}amino)propyl]carbamoyl}-8-(diéthylcarbamoyl)-3,4-dihydropyrrolo[1,2-*a*]pyrazine-2(1*H*)-carboxylate de *tert*-butyle, sous forme d'un solide amorphe blanc.
- RMN : de 0,89 à 1,06 (m, 4H) ; 1,12 (t, J = 7,0 Hz, 6H) ; 1,40 (s, 9H) ; 2,50 (m masqué, 1H) ; 2,59 (m, 2H) ; 2,70 (dd, J = 10,5 et 14,0 Hz, 1H) ; 3,03 (dd, J = 3,5 et 14,0 Hz, 1H) ; 3,40 (q, J = 7,0 Hz, 4H) ; 3,53 (m, 1H) ; 3,60 (m, 2H) ; 3,99 (m, 2H) ; 4,13 (m, 1H) ; 4,58 (m, 2H) ; 4,84 (d, J = 6,0 Hz, 1H) ; 6,75 (s, 1H) ; 7,11 (m, 1H) ; 7,19 (m, 4H) ; de 7,42 à 7,51 (m, 2H) ; 7,54 (d, J = 7,5 Hz, 1H) ; 7,63 (s, 1H) ; 7,88 (d, J = 9,0 Hz, 1H).
- LC-MS-DAD-ELSD: 710⁽⁻⁾ = (M-H)⁽⁻⁾; 712⁽⁺⁾ = (M+H)⁽⁺⁾

### Exemple 5:

### Chlorhydrate (2:1) de 6-{(1S,2R)-1-benzyl-2-hydroxy-3-[1-(3-trifluorométhylphényl)-cyclopropylamino]-propyl}-N-8,N 8-diéthyl-1,2,3,4-tétrahydropyrrolo[1,2-a]pyrazine-6,8-dicarboxamide

200 mg de 6-{[(1*S*,2*R*)-1-benzyl-2-hydroxy-3-({1-[3-(trifluorométhyl)phényl]cyclopropyl} amino)propyl]carbamoyl}-8-(diéthylcarbamoyl)-3,4-dihydropyrrolo[1,2*-a*]pyrazine-2(1*H*)-carboxylate de *tert*-butyle (préparé comme à l'exemple 4) sont dissous dans 5 cm³ de dichlorométhane à une température proche de 20°C. 0,70 cm³ d'une solution d'acide chlorhydrique 4 M dans le dioxanne sont ajoutés. Le mélange réactionnel est maintenu sous agitation 18 h à une température proche de 20°C. Il est ensuite concentré à l'évaporateur rotatif sous pression réduite (5 kPa). Le résidu est trituré dans l'éther de diisopropyle puis le solide est filtré, rincé à l'éther de diisopropyle et séché à l'air. On obtient 123 mg de chlorhydrate (2:1) de 6-{(1S,2R)-1-benzyl-2-hydroxy-3-[1-(3-trifluorométhylphényl)-cyclopropylamino]-propyl}-*N*-8,*N*-8-diéthyl-1,2,3,4-tétrahydropyrrolo[1,2*-a*]pyrazine-6,8-dicarboxamide, sous forme d'une poudre jaune.
- RMN : de 1,10 à 1,35 (m, 8H) ; 1,60 (m, 2H) ; de 2,61 à 2,81 (m, 2H) ; de 2,92 à 3,12 (m, 2H) ; de 3,25 à 3,58 (m partiellement masqué, 6H) ; 4,90 (m, 2H) ; 4,22 (m, 1H) ; de 4,30 à 4,43 (m, 3H) ; 5,89 (m étalé, 1H) ; 6,90 (s, 1H) ; 7,10 (t, J = 7,5 Hz, 1H) ; de 7,13 à 7,24 (m, 4H) ; 7,61 (t, J = 7,5 Hz, 1H) ; 7,77 (d, J = 7,5 Hz, 1H) ; 7,87 (d, J = 7,5 Hz, 1H) ; 8,00 (s, 1H) ; 8,24 (d, J = 9,0 Hz, 1H) ; de 9,35 à 9,53 (m étalé, 2H) ; 9,77 (m étalé, 1H) ; 9,98 (m étalé, 1H).
- LC-MS-DAD-ELSD: 612⁽⁺⁾ = (M+H)⁽⁺⁾
- PF: 143°C

### Exemple 6 :

### 6-{(1S,2R)-1-benzyl-2-hydroxy-3-[1-(3-trifluorométhylphényl)-cyclopropylamino]-propyl}-N-8,N-8-diéthyl-2-(méthylsulfonyl)-1,2,3,4-tétrahydropyrrolo[1,2-a]pyrazine-6,8-dicarboxamide

### 6.1: 6-({(1S)-1-[(5R)-2-oxo-3-{1-[3-(trifluorométhyl)phényl]cyclopropyl}-1,3-oxazolidin-5-yl]-2-phényléthyl}carbamoyl)-3,4-dihydropyrrolo[1,2-a]pyrazine-2,8(1H)-dicarboxylate de 2-tert-butyle et de 8-méthyle

A une solution de 2 g de 1.8: 6-{[(1*S*,2*R*)-1-benzyl-2-hydroxy-3-({1-[3-(trifluorométhyl)phényl]cyclopropyl}amino]propyl}carbamoyl}-3,4-dihydropyrrolo[1,2*-a*] pyrazine-2,8(1*H*)-dicarboxylate de 2-*tert* butyle et de 8-méthyle (préparé à l'exemple 1.8) dans 10 cm³ de dichlorométhane refroidie à une température proche de 0°C et sous atmosphère inerte, on ajoute 0,967 g de N,N'-carbonyldiimidazole puis on agite en laissant revenir à une température voisine de 25°C. L'agitation est maintenue durant 48 H. 6 cm³ de dichlorométhane et 15 cm³ d'une solution aqueuse d'acide chlorhydrique sont ajoutés. Le mélange réactionnel est extrait au moyen de 2 fois 40 cm³ de dichlorométhane. Les phases organiques sont réunies, séchées puis concentrées à l'évaporateur rotatif sous pression réduite (5 kPa). Le solide résiduel est purifié par chromatographie flash sur silice (colonne : 200 g ; granulométrie : 15-40 µm ; débit : 90 cm³/min. ; éluant : cyclohexane 50% - acétate d'éthyle 50%). Les fractions contenant le produit attendu sont concentrées à sec à l'évaporateur rotatif sous pression réduite (5 kPa). On obtient 1.46 g de 6-({(1S)-1-[(5R)-2-oxo-3-{1-[3-(trifluorométhyl) phényl]cyclopropyl}-1,3-oxazolidin-5-yl]-2-phényléthyl}carbamoyl)-3,4-dihydropyrrolo[1,2-a]pyrazine-2,8(1H)-dicarboxylate de 2-tert-butyle et de 8-méthyle sous la forme d'un solide blanc.
- PF: 106°C
- RMN 1.30 - 1.56 (m partiellement masqué, 4 H) 1.46 (s, 9 H) 2.82 (dd, *J*=14.0, 10.7 Hz, 1 H) 2.94 (d, *J*=14.0, 3.6 Hz, 1 H) 3.60 (dd, *J*=9.0, 6.4 Hz, 1 H) 3.68 (m, 2 H) 3.75 (t, *J*=9.0 Hz, 1 H) 3.79 (s, 3 H) 4.13 - 4.29 (m, 2 H) 4.38 (m 1 H) 4.62 (m, 1 H) 4.79 (s large, 2 H) 7.19 (m, 1 H) 7.27 (m, 5 H) 7.48 (s large, 1 H) 7.52 - 7.66 (m, 3 H) 8.18 (d, *J*=9.0 Hz, 1 H).
- LC-MS-DAD-ELSD : Tr (mn) = 2,44 ; MH+ = 697⁺ ; Pureté : 98 %

### 6.2: 6-({(1S)-1-[(5R)-2-oxo-3-{1-[3-(trifluorométhyl)phényl]cyclopropyl}-1,3-oxazolidin-5-yl]-2-phényléthyl}carbamoyl)-1,2,3,4-tétrahydropyrrolo[1,2-a]pyrazine-8-carboxylate de méthyle

1,3 g de 6-({(1S)-1-[(5R)-2-oxo-3-{1-[3-(trifluorométhyl)phényl]cyclopropyl}-1,3-oxazolidin-5-yl]-2-phényléthyl}carbamoyl)-3,4-dihydropyrrolo[1,2*-a*]pyrazine-2,8(1H)-dicarboxylate de 2-tert-butyle et de 8-méthyle sont dissous dans 5 cm³ de dichlorométhane sous atomsphère inerte à une température proche de 20°C puis 5 cm³ d'une solution d'acide chlorhydrique 4M dans le dioxane sont ajoutés en 10 min. Le mélange réactionnel est agité 15 h à une température voisine de 20°C puis concentré à sec à l'évaporateur rotatif sous pression réduite (5 kPa). Le solide résiduel, jaune pâle, est repris par 15 cm³ d'éther de diisoproplyle et trituré. Le solide est ensuite filtré, rincé avec 2 fois 10 cm³ de d'éther de diisopropyle et séché à l'air pour obtenir 1,2 g de 6-({(1S)-1-[(5R)-2-oxo-3-{1-[3-(trifluorométhyl)phényl]cyclopropyl}-1,3-oxazolidin-5-yl]-2-phényléthyl}carbamoyl)-1,2,3,4-tétrahydropyrrolo[1,2*-a*]pyrazine-8-carboxylate de méthyle sous la forme d'un solide blanc-crème dichlorhydraté. Ce solide est utilisé sans autre purification dans l'exemple 6.3.
- RMN : 1.28 - 1.45 (m, 3 H) 1.51 (m, 1 H) 2.78 (dd, *J*=13.8, 10.5 Hz, 1 H) 2.90 (dd, *J*=13.8, 3.8 Hz, 1 H) 3.47 (m, 2 H) 3.59 (m, 1 H) 3.72 (t, *J*=8.9 Hz, 1 H) 3.77 (s, 3 H) 4.24 - 4.48 (m, 3 H) 4.49 (s, 2 H) 4.61 (m, 1 H) 7.16 (m, 1 H) 7.24 (m, 4 H) 7.33 (s, 1 H) 7.44 (s large, 1 H) 7.48 - 7.63 (m, 3 H) 8.24 (d, *J*=9.0 Hz, 1 H) 9.52 (m étalé, 2 H)
- LC-MS-DAD-ELSD : Tr (mn) = 3,26 ; MH+ = 597 + ; MH- = 595 - ; Pureté : 98 %

### 6.3:2-(méthylsulfonyl)-6-({(1S)-1-[(5R)-2-oxo-3-{1-[3-(trifluorométhyl)phényl] cyclopropyl}-1,3-oxazolidin-5-yl]-2-phényléthyl}carbamoyl)-1,2,3,4-tétrahydropyrrolo[1,2-a]pyrazine-8-carboxylate de méthyle

A une solution de 900 mg de 6-({(1S)-1-[(5R)-2-oxo-3-{1-[3-(trifluorométhyl) phényl]cyclopropyl}-1,3-oxazolidin-5-yl]-2-phényléthyl}carbamoyl)-1,2, 3,4-tétrahydro pyrrolo[1,2*-a*]pyrazine-8-carboxylate de méthyle, 0,5 cm³ de triéthylamine et 30 cm³ de dichloromethane, on ajoute, sous atmosphère inerte, goutte à goutte en 5 min, une solution de 0,132 cm³ de chlorure de mésyle dans 2 cm³ de dichlorométhane. Le mélange réactionnel est agité 15 h à une température voisine de 20°C puis on additionne 20 cm³ d'eau et on amène la phase aqueuse à pH 2 par addition d'une solution aqueuse d'acide chlorhydrique 1 N. La phase organique est décantée et séparée puis lavée avec 20 cm³ d'eau. Elle est ensuite séchée sur sulfate de magnésium puis concentrée à sec à l'évaporateur rotatif sous pression réduite (5 kPa). Les 940 mg de solide obtenus sont purifiés par chromatographie flash sur silice (colonne : 100 g ; granulométrie : 15-40µm ; débit : 30 cm³/min. ; éluant : cyclohexane 50% - acétate d'éthyle 50%). Les fractions contenant le produit attendu sont concentrées à sec à l'évaporateur rotatif sous pression réduite (5 kPa). Les 713 mg de solide incolore obtenus sont ensuite repris par 30 cm³ de tétrahydrofurane. On ajoute ensuite 3 cm³ d'une solution d'acide chlorhydrique 4N dans le tétrahydrofurane et on laisse agiter à une température proche de 20°C durant 1 h. Le mélange réactionnel est ensuite concentré à sec à l'évaporateur rotatif sous pression réduite (5 kPa). On obtient 704 mg d'un solide sous la forme d'une meringue blanche, qui sont triturés avec 10 cm³ d'éther de diisopropyle. Le solide est filtré puis séché à l'air à une température voisine de 20°C. On obtient 596 mg de 2-(méthylsulfonyl)-6-({(1S)-1-[(5R)-2-oxo-3-{1-[3-(trifluorométhyl)phényl]cyclopropyl}-1,3-oxazolidin-5-yl]-2-phényléthyl} carbamoyl)-1,2,3,4-tétrahydropyrrolo[1,2*-a*]pyrazine-8-carboxylate de méthyle sous la forme d'un solide blanc à reflets jaunes.
- PF: 104°C
- RMN (400 MHz) 1.26 - 1.55 (m, 4 H) 2.78 (dd, *J*=13.7, 10.3 Hz, 1 H) 2.90 (dd, *J*=13.7, 3.9 Hz, 1 H) 3.02 (s, 3 H) 3.50 - 3.62 (m, 3 H) 3.72 (t, *J*=8.8 Hz, 1 H) 3.76 (s, 3 H) 4.18 - 4.41 (m, 3 H) 4.60 (m, 1 H) 4.63 (s, 2 H) 7.16 (m, 1 H) 7.25 (m, 4 H) 7.29 (s, 1 H) 7.44 (s large, 1 H) 7.49 - 7.64 (m, 3 H) 8.20 (d, *J*=9.3 Hz, 1 H)
- LC-MS-DAD-ELSD : Tr (mn) = 4,42 ; (M+H)⁽⁺⁾ = 675 ⁽⁺⁾; MH⁽⁻⁾ = 673⁽⁻⁾ ; Pureté : 98 %

### 6.4 : Acide 2-(méthylsulfonyl)-6-({(1S)-1-[(5R)-3-{1-méthyl-1-[3-(trifluorométhyl)phényl éthyl}-2-oxo-1,3-oxazolidin-5-yl]-2-phényléthyl}carbamoyl)-1,2,3,4-tétrahydropyrrolo[1,2-a]pyrazine-8-carboxylique

Un mélange de 400 mg de 2-(méthylsulfonyl)-6-({(1S)-1-[(5R)-2-oxo-3-{1-[3-(trifluorométhyl)phényl]cyclopropyl}-1,3-oxazolidin-5-yl]-2-phényléthyl}carbamoyl)-1,2,3,4-tétrahydropyrrolo[1,2*-a*]pyrazine-8-carboxylate de méthyle, de 10 cm³ de methanol et 2 cm³ d'une solution aqueuse d'hydroxyde de sodium 1 N est chauffé durant 3 h à une température proche du reflux. On laisse ensuite le mélange revenir à une température proche de 20°C puis les solvants sont évaporés à l'évaporateur rotatif sous pression réduite (5 kPa). Le résidu de l'évaporation est repris par 10 cm³ d'eau et 10 cm³ d'acétate d'éthyle. La phase organique est séparée et la phase aqueuse est acidifiée à pH ∼3-4 au moyen d'une solution aqueuse 1 N d'acide chlorhydrique. La phase aqueuse est ensuite extraite par 10 cm³ d'acétate d'éthyle. La phase organique est séparée puis lavée successivement avec 10 cm³ d'eau distillée et 5 cm³ d'une solution saturée en chlorure de sodium. La phase organique est séchée sur sulfate de magnésium puis les solvants sont évaporés à sec à l'évaporateur rotatif sous pression réduite (5 kPa). On obtient 330 mg d'acide 2-(méthylsulfonyl)-6-({(1S)-1-[(5R)-3-{1-méthyl-1-[3-(trifluorométhyl)phényl] éthyl}-2-oxo-1,3-oxazolidin-5-yl]-2-phényléthyl}carbamoyl)-1,2,3,4-tétrahydropyrrolo[1,2-*a*]pyrazine-8-carboxylique sous la forme d'un solide jaune orangé.
- PF: 147°C
- RMN : 1.26 -1.53 (m, 4 H) 2.79 (dd, *J*=13.9, 10.3 Hz, 1 H) 2.90 (dd, *J*=13.9, 3.9 Hz, 1 H) 3.00 (s, 3 H) 3.52 (t, *J*=5.6 Hz, 2 H) 3.57 (dd, *J*=9.0, 6.1 Hz, 1 H) 3.71 (t, *J*=9.0 Hz, 1 H) 4.15 - 4.40 (m, 3 H) 4.58 (m, 1 H) 4.63 (s, 2 H) 7.16 (m, 1 H) 7.21 (s, 1 H) 7.24. (m, 4 H) 7.45 (s large, 1 H) 7.49 - 7.64 (m, 3 H) 8.13 (d, *J*=8.8 Hz, 1 H) 12.25 (m étalé, 1 H)
- LC-MS-DAD-ELSD : Tr (mn) = 4,14 ; (M+H)⁽⁺⁾ = 661 ⁽⁺⁾; Pureté : 98 %

### 6.5 : N-8,N-8-diéthyl-2-(méthylsulfonyl)-N 6-{(1S)-1-[(5R)-2-oxo-3-{1-[3-(trifluorométhyl)phényl]cyclopropyl}-1,3-oxazolidin-5-yl]-2-phényléthyl}-1,2,3,4-tétrahydropyrrolo[1,2-a]pyrazine-6,8-dicarboxamide

Dans un tricol, on introduit successivement 5 cm³ de diméthylformamide, 0,170 cm³ de diéthylamine et 0,05 cm³ de diisopropyléthylamine. On ajoute au goutte-à-goutte en 10 minutes une solution de 116 mg d'hexafluorophospate de 1-[bis (diméthylamino)méthylène]-1H-1,2,3-triazolo[4,5-b]pyridinium-3-oxyde (HATU) et de 125 mg d'acide 2-(méthylsulfonyl)-6-({(1S)-1-[(5R)-3-{1-méthyl-1-[3-(trifluorométhyl)phényl] éthyl}-2-oxo-1, 3-oxazolidin-5-yl]-2-phényléthyl}carbamoyl)-1,2,3,4-tétrahydropyrrolo[1,2a] pyrazine-8-carboxylique dans 10 cm³ de diméthylformamide. Le mélange réactionnel est laissé agiter durant 48 h à une température voisine de 20°C. On ajoute 10 cm³ d'acétate d'éthyle et 15 cm³ d'une solution aqueuse 0,1 N d'acide chlorhydrique au milieu réactionnel. La phase organique est séparée puis lavée successivement avec 10 cm³ d'eau et 5 cm³ de solution aqueuse saturée en chlorure de sodium. Elle est ensuite séchée sur sulfate de sodium et concentrée à l'évaporateur rotatif sous pression réduite (5 kPa). Le solide résiduel obtenu est purifié par chromatographie flash sur silice (colonne : 25 g ; granulométrie : 15-40µm ; débit : 10 cm³/min. ; éluant : acétate d'éthyle 100%). Les fractions contenant le produit attendu sous concentrées à sec à l'évaporateur rotatif sous pression réduite (5 kPa). On obtient 100 mg d'un solide qui est repris et trituré avec 3 cm³ d'éther de diisopropyle conduit à 93 mg de *N*-8,*N*-8-diéthyl-2-(méthylsulfonyl)-*N*-6-{(1S)-1-[(5R)-2-oxo-3-{1-[3-(trifluorométhyl)phényl] cyclopropyl}-1,3-oxazolidin-5-yl]-2-phényléthyl}-1,2,3,4-tétrahydropyrrolo[1,2*-a*]pyrazine-6,8-dicarboxamide sous la forme d'un solide blanc à reflets crème.
- PF: 109°C
- RMN : 1.16 (t, *J*=7.1 Hz, 6 H) 1.28 - 1.54 (m, 4 H) 2.79 (dd, *J*=13.7, 10.3 Hz, 1 H) 2.92 (dd, *J*=13.7, 3.9 Hz, 1 H) 2.97 (s, 3 H) 3.36 - 3.62 (m, 7 H) 3.75 (t, *J*=8.8 Hz, 1 H) 4.17 - 4.32 (m, 3 H) 4.46 (m, 2 H) 4.60 (m, 1 H) 6.87 (s, 1 H) 7.16 (t, *J*=7.5 Hz, 1 H) 7.19 - 7.30 (m, 4 H) 7.48 (s large, 1 H) 7.50 - 7.66 (m, 3 H) 8.09 (d, *J*=9.3 Hz, 1 H)
- LC-MS-DAD-ELSD : Tr (mn) = 5,08 ; (M+H)⁽⁺⁾ = 716 ⁽⁺⁾; MH⁽⁻⁾ = 714 ⁽⁻⁾ ; Pureté : 98 %

### 6.6 : 6-{(1S,2R)-1-benzyl-2-hydroxy-3-[1-(3-trifluorométhylphényl)-cydopropylamino]-propyl}-N-8,N-8-diéthyl-2-(méthylsulfonyl)-1,2,3,4-tétrahydropyrrolo[1,2-a]pyrazine-6,8-dicarboxamide

A une solution de 60 mg de *N*-8,*N*-8-diéthyl-2-(méthylsulfonyl)-*N*-6-{(1S)-1-[(5R)-2-oxo-3-{1-[3-(trifluorométhyl)phényl]cyclopropyl}-1,3-oxazolidin-5-yl]-2-phényléthyl}-1,2,3,4 tétrahydropyrrolo[1,2*-a*]pyrazine-6,8-dicarboxamide dans 2 cm³ d'isopropanol et 0,2 cm³ de méthanol, on ajoute 1 cm³ d'une solution aqueuse d'hydroxyde de sodium à 30%. Le mélange réactionnel est chauffé durant 2 h à une température voisine du reflux. On laisse ensuite le mélange revenir à une température proche de 20°C puis les solvants sont évaporés à l'évaporateur rotatif sous pression réduite (5 kPa). Le résidu de l'évaporation est repris par 5 cm³ d'eau et 5 cm³ d'acétate d'éthyle. La phase organique est séparée et la phase aqueuse est acidifiée à pH -4-5 au moyen d'une solution aqueuse 1 N d'acide chlorhydrique. La phase aqueuse est ensuite extraite par 10 cm³ d'acétate d'éthyle. La phase organique est séparée puis lavée successivement avec 2 fois 5 cm³ d'eau distillée puis séchée sur sulfate de magnésium. La phase organique est concentrée à sec à l'évaporateur rotatif sous pression réduite (5 kPa). Le solide résiduel obtenu (38 mg) est purifié par chromatographie flash sur silice (colonne : 15 g; granulométrie:15-40µm ; débit : 10 cm³/min. ; éluant : acétate d'éthyle 100%). Les fractions contenant le produit attendu sous concentrées à sec à l'évaporateur rotatif sous pression réduite (5 kPa). On obtient 6 mg de 6-{(1S,2R)-1-benzyl-2-hydroxy-3-[1-(3-trifluorométhylphényl)-cyclopropylamino]-*propyl}-N-8,N-8-diéthyl-2-(méthylsulfonyl)-1,2,3,4-tétrahydropyrrolo[1,2*-a*]pyrazine-6,8-dicarboxamide sous la forme d'un solide blanc. • RMN : de 0,89 à 1,06 (m, 4H) ; 1,14 (t, J = 7,0 Hz, 6H) ; de 2,42 à 2,77 (m partiellement masqué, 3H) ; 2,98 (s, 3H) ; 3,02 (dd, J = 4,0 et 13,0 Hz, 1H) ; de 3,23 à 3,59 (m partiellement masqué, 7H) ; 3,98 (m, 1H) ; 4,10 (m, 1H) ; 4,23 (m, 1H) ; 4,45 (m, 2H) ; 4,87 (d, J = 5,0 Hz, 1H) ; 6,80 (s, 1H) ; de 7,07 à 7,23 (m, 5H) ; de 7,42 à 7,58 (m, 3H) ; 7,64 (s large, 1H) ; 7,91 (d, J = 8,5 Hz, 1H).

### Exemple 7 :

### 7.1 : Base

### 6-[(1S,2R)-1-(3,5-difluorobenzyl)-2-hydroxy-3-({1-[3-(trifluorométhyl)phényl]cyclopropyl} amino)propyl]-1-oxo-2-(1-propylbutyl)-1,2,3,4-tétrahydropyrrolo[1,2-a]pyrazine-6-carboxamide

### 7.1.1:1-(2-bromoéthyl)-1H-pyrrole-2-carboxylate d'éthyle

5 g de pyrrole-2-carboxylate d'éthyle, 15,5 cm³ de 1,2-dibromoéthane, 21,5 cm³ de soude et 11,58 g de bromure de tétrabutylammonium sont agités sous atmosphère inerte pendant 20 h à une température proche de 20°C. 100 cm³ de dichlorométhane et 50 cm³ d'eau sont ajoutés au milieu réactionnel. La phase aqueuse est extraite par 3 fois 20 cm³ de dichlorométhane. Les phases organiques sont réunies puis lavées avec 50 cm³ de solution aqueuse saturée en chlorure de sodium, séchée sur sulfate de magnésium et concentrée à l'évaporateur rotatif sous pression réduite (5 kPa). Les 29,5 g d'huile limpide obtenus sont purifiés par chromatographie flash sur silice (colonne : 400 g ; granulométrie : 15-40 µm; éluant : dichlorométhane 100%). Après concentration des fractions sous pression réduite, on obtient 6,78 g de 1-(2-bromoéthyl)-1*H-*pyrrole-2-carboxylate d'éthyle.
- LC-MS-DAD-ELSD: 246⁽⁺⁾⁷⁹Br = (M+H)⁽⁺⁾
- RMN : 1.27 (t, *J*=7.1 Hz, 3 H) 3.74 (t, *J*=6.5 Hz, 2 H) 4.21 (q, *J*=7.1 Hz, 2 H) 4.65 (t, *J*=6.5 Hz, 2 H) 6.13 (dd, *J*=3.9, 2.6 Hz, 1 H) 6.88 (dd, *J*=3.9, 1.8 Hz, 1 H) 7.20 (dd, *J*=2.6, 1.8 Hz, 1 H)

### 7.1.2: 1-{2-[(1--propylbutyl)amino]éthyl}-1H-pyrrole-2-carboxylate d'éthyle

6,76 g de 1-(2-bromoéthyl)-1*H-*pyrrole-2-carboxylate d'éthyle, 11,08 g de 4-heptylamine, 0,46 g d'iodure de potassium et 135 cm³ d'acétonitrile sont agités à 70°C sous atmosphère inerte pendant 20 h. Le mélange réactionnel est amené à 30°C pour être concentré à l'évaporateur rotatif sous pression réduite (5 kPa). 200 cm³ de dichlorométhane et 100 cm³ d'eau sont alors ajoutés au résidu de concentration. Les phases sont agitées 5 min puis sont séparées. La phase aqueuse est extraite par 3 fois 50 cm³ de dichlorométhane. Les phases organiques sont réunies, séchées sur sulfate de magnésium et concentrées à l'évaporateur rotatif sous pression réduite (5 kPa). Les 10,8 g d'huile ambrée obtenus sont purifiés par chromatographie flash sur silice (colonne : 450 g ; granulométrie : 20-40 µm sphérique; éluant : gradient cyclohexane 70%-acétate d'éthyle 30% à cyclohexane 50%-acétate d'éthyle 50%). Après concentration des fractions sous pression réduite, on obtient 4,31 g de 1-{2-[(1-propylbutyl)amino]éthyl}-1*H-*pyrrole-2-carboxylate d'éthyle.
- SM-EI: 280⁽⁺⁾=M⁽⁺⁾
- RMN : 0.82 (m, 6 H) 0.93 - 1.32 (m, 11 H) 2.36 (m, 1 H) 2.77 (t, *J*=6.6 Hz, 2 H) 4.19 (q, *J*=7.1 Hz, 2 H) 4.29 (t, *J*=6.6 Hz, 2 H) 6.07 (dd, *J*=4.0, 2.5 Hz, 1 H) 6.83 (dd, *J*=4.0, 1.8 Hz, 1 H) 7.12 (dd, *J*=2.5, 1.8 Hz, 1 H)

### 7.1.3: 2-(1-propylbutyl)-3,4-dihydropyrrolo[1,2-a]pyrazin-1(2H)-one

4,3 g de 1-{2-[(1-propylbutyl)amino]éthyl}-1*H-*pyrrole-2-carboxylate d'éthyle sont dissous sous atmosphère inerte, dans 130 cm³ de toluène à une température proche de 20°C. 23 cm³ de solution toluénique, 2 M de triméthylaluminium sont coulés sur le mélange réactionnel en 5 min. Le mélange réactionnel est chauffé sous agitation pendant 20 h à 110°C puis 72 h à 20°C. II est ensuite versé sur un mélange glace-eau-acétate d'éthyle. La suspension obtenue est filtrée sur culot de Célite 545. La phase aqueuse est extraite par 3 fois 75 cm³ d'acétate d'éthyle. Les phases organiques sont réunies puis lavées avec 50 cm³ de solution aqueuse saturée en chlorure de sodium, séchées sur sulfate de magnésium et concentrées à l'évaporateur rotatif sous pression réduite (5 kPa). Les 4,7 g d'huile brune obtenus sont purifiés par chromatographie flash sur silice (colonne : 200 g ; granulométrie : 15-40 µm; éluant : gradient dichlorométhane 100% à dichlorométhane 80% - acétate d'éthyle 20%). Après concentration des fractions sous pression réduite, on obtient 1,56 g de 2-(1-propylbutyl)-3,4-dihydropyrrolo[1,2*-a*]pyrazin-1(2*H*)-one.
- SM-El: 234⁽⁺⁾ = M⁽⁺⁾
- RMN : 0.86 (t, *J*=7.3 Hz, 6 H) 1.12 - 1.29 (m, 4 H) 1.39 (m, 2 H) 1.47 (m, 2 H) 3.43 (m, 2 H) 4.10 (m, 2 H) 4.58 (m, 1 H) 6.12 (dd, *J*=3.8, 2.5 Hz, 1 H) 6.59 (dd, *J*=3.8, 1.5 Hz, 1 H) 6.94 (dd, *J*=2.5, 1.5 Hz, 1 H)

### 7.1.4: 6-bromo-2-(1-propylbutyl)-3,4-dihydropyrrolo[1,2-a]pyrazin-1(2H)-one

1,53 g de 2-(1-propylbutyl)-3,4-dihydropyrrolo[1,2*-a*]pyrazin-1(2*H*)-one et 1,16 g de *N-*bromosuccinimide sont dissous dans 153 cm³ de tétrachlorure de carbone à une température proche de 20°C. L'agitation est maintenue 2 h 30. Le mélange réactionnel est concentré à l'évaporateur rotatif sous pression réduite (5 kPa). Le résidu solide obtenu est purifié par chromatographie flash sur silice (colonne : 200 g ; granulométrie : 15-40 µm ; éluant : gradient dichlorométhane 100% à dichlorométhane 90%-acétate d'éthyle 10%). Après concentration des fractions sous pression réduite, on obtient 1,54 g de 6-bromo-2-(1-propylbutyl)-3,4-dihydropyrrolo[1,2*-a*]pyrazin-1(2*H*)-one.
- RMN : 0,86 (t, J = 7,5 Hz, 6H) ; de 1,10 à 1,29 (m, 4H) ; de 1,32 à 1,54 (m, 4H) ; 3,48 (m, 2H) ; 4,02 (m, 2H) ; 4,56 (m, 1H) ; 6,30 (d, J = 4,0 Hz, 1H) ; 6,68 (d, J = 4,0 Hz, 1H).
- SM-EI: M ⁽⁺⁾. = 312 ⁽⁺⁾.

### 7.1.5 : Acide 1-oxo-2-(1-propylbutyl)-1,2,3,4-tétrahydropyrrolo[1,2-a]pyrazine-6-carboxylique

Dans un tricol agité et purgé au monoxyde de carbone, 800 mg de 6-bromo-2-(1-propylbutyl)-3,4-dihydropyrrolo[1,2*-a*]pyrazin-1(2*H*)-one, 27 cm³ de diméthylformamide, 1,3 cm³ d'eau, 0,953 g d'acétate de potassium, 85 mg d'iodure de potassium, 230 mg d'acétate de palladium et 535 mg de triphénylphosphine sont introduits successivement à une température proche de 20°C. Le mélange réactionnel est soumis à un bullage de monoxyde de carbone puis est chauffé à 100°C pendant 6 h. Le mélange réactionnel est maintenu à 100°C pendant 20 h puis refroidi à 25°C pour être filtré sur membrane millipore 45µm. Le filtrat est concentré à l'évaporateur rotatif sous pression réduite (5 kPa). Le résidu huileux obtenu est repris dans 15 cm³ d'un mélange eau-glace et 20 cm³ d'acétate d'éthyle. Le pH est alcalinisé avec de la soude 5 M (pH>10). Après décantation du filtrat, la phase aqueuse est lavée par 3 fois 20 cm³ d'acétate d'éthyle. Elle est ensuite acidifiée sous agitation avec une solution d'acide chlorhydrique 5M (pH=1) puis extraite par 3 fois 20 cm³ d'acétate d'éthyle. Les phases organiques sont réunies puis lavées avec 10 cm³ de solution aqueuse saturée en chlorure de sodium, séchées sur sulfate de magnésium et concentrées à l'évaporateur rotatif sous pression réduite (5 kPa). Les 1,2 g d'huile orange obtenus sont purifiés par chromatographie flash sur silice (colonne : 90 g ; granulométrie : 15-40 µm ; éluant : gradient dichlorométhane 100% à dichlorométhane 95% -5%méthanol). Après concentration des fractions sous pression réduite, on obtient 0,69 g d'acide 1-oxo-2-(1-propylbutyl)-1,2,3,4-tétrahydropyrrolo[1,2-a]pyrazine-6-carboxylique.
- SM-EI: 278⁽⁺⁾ = M⁽⁺⁾ ; 261⁽⁺⁾ = M⁽⁺⁾-OH
- RMN:86 (t, *J*=7.2Hz, 6 H) 1.12 - 1.57 (m, 8 H) 3.49 (m, 2 H) 4.49 (m, 2 H) 4.57 (m, 1 H) 6.66 (d, *J*=4.0 Hz, 1 H) 6.81 (d, *J*=4.0 Hz, 1 H) 12.78 (m étalé, 1 H)
- 7.1.6: [(1*S*,2*R*)-1-(3,5-difluorobenzyl)-2-hydroxy-3-({1-[3-(trifluorométhyl)phényl]cyclo propyl}amino)propyl]carbamate de tert-butyle

A une solution de 0,794 g de chlorhydrate de 1-(3-trifluorométhyl-phényl)-cyclopropylamine dans 20 cm³ d'eau sont ajoutés 4 cm³ de soude 1 M à une température proche de 20°C. Cette solution est agitée 15 min. 40 cm³ de dichlorométhane sont ajoutés et le mélange est agité pendant 5 min. La phase aqueuse est extraite par 30 cm³ de dichlorométhane. Les phases organiques sont réunies, séchées sur sulfate de magnésium et concentrées à l'évaporateur rotatif sous pression réduite (5 kPa). On obtient 700 mg de 1-(3-trifluoromethyl-phenyl)-cyclopropylamine qui sont solubilisés dans 12 cm³ de dichlorométhane. 1 g de [(1S)-2-(3,5-difluorophényl)-1-oxiran-2-yléthyl]carbamate de tert-butyle et 0,329 g de triflate de scandium sont ajoutés à la solution. La légère suspension orangée est maintenue sous agitation à température ambiante pendant 20 h. Le mélange réactionnel est concentré à l'évaporateur rotatif sous pression réduite (5 kPa). Le produit brut obtenu est purifié par chromatographie flash sur silice (colonne : 200 g ; granulométrie : 15-40 µm ; débit: 50 cm³/min ; éluant : cyclohexane 70%-acétate d'éthyle 30%). Après concentration des fractions sous pression réduite, on obtient 0,87 g de [(1*S,*2*R*)-1-(3,5-difluorobenzyl)-2-hydroxy-3-({1-[3-(trifluorométhyl)phényl]cyclopropyl}amino)propyl]carbamate de tert-butyle sous forme d'un solide blanc.
- LC-MS-DAD-ELSD: 501⁽⁺⁾ = (M+H)⁽⁺⁾; 445⁽⁺⁾ = (M+H)⁽⁺⁾-tBu+H
- RMN : 0.90 - 1.04 (m, 4 H) 1.22 (s, 9 H) 2.40 (m, 1 H) 2.46 - 2.62 (m partiellement masqué, 2 H) 2.99 (dd, *J*=14.1, 3.2 Hz, 1 H) 3.36 (m, 1 H) 3.52 (m, 1 H) 4.80 (d, *J*=5.9 Hz, 1 H) 6.66 (d, *J*=9.3 Hz, 1 H) 6.86 (m, 2 H) 6.99 (tt, *J*=9.2, 2.4 Hz, 1 H) 7.48 - 7.61 (m, 3 H) 7.65 (s large, 1 H)

### 7.1.7: Chlorhydrate de (1:1) de (2R,3S)-3-amino-4-(3,5-difluorophényl)-1-({1-[3-(trifluorométhyl) phényl]cyclopropyl}amino)butan-2-ol

A une température de 20°C, 870 mg de [(1*S,*2*R*)-1-(3,5-difluorobenzyl)-2-hydroxy-3-({1-[3-(trifluorométhyl)phényl]cyclopropyl}amino)propyl]carbamate de tert-butyle sont dissous dans 20 cm³ de dichlorométhane. 17,38 cm³ d'une solution d'acide chlorhydrique 1 M dans l'éther éthylique sont ajoutés au mélange réactionnel qui est maintenu sous agitation 20 h à température ambiante puis concentré à l'évaporateur rotatif sous pression réduite (5 kPa). On obtient 0,82 g de chlorhydrate (1 :1) de (2*R,*3*S*)-3-amino-4-(3,5-difluorophényl)-1-({1-[3-(trifluorométhyl)phényl]cyclopropyl}amino)butan-2-ol sous forme de solide blanc.
- LC-MS-DAD-ELSD: 401⁽⁺⁾ = (M+H)⁽⁺⁾
- RMN : Pour ce lot, tous les signaux sont larges avec : 1.13 - 1.37 (m, 2 H) 1.53 - 1.71 (m, 2 H) 2.71 (m, 1 H) 2.84 (dd, *J*=14.3, 8.2 Hz, 1 H) 2.93 (dd, *J*=14.3, 5.9 Hz, 1 H) 3.09 (m, 1 H) 3.57 (m, 1 H) 4.18 (m, 1 H) 6.19 (m, 1 H) 7.01 - 7.10 (m, 3 H) 7.65 (t, *J*=7.8 Hz, 1 H) 7.77 (d, *J*=7.8 Hz, 1 H) 7.87 (d, *J*=7.8 Hz, 1 H) 7.98 (s, 1 H) 8.19 (s, 3 H) 9.77 (m, 1 H) 10.23 (m, 1 H)

### 7.1.8: N-[(1S,2R)-1-(3,5-difluorobenzyl)-2-hydroxy-3-({1-[3-(trifluorométhyl)phényl] cyclopropyl}amino)propyl]-1-oxo-2-(1-propylbutyl)-1,2,3,4-tétrahydropyrrolo[1,2a] pyrazine-6-carboxamide

A une suspension de 80 mg d'acide 1-oxo-2-(1-propylbutyl)-1,2,3,4-tétrahydropyrrolo[1,2-a]pyrazine-6-carboxylique, 136 mg de chlorhydrate (1 :1) de (2*R,*3*S*)-3-amino-4-(3,5-difluorophényl)-1-({1-[3-(trifluorométhyl)phényl]cyclopropyl}amino)butan-2-ol, 6 mg d'hydroxybenzotriazole, 69 mg de chlorhydrate de 1-(3-diméthylaminopropyl)-3-éthylcarbodiimide dans 10 cm³ de dichlorométhane est coulé 0,246 cm³ de *N,N-*diisopropyléthylamine à une température proche de 20°C. La solution est maintenue sous agitation 20 h à cette température. 30 cm³ de dichlorométhane et 15 cm³ d'eau sont ajoutés au milieu réactionnel. La phase organique est lavée avec 10 cm³ de solution aqueuse saturée en chlorure de sodium, séchée sur sulfate de sodium et concentrée à l'évaporateur rotatif sous pression réduite (5 kPa). Les 310 mg d'huile brune obtenus sont purifiés par chromatographie flash sur silice (colonne : 35 g ; granulométrie : 20-40µm sphérique; éluant : acétate d'éthyle 100%). Après concentration des fractions sous pression réduite, on obtient 116 mg de *N-*[(1*S,*2*R*)-1-(3,5-difluorobenzyl)-2-hydroxy-3-({1-[3(trifluorométhyl)phényl]cyclopropyl}amino)propyl]-1-oxo-2-(1-propylbutyl)-1,2,3,4-tétrahydropyrrolo[1,2*-a*]pyrazine-6-carboxamide.
- LC-MS-DAD-ELSD: 661⁽⁺⁾ = (M+H)⁽⁺⁾
- RMN: 0.86 (t, *J*=7.1 Hz, 3 H) 0.87 (t, *J*=7.1 Hz, 3 H) 0.92 - 1.54 (m, 12 H) 2.44 - 2.72 (m partiellement masqué, 3 H) 2.76 (dd, *J*=13.9, 10.6 Hz, 1 H) 3.06 (dd, *J*=13.9, 3.4 Hz, 1 H) 3.30 - 3.42 (m masqué, 2 H) 3.55 (m, 1 H) 4.08 (m, 1 H) 4.26 (m, 1 H) 4.35 (m, 1 H) 4.56 (m, 1 H) 4.98 (d, *J*=6.0 Hz, 1 H) 6.62 (d, *J*=4.1 Hz; 1 H) 6.77 (d, *J*=4.1 Hz, 1 H) 6.94 (m, 2 H) 6.99 (tt, *J*=9.2, 2.0 Hz, 1 H) 7.43 - 7.51 (m, 2 H) 7.55 (d large, *J*=7.6 Hz, 1 H) 7.66 (s large, 1 H) 8.06 (d, *J*=9.0 Hz, 1 H)

### 7.2: Sel

### Chlorhydrate (1:1) de N-[(1S,2R)-1-(3,5-difluorobenzyl)-2-hydroxy-3-({1-[3-(trifluorométhyl)phényl]cyclopropyl}amino)propyl]-1-oxo-2-(1-propylbutyl)-1,2,3,4-tétra hydropyrrolo[1,2-a]pyrazine-6-carboxamide

A une température proche de 20°C, 112 mg de *N-*[(1*S,*2*R*)-1-(3,5-difluorobenzyl)-2-hydroxy-3-({1-[3-(trifluorométhyl)phényl]cyclopropyl}amino)propyl]-1-oxo-2-(1-propyl butyl)-1,2,3,4-tétrahydropyrrolo[1,2*-a*]pyrazine-6-carboxamide sont dissous dans 1,5 cm³ d'éther éthylique. 0,43 cm³ d'une solution d'acide chlorhydrique 2 M dans l'éther éthylique est ajouté en agitant sous argon, à une température de 5°C. Le mélange réactionnel précipite. L'agitation est maintenue 20 min puis est arrêtée pour ôter le surnageant. 3 cm³ d'éther éthylique sont ajoutés de nouveau puis ôtés. Cette opération est effectuée 2 fois. La dernière suspension est concentrée sous pression réduite (5 kPa). On obtient 117 mg de chlorhydrate (1:1) de *N-*[(1*S,*2*R*)-1-(3,5-difluorobenzyl)-2-hydroxy-3-({1-[3-(trifluorométhyl)phényl]cyclopropyl}amino)propyl]-1-oxo-2-(1-propylbutyl)-1,2,3,4-tétrahydropyrrolo[1,2*-a*]pyrazine-6-carboxamide sous forme de solide blanc.
- PF: 183°C.
- RMN : 0,85 (t, J = 7,5 Hz, 3H) ; 0,87 (t, J = 7,5 Hz, 3H) ; de 1,12 à 1,65 (m, 12H) ; 2,72 (m, 1H); 2,79 (m, 1H); 2,98 (m, 1H); 3,08 (dd, J = 3,0 et 14,0 Hz, 1H); 3,40 (m partiellement masqué, 2H) ; 3,84 (m, 1H) ; 3,98 (m, 1H) ; 4,20 (m, 1H) ; 4,35 (m, 1H) ; 4,56 (m, 1H) ; 5,83 (m très étalé, 1H) ; 6,62 (d, J = 4,0 Hz, 1H) ; 6,74 (d, J = 4,0 Hz, 1H) ; de 6,90 à 7,02 (m, 3H) ; 7,59 (t, J= 7,5 Hz, 1H) ; 7,71 (d, J = 7,5 Hz, 1H) : 7,88 (d, J = 7,5 Hz, 1H) ; 7,97 (s, 1H) ; 8,16 (d, J = 9,0 Hz, 1H) ; 9,35 (m étalé, 1H) ; 9,62 (m étalé, 1H).
- LC-MS-DAD-ELSD: 659⁽⁻⁾ = (M-H)⁽⁻⁾; 661⁽⁺⁾ = (M+H)⁽⁺⁾

### Exemple 8:

### 8.1: Base

### N-[(1S,2R)-1-Benzyl-2-hydroxy-3-({1-[3-(trifluorométhyl)phényl]cyclopropyl}amino)propyl] -1-oxo-2-(1-propylbutyl)-1,2,3,4-tétrahydropyrrolo[1,2-a]pyrazine-6-carboxamide

A une suspension de 50 mg d'acide 1-oxo-2-(1-propylbutyl)-1,2,3,4-tétrahydropyrrolo[1,2-a]pyrazine-6-carboxylique, 85 mg de chlorhydrate de (2*R,*3*S*)-3-amino-4-phényl-1-({1-[3-(trifluorométhyl)phényl] cyclopropyl}amino)butan-2-ol, 3,6 mg d'hydroxybenzotriazole, 43 mg de chlorhydrate de 1-(3-diméthylaminopropyl)-3-éthylcarbodiimide dans 8 cm³ de dichlorométhane sont coulés 0,185 cm³ de *N,N*-diisopropyléthylamine à une température proche de 20°C. La solution obtenue est maintenue sous agitation 20 h. 20 cm³ de dichlorométhane et 10 cm³ d'eau sont ajoutés au milieu réactionnel. La phase organique est lavée avec 10 cm³ de solution aqueuse saturée en chlorure de sodium, séchée sur sulfate de sodium et concentrée à l'évaporateur rotatif sous pression réduite (5 kPa). Les 170 mg d'huile obtenus sont purifiés par chromatographie flash sur silice (colonne : 15 g ; granulométrie: 20-40 µm sphérique; éluant: gradient dichlorométhane 100% à dichlorométhane 95%- méthanol 5%). Après concentration des fractions sous pression réduite, on obtient 90 mg de *N-*[(1*S,*2*R*)-1-benzyl-2-hydroxy-3-({1-[3-(trifluorométhyl)phényl]cyclopropyl}amino)propyl]-1-oxo-2-(1-propylbutyl)-1,2,3,4-tétrahydropyrrolo[1,2*-a*]pyrazine-6-carboxamide.
- LC-MS-DAD-ELSD: 623⁽⁻⁾ = (M-H)⁽⁻⁾; 669⁽⁻⁾ = (M+Acide formique-H)⁽⁻⁾; 625⁽⁺⁾ = (M+H)⁽⁺⁾
- RMN :0.84 (t, *J*=7.3 Hz, 3 H) 0.86 (t, *J*=7.3 Hz, 3 H) 0.90 - 1.54 (m, 12 H) 2.45 - 2.60 (m partiellement masqué, 2 H) 2.71 (dd, *J*=13.9, 10.5 Hz, 1 H) 3.04 (dd, *J*=13.9, 3.6 Hz, 1 H) 3.17 - 3.47 (m partiellement masqué, 3 H) 3.54 (m, 1 H) 4.03 (m, 1 H) 4.23 (m, 1 H) 4.33 (m, 1 H) 4.54 (m, 1 H) 4.86 (d, *J*=6.1 Hz, 1 H) 6.59 (d, *J*=4.1 Hz, 1 H) 6.73 (d, *J*=4.1 Hz, 1 H) 7.12 (m, 1 H) 7.21 (m, 4 H) 7.42 - 7.59 (m, 3 H) 7.64 (s large, 1 H) 8.01 (d, *J*=9.0 Hz, 1 H).

### 8.2: Sel

### Chlorhydrate (1:1) de N-[(1S,2R)-1-benzyl-2-hydroxy-3-({1-[3-(trifluorométhyl)phényl] cyclopropyl}amino)propyl]-1-oxo-2-(1-propylbutyl)-1,2,3,4-tétrahydropyrrolo[1,2-a] pyrazine-6-carboxamide

A une température proche de 20°C, 85 mg de *N-*[(1*S,*2*R*)-1-benzyl-2-hydroxy-3-({1-[3-(trifluorométhyl)phényl]cyclopropyl}amino)propyl]-1-oxo-2-(1-propylbutyl)-1,2,3,4-tétra hydropyrrolo[1,2*-a*]pyrazine-6-carboxamide sont dissous dans 1 cm³ d'éther éthylique. 0,4 cm³ d'une solution d'acide chlorhydrique 2 M dans l'éther éthylique sont ajoutés en agitant sous argon, à une température de 5°C. Le mélange réactionnel précipite. L'agitation est maintenue 15 min puis est arrêtée pour ôter le surnageant. 3 cm³ d'éther éthylique sont ajoutés de nouveau puis ôtés. Cette opération est effectuée 3 fois. La dernière suspension est alors concentrée sous pression réduite (5 kPa). On obtient 87 mg de chlorhydrate (1 :1) de *N-*[(1*S,*2*R*)-1-benzyl-2-hydroxy-3-({1-[3-(trifluorométhyl) phényl]cyclopropyl}amino)propyl]-1-oxo-2-(1-propylbutyl)-1,2, 3,4-tétrahydropyrrolo[1,2a] pyrazine-6-carboxamide sous forme de solide blanc.
- PF: 176°C.
- RMN : 0,84 (t, J = 7,5 Hz, 3H) ; 0,87 (t, J = 7,5 Hz, 3H) ; de 1,11 à 1,62 (m, 12H) ; 2,74 (m, 2H) ; 2,95 (m, 1H) ; 3,09 (dd, J = 3,0 et 14,0 Hz, 1H) ; 3,38 (m partiellement masqué, 2H) ; 3,83 (m, 1H) ; 3,94 (m, 1H) ; 4,17 (m, 1H) ; 4,34 (m, 1H) ; 4,55 (m, 1H) ; 5,82 (m très étalé, 1 H) ; 6,61 (d, J = 4,0 Hz, 1H) ; 6,72 (d, J = 4,0 Hz, 1H) ; 7,11 (m, 1H) ; 7,20 (m, 4H) ; 7,58 (t, J= 7,5 Hz, 1H) ; 7,71 (d, J = 7,5 Hz, 1H) ; 7,87 (d, J = 7,5 Hz, 1H) ; 7,96 (s, 1H) ; 8,14 (d, J = 9,0 Hz, 1H) ; 9,33 (m étalé, 1H) ; 9,61 (m étalé, 1H) .
- LC-MS-DAD-ELSD: 623⁽⁻⁾ = (M-H)⁽⁻⁾; 625⁽⁺⁾ = (M+H)⁽⁺⁾

### Exemple 9:

### 9.1 : Base

### N-[(1S,2R)-1-Benzyl-2-hydroxy-3-{[3-(trifluorométhyl)benzyl]amino}propyl]-1-oxo-2-(1-propylbutyl)-1,2,3,4-tétrahydropyrrolo[1,2-a]pyrazine-6-carboxamide

### 9.1.1 :[(1S,2R)-1-benzyl-2-hydroxy-3-{[3-(trifluorométhyl)benzyl]amino}propyl]carbamate de tert-butyle

A une suspension sous argon de 10 g de [S-(*R,R*)]-(-)-(1-oxiranyl-2-phényléthyl)carbamate de tert-butyle dans 100 cm³ de 2-propanol, est coulée en 20 min une solution de 6,5 cm³ de 3-(trifluoromethyl)benzylamine dans 20 cm³ de 2-propanol à une température proche de 20°C. Le mélange réactionnel est chauffé à une température de 65°C pendant 20 h. II est ensuite refroidi pour être concentré à l'évaporateur rotatif sous pression réduite (5 kPa). Les 20 g d'huile incolore obtenus sont purifiés par chromatographie flash sur silice (colonne : 400g ; granulométrie : 15-40µm; débit : 20 cm³/min ; éluant : gradient de dichlorométhane 100% à dichlorométhane 95%-méthanol 5% en 140 mn). Après concentration des fractions sous pression réduite, on obtient 11 g de [(1*S,*2*R*)-1-benzyl-2-hydroxy-3-{[3-(trifluorométhyl)benzyl]amino}propyl]carbamate de tert-butyle sous forme d'un solide blanc.
- LC-MS-DAD-ELSD: 439⁽⁺⁾=(M+H)⁽⁺⁾; 483⁽⁻⁾=(M+Acide formique-H)⁽⁻⁾
- RMN : Pour ce lot, tous les signaux sont larges avec :1.22 (s, 9 H) 2.25 (m, 1 H) 2.38 - 2.65 (m partiellement masqué, 3 H) 2.99 (m, 1 H) 3.51 - 3.65 (m, 2 H) 3.80 (m, 2 H) 4.83 (d, J=6.3 Hz, 1 H) 6.61 (d, *J*=9.1 Hz, 1 H) 6.99 - 7.30 (m, 5 H) 7.48 - 7.80 (m, 4 H).

### 9.1.2: Chlorhydrate (1:1) de (2R,3S)-3-amino-4-phényl-1-{[3-(trifluorométhyl)benzyl] amino}butan-2-ol

11,2 g de [(1*S,*2*R*)-1-benzyl-2-hydroxy-3-{[3-(trifluorométhyl)benzyl]amino}propyl] carbamate de tert-butyle sont dissous dans 111 cm³ de dichlorométhane à une température proche de 20°C. 63 cm³ d'une solution d'acide chlorhydrique 4 M dans du dioxanne sont ajoutés à une température de 0°C. Le mélange réactionnel précipite. II est maintenu sous agitation 2 h à 20°C. Le précipité est filtré, lavé avec 4 fois 30 cm³ de dichlorométhane et 3 fois 30 cm³ d'éther de diisopropyle. Le solide blanc obtenu est séché dans un dessiccateur pendant 2 jours. On obtient 10,5 g de chlorhydrate de (2*R,*3*S*)-3-amino-4-phényl-1-{[3-(trifluorométhyl)benzyl]amino}butan-2-ol.
- LC-MS-DAD-ELSD: 339⁽⁺⁾=(M+H)⁽⁺⁾
- RMN : 2.79 - 2.96 (m, 3 H) 3.13 (m, 1 H) 3.46 - 3.59 (m partiellement masqué, 1 H) 4.16 - 4.30 (m, 3 H) 6.30 (m large, 1 H) 7.21 - 7.36 (m, 5 H) 7.67 (t, *J*=7.8 Hz, 1 H) 7.79 (d, *J*=7.8 Hz, 1 H) 7.87 (d, *J*=7.8 Hz, 1 H) 8.00 (s, 1 H) 8.21 (s large, 3 H) 9.41 (m large, 1 H) 9.72 (m large, 1 H).

### 9.1.3: (2R,3S)-3-amino-4-phényl-1-{[3-(trifluorométhyl)benzyl]amino}butan-2-ol

A une solution de 0,6 g de chlorhydrate (1 :1) de (2*R,*3*S*)-3-amino-4-phényl-1-{[3-(trifluorométhyl)benzyl]amino}butan-2-ol dans 30 cm³ de dichlorométhane sont ajoutés 10 cm³ d'eau et 2,9 cm³ de soude 1 M à une température proche de 20°C. Le mélange réactionnel est maintenu 1 h sous agitation. La phase organique est séchée sur sulfate de magnésium puis filtrée. Le filtrat est évaporé sous pression réduite (5 kPa). On obtient 360 mg de (2*R,*3*S*)-3-amino-4-phényl-1-{[3-(trifluorométhyl)benzyl]amino}butan-2-ol sous forme d'une huile incolore qui cristallise.
- RMN : 1.67 (m large, 3 H) 2.35 (dd, *J*=12.5, 8.1 Hz, 1 H) 2.56 (dd, *J*=11.9, 7.4 Hz, 1 H) 2.68 (dd, *J*=12.0, 3.7 Hz, 1 H) 2.76 - 2.89 (m, 2 H) 3.38 (m partiellement masqué, 1 H) 3.80 (s, 2 H) 4.65 (s large, 1 H) 7.11 - 7.22 (m, 3 H) 7.24 - 7.30 (m, 2 H) 7.52 - 7.61 (m, 2 H) 7.64 (d large, *J*=7.6 Hz, 1 H) 7.70 (s large, 1 H)
- LC-MS-DAD-ELSD: Tr (mn) = 2,23 ; MH⁽⁺⁾ = 339⁽⁺⁾ ; Pureté : 95 %

### 9.1.4 : N-[(1S,2R)-1-Benzyl-2-hydroxy-3-{[3-(trifluorométhyl)benzyl]amino}propyl]-1-oxo-2-(1-propylbutyl)-1,2,3,4-tétrahydropyrrolo[1,2-a]pyrazine-6-carboxamide

A une suspension de 230 mg d'acide 1-oxo-2-(1-propylbutyl)-1,2,3,4-tétrahydropyrrolo[1,2*-a*]pyrazine-6-carboxylique, 340 mg de chlorhydrate de (2*R,*3*S*)-3-amino-4-phényl-1-{[3-(trifluorométhyl)benzyl]amino}butan-2-ol, 17 mg d'hydroxybenzotriazole, 198 mg de chlorhydrate de 1-(3-diméthylaminopropyl)-3-éthylcarbodiimide dans 23 cm³ de dichlorométhane sont coulés 0,566 cm³ de *N,N*-diisopropyléthylamine. La solution est maintenue sous agitation 24 h à 20°C. 15 cm³ d'eau sont ajoutés au milieu réactionnel. La phase aqueuse est extraite par 15 cm³ de dichlorométhane. Les phases organiques sont réunies, lavées avec 10 cm³ de solution aqueuse saturée en chlorure de sodium, séchées sur sulfate de sodium et concentrées à l'évaporateur rotatif sous pression réduite (5 kPa). Les 840 mg de produit obtenus sont purifiés par chromatographie flash sur silice (colonne : 35 g ; granulométrie : 20-40 µm sphérique; éluant : gradient dichlorométhane 100% à dichlorométhane 95%-méthanol 5%). Après concentration des fractions sous pression réduite, on obtient 237 mg de *N-*[(1*S,*2*R*)-1-benzyl-2-hydroxy-3-{[3-(trifluorométhyl)benzyl]amino}propyl]-1-oxo-2-(1-propylbutyl)-1,2,3,4-tétrahydropyrrolo[1,2*-a*]pyrazine-6-carboxamide sous forme d'une meringue.
- LC-MS-DAD-ELSD: 599⁽⁺⁾ = (M+H)⁽⁺⁾
- RMN : 0.84 (t, *J*=7.3 Hz, 3 H) 0.85 (t, *J*=7.3 Hz, 3 H) 1.10 - 1.28 (m, 4 H) 1.36 (m, 2 H) 1.46 (m, 2 H) 2.55 (m partiellement masqué, 1 H) 2.65 (m, 1 H) 2.74 (dd, *J*=13.8, 10.7 Hz, 1 H) 3.05 (dd, *J*=13.7, 3.8 Hz, 1 H) 3.37 (t, *J*=5.9 Hz, 2 H) 3.62 (m large, 1 H) 3.80 (s, 2 H) 4.06 (m, 1 H) 4.21 - 4.39 (m, 2 H) 4.54 (m, 1 H) 4.97 (d large, *J*=5.0 Hz, 1 H) 6.58 (d, *J*=4.0 Hz, 1 H) 6.72 (d, *J*=4.0 Hz, 1 H) 7.12 (m, 1 H) 7.21 (m, 4 H) 7.50 (t, *J*=7.5 Hz, 1 H) 7.55 (d, *J*=7.5 Hz, 1 H) 7.62 (d, *J*=7.5 Hz, 1 H) 7.68 (s, 1 H) 8.10 (d, *J*=9.0 Hz, 1 H).

### 9.2: Sel

### Chlorhydrate (1:1) de N-[(1S,2R)-1-benzyl-2-hydroxy-3-{[3-(trifluorométhyl)benzyl]amino} propyl]-1-oxo-2-(1-propylbutyl)-1,2,3,4-tétrahydropyrrolo[1,2-a]pyrazine-6-carboxamide

A une température proche de 20°C, 230 mg de *N-*[(1*S,*2*R*)-1-benzyl-2-hydroxy-3-{[3-(trifluorométhyl)benzyl]amino}propyl]-1-oxo-2-(1-propylbutyl)-1,2,3,4-tétrahydropyrrolo [1,2*-a*]pyrazine-6-carboxamide sont dissous dans 3 cm³ d'éther éthylique. 0,7 cm³ d'une solution d'acide chlorhydrique 4 M dans le dioxanne est ajouté en agitant sous argon, à une température de 5°C. Le mélange réactionnel précipite. L'agitation est maintenue 10 min puis est arrêtée pour ôter le surnageant. 5 cm³ d'éther éthylique sont ajoutés de nouveau. Cette opération est effectuée 3 fois. La dernière suspension est alors concentrée sous pression réduite (5 kPa). On obtient 218 mg de chlorhydrate (1 :1) de *N-*[(1*S,*2*R*)-1-benzyl-2-hydroxy-3-{[3-(trifluorométhyl)benzyl]amino}propyl]-1-oxo-2-(1-propylbutyl)-1,2,3,4-tétrahydropyrrolo[1,2*-a*]pyrazine-6-carboxamide sous forme de solide beige.
- PF: 168°C.
- RMN : 0,84 (t, J = 7,5 Hz, 3H) ; 0,86 (t, J = 7,5 Hz, 3H) ; de 1,10 à 1,58 (m, 8H) ; 2,80 (dd, J = 11,0 et 14,0 Hz, 1H) ; 2,86 (m, 1H) ; 3,09 (m, 1H) ; 3,15 (dd, J = 3,5 et 14,0 Hz, 1H) ; 3,40 (m partiellement masqué, 2H) ; 3,88 (m, 1H) ; 4,00 (m, 1H) ; de 4,22 à 4,42 (m, 4H) ; 4,55 (m, 1H) ; 5,90 (m étalé, 1H) ; 6,62 (d, J = 4,0 Hz, 1H) ; 6,82 (d, J = 4,0 Hz, 1H) ; 7,13 (m, 1H) ; 7,23 (m, 4H) ; 7,65 (t, J= 7,5 Hz, 1H) ; 7,76 (d, J = 7,5 Hz, 1H) ; 7,86 (d, J = 7,5 Hz, 1H) ; 7,97 (s, 1H) ; 8,22 (d, J = 9,0 Hz, 1H) ; 8,99 (m étalé, 1H) ; 9,38 (m étalé, 1H).
- LC-MS-DAD-ELSD: 597⁽⁻⁾ = (M-H)⁽⁻⁾, 599⁽⁺⁾ = (M+H)⁽⁺⁾

### Exemple 10 :

### 10.1: Base

### N-[(1S,2R)-1-Benzyl-2-hydroxy-3-{[3-(trifluorométhyl)benzyl]amino}propyl]-2-(1-éthyl propyl)-1-oxo-1,2,3,4-tétrahydropyrrolo[1,2-a]pyrazine-6-carboxamide

### 10.1.1 : 1-{2-[(1-ethylpropyl)amino]éthyl}-1H-pyrrole-2-carboxylate d'éthyle

3,15 g de 1-(2-bromoéthyl)-1*H-*pyrrole-2-carboxylate d'éthyle, 6 cm³ de 3-pentylamine, 212 mg d'iodure de potassium et 63 cm³ d'acétonitrile sont agités à une température proche de 20°C sous atmosphère inerte pendant 72 h. Le mélange est ensuite chauffé 7 h à 70°C puis il est agité 48 h à une température proche de 20°C. Le mélange réactionnel est concentré à l'évaporateur rotatif sous pression réduite (5 kPa). 80 cm³ de dichlorométhane et 50 cm³ d'eau sont alors ajoutés au résidu de concentration. Les phases sont agitées 10 min puis sont séparées. La phase aqueuse est extraite par 2 fois 50 cm³ de dichlorométhane. Les phases organiques sont réunies, lavées avec 40 cm³ de solution aqueuse saturée en chlorure de sodium, séchées sur sulfate de magnésium et concentrées à l'évaporateur rotatif sous pression réduite (5 kPa). Les 2,85 g de produit obtenus sont purifiés par chromatographie flash sur silice (colonne: 200g ; granulométrie : 15-40 µm ; éluant : cyclohexane 50%-acétate d'éthyle 50%). Après concentration des fractions sous pression réduite, on obtient 1,66 g de 1-{2-[(1-éthylpropyl)amino]éthyl}-1*H-*pyrrole-2-carboxylate d'éthyle.
- RMN : 0,76 (t, J = 7,5 Hz, 6H) ; 1,26 (t, J = 7,5 Hz, 3H) ; 1,27 (m partiellement masqué, 4H) ; 1,64 (m, 1H) ; 2,25 (m, 1H) ; 2,77 (t, J = 6,5 Hz, 2H) ; 4,19 (q, J = 7,5 Hz, 2H) ; 4,30 (t, J = 6,5 Hz, 2H) ; 6,08 (dd, J = 2,5 et 4,0 Hz, 1H) ; 6,83 (dd, J = 2,0 et 4,0 Hz, 1H) ; 7,13 (t, J = 2,5 Hz, 1H).
- SM-EI : M⁽⁺⁾. = 252⁽⁺⁾. ; IC : (M+H)⁽⁺⁾ = 253⁽⁺⁾

### 10.1.2: 2-(1-Ethylpropyl)-3,4-dihydropyrrolo[1,2-a]pyrazin-1(2H)-one

A une température proche de 20°C, 1,6 g de 1-{2-[(1-éthylpropyl)amino]éthyl}-1*H-*pyrrole-2-carboxylate d'éthyle est dissous, sous atmosphère inerte, dans 60 cm³ de toluène. 6,35 cm³ de solution toluénique 2 M de triméthylaluminium sont coulés sur le mélange réactionnel en 5 mn. Le mélange réactionnel est chauffé sous agitation pendant 18 h à 100°C puis est refroidi à 20°C. II est ensuite versé sur un mélange glace-eau-acétate d'éthyle. La phase aqueuse est extraite par 3 fois 40 cm³ d'acétate d'éthyle. Les phases organiques sont réunies puis lavées avec 40 cm³ de solution aqueuse saturée en chlorure de sodium, séchées sur sulfate de magnésium et concentrées à l'évaporateur rotatif sous pression réduite (5 kPa). Le produit brut obtenu (1,55 g) est purifié par chromatographie flash sur silice (colonne : 70 g ; granulométrie : 15-40 µm ; éluant : cyclohexane 50%-acétate d'éthyle 50%). Après concentration des fractions sous pression réduite, on obtient 0,43 g de 2-(1-éthylpropyl)-3,4-dihydropyrrolo[1,2*-a*]pyrazin-1(2*H*)-one.
- SM-El: 206⁽⁺⁾ = M⁽⁺⁾
- RMN : 0.94 (t, *J*=7.5 Hz, 6 H) 1.39 - 1.56 (m, 4 H) 3.43 (m, 2 H) 4.12 (m, 2 H) 4.36 (m, 1 H) 6.13 (dd, *J*=3.8, 2.5 Hz, 1 H) 6.60 (dd, *J*=3.8, 1.5 Hz, 1 H) 6.95 (dd, *J*=2.5, 1.5 Hz,1 H)

### 10.1.3 : 6-bromo-2-(1-éthylpropyl)-3,4-dihydropyrrolo[1,2-a]pyrazin-1(2H)-one

A une température proche de 20°C, 0,42 g de 2-(1-éthylpropyl)-3,4-dihydropyrrolo[1,2-a]pyrazin-1 (2*H*)-one est dissous dans 42 cm³ de tétrachlorure de carbone 363 mg de *N-*bromosuccinimide sont ajoutés à la solution. L'agitation est maintenue 18 h à température ambiante. Le mélange réactionnel est concentré à l'évaporateur rotatif sous pression réduite (5 kPa). Le résidu solide obtenu est purifié par chromatographie flash sur silice (colonne : 90 g ; granulométrie : 15-40 µm ; éluant : dichlorométhane 90%-acétate d'éthyle 10%). Après concentration des fractions sous pression réduite, on obtient 0,5 g de 6-bromo-2-(1-éthylpropyl)-3,4-dihydropyrrolo[1,2*-a*]pyrazin-1(2*H*)-one.
- SM-El: 284^{(+) 79}Br = M⁽⁺⁾
- RMN : 0.79 (t, *J*=7.3 Hz, 6 H) 1.38 - 1.57 (m, 4 H) 3.49 (m, 2 H) 4.04 (m, 2 H) 4.33 (m, 1 H) 6.31 (d, *J*=3.9 Hz, 1 H) 6.69 (d, *J*=3.9 Hz, 1 H)

### 10.1.4: Acide 2-(1-éthylpropyl)-1-oxo-1,2,3,4-tétrahydropyrrolo[1,2-a]pyrazine-6-carboxylique

Dans un tricol agité et purgé au monoxyde de carbone, 490 mg de 6-bromo-2-(1-éthylpropyl)-3,4-dihydropyrrolo[1,2*-a*]pyrazin-1(2*H*)-one, 15 cm³ de diméthylformamide, 0,75 cm³ d'eau, 0,641 g d'acétate de potassium, 285 mg d'iodure de potassium, 39 mg d'acétate de palladium et 91 mg de triphénylphosphine sont introduits successivement à une température proche de 20°C. Le mélange réactionnel est soumis à un bullage de monoxyde de carbone puis est chauffé à 100°C pendant 6 h 30. Le mélange réactionnel est refroidi à 20°C et agité pendant 20 h. Le diméthylformamide est évaporé à l'évaporateur rotatif sous pression réduite (5 kPa). Le résidu huileux obtenu est repris dans 80 g de glace et 80 cm³ d'acétate d'éthyle. Le pH est alcalinisé avec 15 cm³ de soude 1 M. Après décantation, la phase aqueuse est lavée avec 2 fois 40 cm³ d'acétate d'éthyle. Elle est ensuite acidifiée sous agitation avec une solution d'acide chlorhydrique 5 M (pH=1) puis extraite par 3 fois 40 cm³ d'acétate d'éthyle. Les phases organiques sont réunies puis lavées avec 30 cm³ de solution aqueuse saturée en chlorure de sodium, séchées sur sulfate de magnésium et concentrées à l'évaporateur rotatif sous pression réduite (5 kPa). On obtient 0,31 g d'acide 2-(1-éthylpropyl)-1-oxo-1,2,3,4-tétrahydropyrrolo[1,2*-a*]pyrazine-6-carboxylique.
- SM-El: 250⁽⁺⁾ = M⁽⁺⁾
- RMN : 0.80 (t, *J*=7.4 Hz, 6 H) 1.35 - 1.60 (m, 4 H) 3.49 (m, 2 H) 4.35 (m, 1 H) 4.51 (m, 2 H) 6.67 (d, *J*=4.1 Hz, 1 H) 6.82 (d, *J*=4.1 Hz, 1 H) 12.55 (m étalé, 1 H)

### 10.1.5: N-[(1S,2R)-1-Benzyl-2-hydroxy-3-{[3-(trifluorométhyl)benzyl]amino}propyl]-2-(1-éthylpropyl)-1-oxo-1,2,3,4-tétrahydropyrrolo[1,2-a]pyrazine-6-carboxamide

A une suspension de 150 mg d'acide 2-(1-éthylpropyl)-1-oxo-1,2,3,4-tétrahydropyrrolo[1,2*-a*]pyrazine-6-carboxylique, 247 mg de chlorhydrate de (2*R,*3*S*)-3-amino-4-phényl-1-{[3-(trifluorométhyl)benzyl]amino}butan-2-ol, 12,2 mg d'hydroxy-benzotriazole, 144 mg de chlorhydrate de 1-(3-diméthylaminopropyl)-3-éthylcarbodiimide dans 15 cm³ de dichloromethane sont coulés 0,410 cm³ de *N,N*-diisopropyléthylamine à une température proche de 20°C. La solution est maintenue sous agitation 72 h. 30 cm³ de dichlorométhane et 15 cm³ d'eau sont ajoutés au milieu réactionnel. La phase aqueuse est extraite par 15 cm³ de dichlorométhane. Les phases organiques sont réunies, lavées avec 10 cm³ de solution aqueuse saturée en chlorure de sodium, séchées sur sulfate de sodium et concentrées à l'évaporateur rotatif sous pression réduite (5 kPa). Les 520 mg de produit obtenus sont purifiés par chromatographie flash sur silice (colonne : 70 g ; granulométrie : 20-40 µm sphérique; éluant : dichlorométhane 95% - methanol 5% puis dichlorométhane 90%-méthanol 10%). Après concentration des fractions sous pression réduite, on obtient 163 mg de *N-*[(1*S,*2*R*)-1-benzyl-2-hydroxy-3-{[3-(trifluorométhyl)benzyl]amino}propyl]-2-(1-éthylpropyl)-1-oxo-1,2,3,4-tétrahydropyrrolo[1,2*-a*]pyrazine-6-carboxamide.
- LC-MS-DAD-ELSD: 571⁽⁺⁾ = (M+H)⁽⁺⁾
- RMN : 0.77 (t large, *J*=7.3 Hz, 6 H) 1.37 - 1.56 (m, 4 H) 2.52 - 2.80 (m, 3 H) 3.05 (dd, *J*=13.9, 3.6 Hz, 1 H) 3.37 (t, *J*=6.0 Hz, 2 H) 3.62 (m large, 1 H) 3.80 (s, 2 H) 4.07 (m, 1 H) 4.22 - 4.44 (m, 3 H) 4.96 (d large, *J*=4.8 Hz, 1 H) 6.58 (d, *J*=4.1 Hz, 1 H) 6.72 (d, *J*=4.1
Hz, 1 H) 7.12 (m, 1 H) 7.21 (m, 4 H) 7.45 - 7.58 (m, 2 H) 7.62 (d large, *J*=7.6 Hz, 1 H) 7.69 (s large, 1 H) 8.09 (d, *J*=8.7 Hz, 1 H)

### 10.2: Sel

### Chlorhydrate (1:1) de N-[(1S,2R)-1-benzyl-2-hydroxy-3g{[3-(trifluorométhyl)benzyl] amino}propyl]-2-(1-éthylpropyl)-1-oxo-1,2,3,4-tétrahydropyrrolo[1,2-a]pyrazine-6-carboxamide

A une température proche de 20°C, 155 mg de *N-*[(1*S,*2*R)*-1-benzyl-2-hydroxy-3-{[3-(trifluorométhyl)benzyl]amino}propyl]-2-(1-éthylpropyl)-1-oxo-1,2,3,4-tétrahydropyrrolo [1,2*-a*]pyrazine-6-carboxamide sont dissous dans 1,5 cm³ d'éther éthylique. 0,5 cm³ d'une solution d'acide chlorhydrique 4 M dans le dioxanne est ajouté en agitant sous argon, à une température de 5°C. Le mélange réactionnel précipite. 5 cm³ d'éther éthylique sont ajoutés. L'agitation est maintenue 10 min puis est arrêtée pour ôter le surnageant. 2 cm³ d'éther éthylique sont ajoutés de nouveau puis ôtés. Cette opération est effectuée 3 fois. La dernière suspension est alors concentrée sous pression réduite (5 kPa). On obtient 162 mg de chlorhydrate (1 :1) de *N-*[(1*S,*2*R*)-1-benzyl-2-hydroxy-3-{[3-(trifluorométhyl)benzyl]amino}propyl]-2-(1-éthylpropyl)-1-oxo-1,2,3,4-tétrahydropyrrolo [1,2*-a*]pyrazine-6-carboxamide sous forme de solide blanc.
- PF: 178°C.
- RMN : 0,77 (t, J = 7,5 Hz, 3H) ; 0,78 (t, J = 7,5 Hz, 3H) ; 1,47 (m, 4H) ; 2,79 (dd, J = 11,0 et 14,0 Hz, 1H) ; 2,86 (m, 1H) ; 3,10 (m, 1 H) ; 3,15 (dd, J = 3,0 et 14,0 Hz, 1H) ; 3,41 (m partiellement masqué, 2H) ; 3,88 (m, 1H) ; 4,01 (m, 1 H) ; de 4,22 à 4,44 (m, 5H) ; 5,90 (m très étalé, 1H) ; 6,63 (d, J = 4,0 Hz, 1H) ; 6,82 (d, J = 4,0 Hz, 1H) ; 7,14 (m, 1H) ; 7,23 (m, 4H) ; 7,65 (t, J= 7,5 Hz, 1H) ; 7,76 (d, J = 7,5 Hz, 1H) ; 7,85 (d, J = 7,5 Hz, 1H) ; 7,97 (s, 1H) ; 8,21 (d, J = 9,0 Hz, 1H) ; 8,97 (m étalé, 1 H) ; 9,33 (m étalé, 1 H).
- LC-MS-DAD-ELSD: 569⁽⁻⁾=(M-H)⁽⁻⁾; 615⁽⁻⁾=(M+Ac formique-H)⁽⁻⁾; 571⁽⁺⁾=(M+H)⁽⁺⁾

### Exemple 11:

### 11.1 : Base N-[(1S,2R)-1-Benzyl-2-hydroxy-3-{[3-(trifluorométhyl)benzyl]amino}propyl]-1-oxo-2-(1-propylbutyl)-2,3,4,5-tétrahydro-1H-pyrrolo[1,2-a][1,4]diazépine-7-carboxamide

### 11.1.1 : 1-(3-bromopropyl)-1H-pyrrole-2-carboxylate d'éthyle

5,15 g d'éthyle pyrrole-2-carboxylate, 18 cm³ de 1,3-dibromopropane, 22 cm³ de soude concentrée et 11,93 g de bromure de tétrabutylammonium sont agités sous atmosphère inerte pendant 48 h à une température proche de 20°C. 80 cm³ de dichlorométhane et 80 cm³ d'eau sont ajoutés au milieu réactionnel. La phase aqueuse est extraite par 3 fois 40 cm³ de dichlorométhane. Les phases organiques sont réunies puis lavées avec 50 cm³ de solution aqueuse saturée en chlorure de sodium, séchées sur sulfate de magnésium et concentrées à l'évaporateur rotatif sous pression réduite (5 kPa). Les 50 g d'huile obtenus sont purifiés par chromatographie flash sur silice (colonne : 400 g ; granulométrie : 15-40 µm ; éluant : dichlorométhane 100%). Après concentration des fractions sous pression réduite, on obtient 9,8 g de 1-(3-bromopropyl)-1*H-*pyrrole-2-carboxylate d'éthyle.
- SM-EI: 259^{(+) 79}Br = M⁽⁺⁾
- 1H NMR (400 MHz, DMSO-*d₆*) d ppm 1.27 (t, *J*=7.1 Hz, 3 H) 2.22 (m, 2 H) 3.40 (t, *J*=6.7 Hz, 2 H) 4.21(q, *J*=7.1 Hz, 2 H) 4.38 (t, *J*=6.7 Hz, 2 H) 6.12 (dd, *J*=3.9, 2.5 Hz, 1 H) 6.87 (dd, *J*=3.9, 2.0 Hz, 1 H) 7.14(dd, *J*=2.5, 2.0 Hz, 1 H)

### 11.1.2:1-{3-[(1-propylbutyl)amino]propyl}-1H-pyrrole-2-carboxylate d'éthyle

7 g de 1-(3-bromopropyl)-1*H-*pyrrole-2-carboxylate d'éthyle, 9,3 g de 4-aminoheptane, 4,46 g d'iodure de potassium et 140 cm³ d'acétonitrile sont agités sous atmosphère inerte pendant 20 h à 70 °C puis refroidis à une température proche de 20°C. Le mélange réactionnel est concentré à l'évaporateur rotatif sous pression réduite (5 kPa). 100 cm³ de dichlorométhane et 100 cm³ d'eau sont alors ajoutés au résidu de concentration. La phase aqueuse est extraite par 3 fois 50 cm³ de dichlorométhane. Les phases organiques sont réunies, lavées avec 40 cm³ de solution aqueuse saturée en chlorure de sodium, séchées sur sulfate de magnésium et concentrées à l'évaporateur rotatif sous pression réduite (5 kPa). Le produit obtenu est purifié par chromatographie flash sur silice (colonne : 400 g ; granulométrie : 15-40 µm ; éluant : gradient heptane 75%-acétate d'éthyle 25% à 100% acétate d'éthyle). Après concentration des fractions sous pression réduite, on obtient 5,87 g de 1-{3-[(1-propylbutyl)amino]propyl}-1*H-*pyrrole-2-carboxylate d'éthyle.
- SM-EI: 294⁽⁺⁾ = M⁽⁺⁾; 251⁽⁺⁾ = M⁽⁺⁾ - C₃H₇
- 1 H NMR (300 MHz, DMSO-*d₆*) d ppm 0.85 (t, *J*=6.9 Hz, 6 H) 1.13 - 1.35 (m, 11 H) 1.76 (m, 2 H) 2.28 -2.44 (m, 3 H) 4.19 (q, *J*=7.1 Hz, 2 H) 4.32 (t, *J*=6.9 Hz, 2 H) 6.08 (dd, *J*=2.5, 1.8 Hz, 1 H) 6.83 (dd, *J*=3.9,1.8 Hz, 1 H) 7.10 (dd, *J*=2.5, 1.9 Hz, 1 H)

### 11.1.3: 2-(1-propylbutyl)-2,3,4,5-tétrahydro-1H-pyrrolo[1,2-a][1,4]diazépin-1-one

A une température proche de 20°C, 5,85 g de 1-{3-[(1-propylbutyl)amino]propyl}-1*H-*pyrrole-2-carboxylate d'éthyle sont dissous sous atmosphère inerte, dans 150 cm³ de toluène. 29,8 cm³ de solution toluénique 2M de triméthylaluminium sont coulés sur le mélange réactionnel en 5 min. Le mélange réactionnel est chauffé sous agitation pendant 20 h à 100°C puis est refroidi à une température proche de 20°C. II est ensuite versé sur 200 g de glace et 100 cm³ d'acétate d'éthyle. La suspension obtenue est filtrée sur culot de Célite 545. La phase aqueuse est extraite par 3 fois 40 cm³ d'acétate d'éthyle. Les phases organiques sont réunies puis lavées avec 40 cm³ d'eau, 40 cm³ de solution aqueuse saturée en chlorure de sodium, séchées sur sulfate de magnésium et concentrées à l'évaporateur rotatif sous pression réduite (5 kPa). Les 4,71 g de produit brut obtenus sont purifiés par chromatographie flash sur silice (colonne : 200 g ; granulométrie : 15-40 µm ; éluant : gradient de dichlorométhane 100% à dichlorométhane 80%-acétate d'éthyle 20%). Après concentration des fractions sous pression réduite, on obtient 2,97 g de 2-(1-propyl-butyl)-2,3,4,5-tétrahydro-pyrrolo[1,2*-a*][1,4]diazepin-1-one.
- SM-EI: 248⁽⁺⁾ = M⁽⁺⁾; 249⁽⁺⁾ = (M+H)⁽⁺⁾
- 1H NMR (300 MHz, DMSO-*d₆*) d ppm 0.88 (t, *J*=7.3 Hz, 6 H) 1.13 - 1.34 (m, 4 H) 1.35 - 1.58 (m, 4 H)1.98 (m, 2 H) 3.13 (t, *J*=6.7 Hz, 2 H) 4.11 (t, *J*=6.7 Hz, 2 H) 4.46 (m, 1 H) 6.02 (m, 1 H) 6.48 (m, 1 H)6.88 (s large, 1 H)

### 11.1.4: 7-bromo-2-(1-propylbutyl)-2,3,4,5-tétrahydro-1H-pyrrolo[1,2-a][1,4]diazépin-1-one

A une température proche de 20°C, 1,5 g de 2-(1-propylbutyl)-2,3,4,5-tétrahydro-1H pyrrolo[1,2*-a*][1,4]diazépin-1-one sont dissous dans 120 cm³ de tétrachlorure de carbone 1,075 g de *N-*bromosuccinimide est ajouté à la solution. L'agitation est maintenue 3 h. Le mélange réactionnel est concentré à l'évaporateur rotatif sous pression réduite (5 kPa). Le résidu solide obtenu est purifié par chromatographie flash sur silice (colonne : 200 g ; granulométrie : 15-40 µm ; éluant : dichlorométhane 90%-acétate d'éthyle 10%). Après concentration des fractions sous pression réduite, on obtient 1,92 g de 7-bromo-2-(1-propylbutyl)-2,3,4,5-tétrahydro-1*H-*pyrrolo[1,2*-a*][1,4]diazépin-1-one.
- SM-EI: 326^{(+) 79}Br = M⁽⁺⁾
- 1H NMR (300 MHz, DMSO-*d₆*) d ppm 0.88 (t, *J*=7.2 Hz, 6 H) 1.12 - 1.33 (m, 4 H) 1.35 - 1.56 (m, 4 H)1.97 (m, 2 H) 3.12 (t, *J*=6.7 Hz, 2 H) 4.11 (t, *J*=6.7 Hz, 2 H) 4.45 (m, 1 H) 6.23 (d, *J*=3.9 Hz, 1 H) 6.56 (d, *J*=3.9 Hz, 1 H)

### 11.1.5 : Acide 1-oxo-2-(1-propylbutyl)-2,3,4,5-tétrahydro-1H-pyrrolo[1,2-a][1,4]diazépine-7-carboxylique

Dans un tricol agité et purgé au monoxyde de carbone, 1 g de 7-bromo-2-(1-propylbutyl)-2,3,4,5-tétrahydro-1*H-*pyrrolo[1,2*-a*1][1,4]diazépin-1-one, 25 cm³ de diméthylformamide, 2,5 cm³ d'eau, 1,14 g d'acétate de potassium, 102 mg d'iodure de potassium, 275 mg d'acétate de palladium et 640 mg de triphénylphosphine sont introduits successivement à une température proche de 20°C. Le mélange réactionnel est soumis à un bullage de monoxyde de carbone puis est chauffé à 100°C pendant 7 h. Il est refroidi à 20°C pour être filtré sur culot de Célite 545. Le filtrat est évaporé à l'évaporateur rotatif sous pression réduite (5 kPa). Le résidu huileux obtenu est repris dans 40 g de glace et 40 cm³ d'acétate d'éthyle. Le pH est amené à 10 avec de la soude 5 M. Après décantation, la phase aqueuse est lavée avec 3 fois 40 cm³ d'acétate d'éthyle puis filtré sur un culot de Célite 545. Le filtrat est acidifié sous agitation avec une solution d'acide chlorhydrique 5 M (pH=1) puis extrait par 3 fois 40 cm³ d'acétate d'éthyle. Les phases organiques sont réunies puis lavées avec 30 cm³ de solution aqueuse saturée en chlorure de sodium, séchées sur sulfate de magnésium et concentrées à l'évaporateur rotatif sous pression réduite (5 kPa). Le produit obtenu (1 g) est purifié par chromatographie flash sur silice (colonne : 90 g; granulométrie : 15-40 µm ; éluant : dichlorométhane 95%- méthanol 5%). Après concentration des fractions sous pression réduite, on obtient 0,74 g d'acide 1-oxo-2-(1-propylbutyl)-2,3,4,5-tétrahydro-1H pyrrolo[1,2*-a*][1,4]diazépine-7-carboxylique.
- SM-El: 292⁽⁺⁾ = M⁽⁺⁾
- 1H NMR (400 MHz, DMSO-*d₆*) d ppm 0.88 (t, *J*=7.2 Hz, 6 H) 1.25 (m, 4 H) 1.46 (m, 4 H) 1.99 (m, 2H) 3.11 (t, *J*=6.7 Hz, 2 H) 4.51 (m, 1 H) 4.62 (t, *J*=6.7 Hz, 2 H) 6.47 (d, *J*=4.1 Hz, 1 H) 6.79 (d, *J*=4.1Hz, 1 H) 12.59 (m étalé, 1 H)

### 1.1.6 : N-[(1S,2R)-1-benzyl-2-hydroxy-3-{[3-(trifluorométhyl)benzyl]amino}propyl]-1-oxo-2-(1-propylbutyl)-2,3,4,5-tétrahydro-1H-pyrrolo[1,2-a][1,4]diazépine-7-carboxamide

A une suspension de 200 mg d'acide 1-oxo-2-(1-propylbutyl)-2,3,4,5-tétrahydro-1*H-*pyrrolo[1,2*-a*][1,4]diazépine-7-carboxylique, 281 mg de chlorhydrate (1 :1) de (2*R,*3*S*)-3-amino-4-phényl-1-{[3-(trifluorométhyl)benzyl]amino}butan-2-ol, 14 mg d'hydroxybenzotriazole, 164 mg de chlorhydrate de 1-(3-diméthylaminopropyl)-3-éthylcarbodiimide dans 25 cm³ de dichloromethane est coulé 0,468 cm³ de *N,N*-diisopropyléthylamine à une température proche de 20°C. La solution obtenue est maintenue sous agitation pendant 3 h 30 à 20°C sous atmosphère inerte. 25 cm³ de dichlorométhane et 15 cm³ d'eau sont ajoutés au milieu réactionnel. La phase organique est lavée avec 20 cm³ de solution aqueuse saturée en chlorure de sodium, séchée sur sulfate de sodium et concentrée à l'évaporateur rotatif sous pression réduite (5 kPa). Le produit obtenu est purifié par chromatographie flash sur silice (colonne : 15 g ; granulométrie : 20-40 µm sphérique; éluant : gradient dichlorométhane 95%- méthanol 5% à dichlorométhane 90%- méthanol 10%). Après concentration des fractions sous pression réduite, on obtient 206 mg de *N-*[(1*S,*2*R*)-1-benzyl-2-hydroxy-3-{[3-(trifluorométhyl)benzyl]amino}propyl]-1-oxo-2-(1-propylbutyl)-2,3,4,5-tétrahydro-1*H-*pyrrolo[1,2*-a*][1,4]diazépine-7-carboxamide.
- LC-MS-DAD-ELSD: 611⁽⁻⁾ = (M-H)⁽⁻⁾; 657⁽⁻⁾ = (M+Ac Formique-H)⁽⁻⁾; 613⁽⁺⁾ = (M+H)⁽⁺⁾
- 1H NMR (400 MHz, DMSO-*d₆*) d ppm 0.86 (t, *J*=7.3 Hz, 3 H) 0.88 (t, *J*=7.3 Hz, 3 H) 1.15 -1.30 (m, 4H) 1.34 - 1.51 (m, 4 H) 1.86 (m, 2 H) 2.46 - 2.77 (m partiellement masqué, 3 H) 2.93 - 3.14 (m, 3 H) 3.61 (m, 1 H) 3.73 - 3.86 (m, 2 H) 4.03 (m, 1 H) 4.22 (m, 1 H) 4.35 (m, 1 H) 4.47 (m, 1 H) 4.98 (d, *J*=5.9 Hz, 1 H) 6.39 (d, *J*=3.9 Hz, 1 H) 6.54 (d, *J*=3.9 Hz, 1 H) 7.12 (m, 1 H) 7.21 (m, 4 H) 7.47 - 7.58 (m, 2 H) 7.62 (d large, *J*=7.5 Hz, 1 H) 7.69 (s large, 1 H) 8.08 (d, *J*=9.0 Hz, 1 H)

### 11.2: Sel

### Chlorhydrate (1:1) de N-[(1S,2R)-1-benzyl-2-hydroxy-3-{[3-(trifluorométhyl)benzyl]amino} propyl]-1-oxo-2-(1-propylbutyl)-2,3,4,5-tétrahydro-1H-pyrrolo[1,2-a][1,4]diazépine-7-carboxamide

A une température proche de 20°C, 200 mg de *N-*[(1*S,*2*R*)-1-benzyl-2-hydroxy-3-{[3-(trifluorométhyl)benzyl]amino}propyl]-1-oxo-2-(1-propylbutyl)-2,3,4,5-tétrahydro-1*H-*pyrrolo[1,2*-a*][1,4]diazépine-7-carboxamide sont dissous dans 2,5 cm³ d'éther éthylique. 0,6 cm3 d'une solution d'acide chlorhydrique 4 M dans le dioxanne sont ajoutés en agitant sous argon, à une température de 5°C. Le mélange réactionnel précipite partiellement. La suspension est alors concentrée sous pression réduite (5 kPa). 3 cm³ d'éther éthylique sont ajoutés. La suspension est agitée pendant 15 min puis l'agitation est arrêtée et on ôte le surnageant. Cette opération est effectuée 2 fois. La dernière suspension est alors concentrée sous pression réduite (5 kPa). On obtient 183 mg de chlorhydrate (1 :1) de *N-*[(1*S,*2*R*)-1-benzyl-2-hydroxy-3-{[3-(trifluorométhyl)benzyl]amino} propyl]-1-oxo-2-(1-propylbutyl)-2,3,4,5-tétrahydro-1*H-*pyrrolo[1,2*-a*][1,4]diazépine-7-carboxamide sous forme de solide blanc.
- 1H NMR (400 MHz, DMSO-*d₆*) d ppm 0,86 (t, J = 7,5 Hz, 3H) ; 0,88 (t, J = 7,5 Hz, 3H) ; de 1,15 à 1,30 (m, 4H) ; 1,44 (m, 4H) ; 1,88 (m, 2H) ; 2,77 (dd, J = 11,0 et 14,0 Hz, 1H) ; 2,87 (m, 1H) ; de 2,96 à 3,17 (m, 4H) ; 3,87 (m, 1H) ; 3,97 (m, 1H) ; 4,17 (m, 1H) ; 4,31 (m, 2H) ; de 4,41 à 4,53 (m, 2H) ; 5,92 (m étalé, 1H) ; 6,44 (d, J = 4,0 Hz, 1H) ; 6,62 (d, J = 4,0 Hz, 1H) ; 7,14 (m, 1H) ; 7,24 (m, 4H) ; 7,65 (t, J = 7,5 Hz, 1H) ; 7,76 (d, J = 7,5 Hz, 1H) ; 7,86 (d, J = 7,5 Hz, 1H) ; 7,96 (s, 1H) ; 8,18 (d, J = 9,0 Hz, 1H) ; 8,97 (m étalé, 1H) ; 9,32 (m étalé, 1H).
- LC-MS-DAD-ELSD: 611⁽⁻⁾ = (M-H)⁽⁻⁾; 657⁽⁻⁾ = (M+Ac Formique-H)⁽⁻⁾; 613⁽⁺⁾ = (M+H)⁽⁺⁾

Le tableau 1 qui suit illustre les structures chimiques et les propriétés physiques de quelques exemples de composés selon l'invention. Dans ce tableau :
- PF (°C) représente le point de fusion du composé en degrés Celsius,
- dans la colonne « sel », « - » représente un composé sous forme de base libre, alors que « HCl » représente un composé sous forme de chlorhydrate, le rapport entre parenthèses est le rapport (acide : base) ;
- R3 représente un groupe trifluorométhyle ;
- Me et Et représentent respectivement des groupes méthyle et éthyle ;
- « nd » : non déterminé.

Les composés décrits dans ce tableau ont été préparés selon les méthodes décrites précédemment.

**Tableau 1 :**

| | | | | | | | | |
|---|---|---|---|---|---|---|---|---|
| 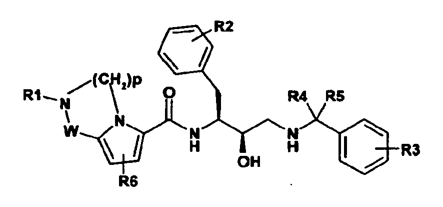 | | | | | | | | |

| Composé | R1 | W | ρ | R2 | R4, R5 | R6 | PF (°C) | Sel |
|---|---|---|---|---|---|---|---|---|
| 1 | BOC- | -CH₂- | 2 | H- | -CH₂CH₂- | 8-COOMe | 136 | - |
| 2 | BOC- | -CH₂- | 2 | H- | -CH₂CH₂- | 8-COOH | 134 ,5 | - |
| 3 | H- | -CH₂- | 2 | H- | -CH₂CH₂- | 8-COOH | 196 | HCl (2:1) |
| 4 | BOC- | -CH₂- | 2 | H- | -CH₂CH₂- | 8-CON(Et)₂ | nd^{(a)} | - |
| 5 | H- | -CH₂- | 2 | H- | -CH₂CH₂- | 8-CON(Et)₂ | 143 | HCl (2 :1) |
| 6 | MeSO₂- | -CH₂- | 2 | H- | -CH₂CH₂₋ | 8-CON(Et)₂ | nd^{(b)} | - |
| 7 | (n-C₃H₇)₂CH- | C=O | 2 | 3,5-diF | -CH₂CH₂- | H- | 183 | HCl (1:1) |
| 8 | (n-C₃H₇)₂CH- | C=O | 2 | H- | -CH₂CH₂- | H- | 176 | HCl (1:1) |
| 9 | (n-C₃H₇)₂CH- | C=O | 2 | H- | H-, H- | H- | 168 | HCl (1:1) |
| 10 | Et₂CH- | C=O | 2 | H- | H-, H- | H- | 178 | HCl (1 :1) |
| 11 | Et₂CH- | C=O | 3 | H- | H-, H- | H- | nd^{(a)} | HCl (1 :1) |

| | | | | | | | | |
|---|---|---|---|---|---|---|---|---|
| (a) caractérisé par un spectre RMN-¹H et par une chromatographie liquide couplée à un spectromètre de masse (b) caractérisé par un spectre RMN-¹H | | | | | | | | |

Les composés selon l'invention ont fait l'objet d'essais pharmacologiques permettant de déterminer leur effet inhibiteur vis-à-vis de l'activité β-secrétase.

Des essais ont consisté à mesurer l'inhibition *in vitro* de l'activité β-secrétage par les composés de l'invention.

L'activité β sécrétase mesurée correspond à celle d'une forme recombinante purifiée de l'aspartyl-protéase BACE1 humaine (cette dernière comportant un tag hexa-histidine en C-terminal) produite par expression en cellules de Drosophile. L'enzyme purifiée est conditionnée dans du tampon TRIS (18 mM) à pH 7,5 contenant NaCl (0,45 M), MnCl₂ (0,9 mM), CaCl₂ (0,9 mM), alpha D methylmannoside 10% glycérol, et conservée à -80°C jusqu'à utilisation.

L'activité BACE1 est mesurée d'après le clivage d'un substrat peptidique fluorogénique, appelé FS1, décrit à l'origine par Ermolieff et Coll. (2000, Biochemistry, 39, 12450-12456), et basé sur le principe du transfert d'énergie de fluorescence par résonance (FRET); le clivage du peptide FS1 est mesuré d'après l'augmentation du signal fluorescent émis par le groupe EDANS (ou 5-[(2-aminoethyl)amino]-naphthalene-1-sulfonic acid).

L'essai est pratiqué en microplaque 96 puits pour déterminer l'inhibition de l'activité enzymatique par les produits de l'invention. Le substrat FS1 est solubilisé à une concentration de 1 mM dans 100% diméthylsulfoxide (DMSO) et stocké à -20°C jusqu'à utilisation. Les dilutions des produits à tester sont préparées en DMSO à partir de solution stock à 10 mM. Les produits de l'invention, aux concentrations finales de 0,003 à 10 µM, sont incubés à 37°C avec le substrat FS1 (concentration finale de 5 µM) et l'enzyme purifiée (concentration finale de 10 nM), dans du tampon acétate de sodium (0,1 M) pH 4.5 contenant 0,02% de détergent CHAPS et 200 mM NaCl pendant 45 minutes. Le pourcentage final de DMSO n'excède pas 7%. Lorsque l'incubation est terminée, la fluorescence est mesurée dans un spectrofluorimètre, aux longueurs d'onde d'excitation de 355 nM et d'émission de 509 nM. Pour chaque concentration de produit testée, le signal fluorescent est comparé au signal maximal obtenu lorsque le substrat FS1 est uniquement incubé avec l'enzyme.

L'activité inhibitrice des produits de l'invention est alors évaluée par la mesure de CI50 (concentration de produit donnant 50% d'inhibition de l'activité enzymatique) à l'aide d'une analyse par régression non-linéaire (applicatif informatique Xlfit, IDBS™).

Les CI₅₀ sont comprises entre 0,1 et 5 µM.

Par exemple, les composés n°5, 7 ,9 et 11 ont montré une CI₅₀ de respectivement 0,31 ; 0,48 ; 0,37 et 1,40 µM.

II apparaît donc que les composés selon l'invention ont une activité inhibitrice vis-à-vis de l'activité de la β-secrétase.

Les composés selon l'invention peuvent donc être utilisés pour la préparation de médicaments, en particulier de médicaments inhibiteurs de la production d'Aβ.

Ainsi, selon un autre de ses aspects, l'invention a pour objet des médicaments qui comprennent un composé de formule (1), ou un sel d'addition de ce dernier à un acide pharmaceutiquement acceptable, ou encore un hydrate ou un solvate du composé de formule (I).

Ces médicaments trouvent leur emploi en thérapeutique, notamment dans le traitement et la prévention des maladies associées à la production de peptide Aβ, parmi lesquelles on peut citer les maladies neurodégénératives telles que la maladie d'Alzheimer, la maladie de Parkinson, la maladie d'Huntington, la maladie de Creutzfeld-Jacob, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, la sclérose latérale amyotrophique, la sclérose multiple, les neuropathies périphériques, les neuropathies diabétiques, la migraine, les troubles de l'humeur, la dépression, l'anxiété, les désordres vasculaires tels que l'athérosclérose, l'ischémie cérébro-vasculaire, les tumeurs et les troubles de la prolifération cellulaire.

Ces médicaments trouvent en particulier leur emploi dans le traitement et la prévention des maladies neurodégénératives telles que la maladie d'Alzheimer, la maladie de Parkinson, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, l'ischémie cérébro-vasculaire.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maTs. | 15,0 mg |
| Hydroxypropyl-méthylcellulose | 2,25 mg |
| Stéarate de magnésium | 3,0 mg |

Est également décrite une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé répondant à la formule (I) 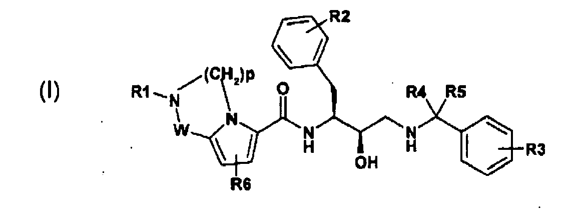 dans laquelle :
R1 représente un atome d'hydrogène, un groupe (C₁-C₁₀)alkyle, (C₃-C₇)cycloalkyle, (CH₂)ₙ-(C₁-C₆)alcényle, (CH₂)ₙ-(C₁-C₆)alcynyle, (C₁-C₆)alkyle-Z-(C₁-C₆)alkyle, dans lequel Z représente un hétèroatome choisi parmi O, N et S(O)ₘ, ou bien R1 représente un groupe COOR, S(O)ₘR, un aryle ou un aralkyle ; les groupes (C₁-C₁₀)alkyle, (C₃-C₇)cycloalkyle, (CH₂)ₙ-(C₁-C₉)alcényle, (CH₂)ₙ-(C₁-C₆)alcynyle, (C₁-C₆)alkyle-Z-(C₁-C₆)alkyle, aryle ou aralkyle étant éventuellement substitués par un ou plusieurs groupes choisis parmi un atome d'halogène, un groupe (C₁-C₆)alkyle, (C₃-C₇)cycloalkyle, halo(C₁-C₆)alkyle, (C₁-C₆)alcoxy, halo(C₁-C₆)alcoxy, NR7R8, nitro, cyano, OR, COOR, C(O)NR7R8, S(O)ₘNR7R8,
R2 représente un ou plusieurs groupes choisis parmi un atome d'hydrogène, un atome d'halogène, un groupe (C₁-C₆)alkyle, (C₃-C₇)cycloalkyle, (C₁-C₈)alcényle, (C₁-C₆)alcynyle, (C₁-C₆)alkyle-Z-(C₁-C₆)alkyle, dans lequel Z représente un hétéroatome choisi parmi O, N et S(O)ₘ, ou bien R2 représente un groupe halo(C₁-C₆)alkyle, halo(C₁-C₆)alcoxy, hydroxy, (C₁-C₆)alcoxy, nitro, cyano, amino, un groupe NR7R8, COOR, C(O)NR7R8, OC(0)(C₁-C₆)alkyle, S(O)ₘ-NR7R8, un groupe aryle, le groupe aryle pouvant être éventuellement substitué par un ou plusieurs groupes choisis parmi un atome d'halogène, un groupe (C₁-C₆)alkyle, (C₃-C₇)cycloalkyle, halo(C₁-C₆)alkyle, (C₁-C₆)alcoxy, halo(C₁-C₆)alcoxy, NR7R8, OR, nitro, cyano, COOR, C(O)NR7R8, S(O)ₘNR7R8,
R3 représente un groupe trifluorométhyle,
R4 et R5 représentent un atome d'hydrogène, ou bien R4 et R5 forment avec l'atome de carbone qui les porte un cycle saturé contenant de 3 à 6 atomes de carbone et contenant éventuellement de 0 à 1 hétéroatome choisi parmi O, N ou S,
R6 représente un groupe choisi parmi un atome d'hydrogène, un atome d'halogène, un groupe (C₁-C₆)alkyle, (C₃-C₇)cycloalkyle, (C₃-C₇)cycloalkyle(C₁-C₆)alkyle, nitro, amino, un groupe NR7R8, COOR, un groupe aryle, un groupe NR7(SO₂)R8 ou C(O)NR7R8,
R, R7 et R8 représentent, indépendamment l'un de l'autre, un ou plusieurs groupes choisis parmi un atome d'hydrogène, un groupe (C₁-C₆)alkyle, (C₃-C₇)cycloalkyle, (C₃-C₇)cycloalkyle(C₁-C₆)alkyle, un groupe aryle, aryle(C₁-C₆)alkylène, ou bien R7 et R8 peuvent former avec l'atome qui les porte un cycle saturé, partiellement insaturé ou insaturé, contenant de 5 à 7 atomes de carbone et contenant éventuellement en plus un hétéroatome choisi parmi O, N ou S(O)ₘ,
W représente un groupe méthylène ou C(O),
m représente un nombre entier pouvant prendre les valeurs 0, 1 ou 2,
n représente un nombre entier pouvant prendre les valeurs 1, 2, 3, 4, 5 ou 6,
p représente un nombre entier pouvant prendre les valeurs 2 ou 3,
le carbone portant le groupe benzyle substitué par R2 est de configuration absolue S,
le carbone portant le groupe hydroxyle est de configuration absolue R, à l'état de base ou de sel d'addition à un acide.

2. Composé de formule (I) selon la revendication 1, **caractérisé en ce que** W représente un groupe méthylène, à l'état de base ou de sel d'addition à un acide.

3. Composé de formule (I) selon l'une des revendications 1 à 2, **caractérisé en ce que** W représente un groupe C(O), à l'état de base ou de sel d'addition à un acide.

4. Composé de formule (I) selon l'une des revendications 1 à 3, **caractérisé en ce que** p représente 2, à l'état de base ou de sel d'addition à un acide.

5. Composé de formule (I) selon l'une des revendications 1 à 3, **caractérisé en ce que** p représente 3, à l'état de base ou de sel d'addition à un acide.

6. Composé de formule (I) selon l'une des revendications 1 à 5, **caractérisé en ce que** R6 représente un groupe choisi parmi un atome d'hydrogène, un groupe COOR ou un groupe C(O)NR7R8, à l'état de base ou de sel d'addition à un acide.

7. Composé de formule (I) selon l'une des revendications 1 à 6, **caractérisé en ce que** R6 représente un groupe choisi parmi un atome d'hydrogène, un groupe COOH, COOMe ou un groupe C(O)N(Et)₂, à l'état de base ou de sel d'addition à un acide,

8. Composé de formule (I) selon l'une des revendications 1 à 7, **caractérisé en ce que**
W représente un groupe méthylène ou C(O),
p représente 2 ou 3,
R1 représente un atome d'hydrogène, un groupe (C₁-C₁₀)alkyle, COOR ou S(O)ₘR, le groupe (C₁-C₁₀)alkyle étant éventuellement substitué par un ou plusieurs groupes (C₁-C₆)alkyle,
R2 représente un ou plusieurs groupes choisis parmi un atome d'hydrogène ou un atome d'halogène,
R4 et R5 représentent un atome d'hydrogène ou forment avec l'atome de carbone qui les porte un groupe cyclopropyle,
R6 représente un groupe choisi parmi un atome d'hydrogène, un groupe COOR ou un groupe C(O)NR7R8,
R, R7 et R8 représentent, indépendamment l'un de l'autre, un atome d'hydrogène ou un ou plusieurs groupes (C₁-C₆)alkyle, à l'état de base ou de sel d'addition à un acide.

9. Composé de formule (I) selon l'une des revendications 1 à 8, **caractérisé en ce qu'**il est choisi parmi :
• 6-{[(1*S*,2*R*)-1-benzyl-2-hydroxy-3-({1-[3-(trifluorométhyl)phényl]cyclopropyl}amino) propyl]carbamoyl}-3,4-dihydropyrrolo[1,2-a]pyrazine-2,8(1*H*)-dicarboxylate de 2-*tert*-butyle et de 8-méthyle
• Acide 6-{[(1*S*,2*R*)-1-benzyl-2-hydroxy-3-({1-[3-(trifluorométhyl)phényl]cyclopropyl} amino)propyl]carbamoyl}-2-(*tert*-butoxycarbonyl)-1,2,3,4-tétrahydropyrrolo[1,2-*a*] pyrazine-8-carboxylique
• Chlorhydrate (2:1) d'acide 6-{[(1S,2R)-1-benzyl-2-hydroxy-3-({1-[3-(trifluorométhyl) phényl]cyclopropyl}amino)propyl]carbamoyl}-1,2,3,4-tétrahydropyrrolo [1,2-*a*]pyrazine-8-carboxylique
• 6-{[(1*S*,2*R*)-1-benzyl-2-hydroxy-3-({1-[3-(trifluorométhyl)phényl] cyclopropyl}amino) propyl]carbamoyl}-8-(diéthylcarbamoyl)-3,4-dihydropyrrolo[1,2-*a*]pyrazine-2(1*H*)-carboxylate de *tert*-butyle
• 6-{(1S,2R)-1-benzyl-2-hydroxy-3-[1-(3-trifluorométhylphényl)-cyclopropylamino]-propyl}-*N-*8,*N*-8-diéthyl-1,2,3,4-tétrahydropyrrolo[1,2-a]pyrazine-6,8-dicarboxamide et son chlorhydrate (2:1)
• 6-{(1S,2R)-1-benzyl-2-hydroxy-3-[1-(3-trifluorométhylphényl)-cyclopropylamino]-propyl}-*N-*8,*N*-8-diethyl-2-(méthylsulfonyl)-1',2,3,4-tétrahydropyrrolo[1,2-*a*]pyrazine-6,8-dicarboxamide
• *N*-[(1*S*,2*R*)-1-(3,5-difluorobenzyl)-2-hydroxy-3-({1-[3-(trfluorométhyl)phényl]cyclopropyl} amino)propyl]-1-oxo-2-(1-propylbutyl)-1,2,3,4-tétrahydropyrrolo[1,2-*a*]pyrazine-6-carboxamide et son chlorhydrate (1 : 1)
• *N*-[(1*S*,2*R*)-1-benzyl-2-hydroxy-3-({1-[3-(trifluorométhyl)phényl]cyclopropyl}amino) propyl]-1-oxo-2-(1-propylbutyl)-1,2,3,4-tétrahydropyrrolo[1,2-*a*]pyrazine-6-carboxamide et son chlorhydrate (1 :1)
• *N*-[(1*S*,2*R*)-1-benzyl-2-hydroxy-3-{[3-(trifluorométhyl)benzyl]amino}propyl]-1-oxo-2-(1-propylbutyl)-1,2,3,4-tétrahydropyrrolo[1,2-*a*]pyrazine-6-carboxamide et son chlorhydrate (1 :1)
• *N*-[(1*S*,2*R*)-1-benzyl-2-hydroxy-3-{[3-(trifluorométhyl)benzyl]amino}propyl]-2-(1-éthyl propyl)-1-oxo-1,2,3,4-tétrahydropyrrolo[1,2-*a*]pyrazine-6-carboxamide et son chlorhydrate (1 :1).
• *N*-[(1S,2R)-1-benzyl-2-hydroxy-3-{[3-(trifluorométhyl)benzyl]amino}propyl]-1-oxo-2-(1-propylbutyl)-2,3,4,5-tétrahydro-1H-pyrrolo[1,2-a][1,4]diazépine-7-carboxamide et son chlorhydrate (1 :1).

10. Composé de formule (IIIa) 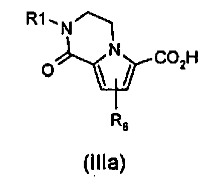 dans laquelle R1 et R6 sont tels que définis dans la formule générale (I) selon la revendication 1.

11. Composé de formule (IIIb) 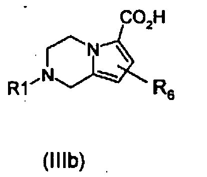 dans laquelle R1 et R6 sont tels que définis dans la formule générale (I) selon la revendication 1.

12. Composé selon la revendication 11, **caractérisé en ce que** le composé de formule (IIIb) est l'acide 2-(*tert*-butoxycarbonyl)-8-(méthoxycarbonyl)-1,2,3,4-tétra hydropyrrolo [1,2-*a*]pyrazine-6-carboxylique.

13. Composé de formule (IIIc) 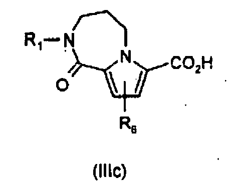 dans laquelle R1 et R6 sont tels que définis dans la formule générale (I) selon la revendication 1.

14. Médicament, **caractérisé en ce qu'**il comprend un composé de formule (I) selon l'une quelconque des revendications 1 à 9, ou un sel d'addition de ce composé, à un acide pharmaceutiquement acceptable.

15. Composition pharmaceutique, **caractérisée en ce qu'**elle comprend un composé de formule (I) selon l'une quelconque des revendications 1 à 9, ou un sel pharmaceutiquement acceptable, ainsi qu'au moins un excipient pharmaceutiquement acceptable.

16. Utilisation d'un composé de formule (I) selon l'une quelconque des revendications 1 à 9 pour la préparation d'un médicament destiné au traitement et à la prévention de la maladie d'Alzheimer, la maladie de Parkinson, la maladie d'Huntington, la maladie de Creutzfeld-Jacob, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, la sclérose latérale amyotrophique, la sclérose multiple, les neuropathies périphériques, les neuropathies diabétiques, la migraine, les troubles de l'humeur, la dépression, l'anxiété, les désordres vasculaires tels que l'athérosclérose, l'ischémie cérébro-vasculaire, les tumeurs et les troubles de la prolifération cellulaire.

17. Utilisation d'un composé de formule (I) selon l'une quelconque des revendications 1 à 9 pour la préparation d'un médicament destiné au traitement et à la prévention de la maladie d'Alzheimer, la maladie de Parkinson, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, l'ischémie cérébro-vasculaire.

18. Composé de formule (I) selon l'une quelconque des revendications 1 à 9, à l'état de base, pour la prévention ou le traitement de la maladie d'Alzheimer, la maladie de Parkinson, la maladie d'Huntington, la maladie de Creutzfeld-Jacob, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, la sclérose latérale amyotrophique, la sclérose multiple, les neuropathies périphériques, les neuropathies diabétiques, la migraine, les troubles de l'humeur, la dépression, l'anxiété, les désordres vasculaires tels que l'athérosclérose, l'ischémie cérébro-vasculaire, les tumeurs et les troubles de la prolifération cellulaire.

19. Composé de formule (I) selon l'une quelconque des revendications 1 à 9, à l'état de base, pour la préparation d'un médicament destiné au traitement et à la prévention de la maladie d'Alzheimer, la maladie de Parkinson, le syndrome de Down, la démence à corps de Lewy, la démence sénile, la démence fronto-temporale, l'amyloïdose cérébrale et systémique, les troubles cognitifs légers, l'angiopathie cérébrale amyloïde, les troubles primaires et secondaires de la mémoire, l'ischémie cérébro-vasculaire.

## Claims

1. Compound corresponding to the formula (I) 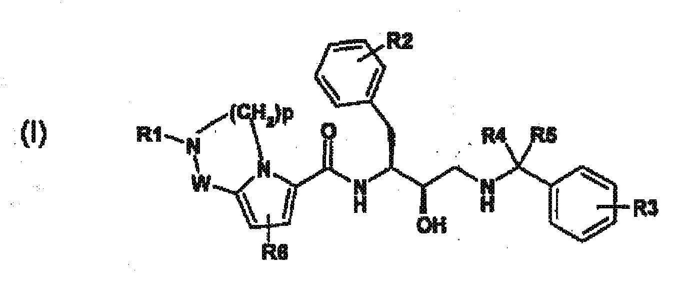 in which:
R1 represents a hydrogen atom, a (C₁-C₁₀) alkyl, (C₃-C₇) cycloalkyl, (CH₂)ₙ- (C₁-C₆) alkenyl, (CH₂)ₙ- (C₁-C₆)alkynyl or (C₁-C₆)alkyl-Z-(C₁-C₆)alkyl group, in which Z represents a heteroatom chosen from O, N and S(O)ₘ, or else R1 represents a COLOR, S(O)ₘR, aryl or aralkyl group; (C₁-C₁₀) alkyl, (C₃-C₇) cycloalkyl, (CH₂)ₙ- (C₁-C₆) alkenyl, (CH₂)ₙ-(C₁-C₆)alkynyl; (C₁-C₆) alkyl-Z- (C₁-C₆)alkyl, aryl or aralkyl groups being optionally substituted with one or more groups chosen from a halogen atom, a (C₁-C₆) alkyl, (C₃-C₇)cycloalkyl, halo(C₁-C₆)alkyl, (C₁-C₆)alkoxy, halo(C₁-C₆)alkoxy, NR7R8, nitro, cyano, OR, COOR, C(O)NR7R8 or S(O)ₘNR7R8 group;
R2 represents one or more groups chosen from a hydrogen atom, a halogen atom, a (C₁-C₆) alkyl, (C₃-C₇)cycloalkyl, (C₁-C₆) alkenyl, (C₁-C₆)alkynyl or (C₁-C₆)alkyl-Z-(C₁-C₆) alkyl group, in which Z represents a heteroatom chosen from O, N and S(O)ₘ, or else R² represents a halo (C₁-C₆) alkyl, halo (C₁-C₆) alkoxy, hydroxy, (C₁-C₆)alkoxy, nitro, cyano or amino group, an NR7R8, COOR, C(O)NR7R8, O-C(O)(C₁-C₆)alkyl or S(O)ₘ-NR7R8 group, or an aryl group, the aryl group possibly being optionally substituted with one or more groups chosen from a halogen atom, (C₁-C₆)alkyl, (C₃-C₇)cycloalkyl, halo (C₁-C₆)alkyl, (C₁-C₆)alkoxy, halo(C₁-C₆)alkoxy, NR7R8, OR, nitro, cyano, COOR, C(O)NR7R8 or S(O)ₘNR7R8 group;
R3 represents a trifluoromethyl group;
R4 and R5 represent a hydrogen atom, or else R4 and R5 form, with the carbon atom that bears them, a saturated ring containing from 3 to 6 carbon atoms and optionally containing from 0 to 1 heteroatom, chosen from O, N or S;
R6 represents a group chosen from a hydrogen atom, a halogen atom, a (C₁-C₆)alkyl, (C₃-C₇) cycloalkyl, (C₃-C₇)cycloalkyl(C₁-C₆)alkyl, nitro or amino group, an NR7R8 or COOR group, an aryl group, or an NR7(SO₂)R8 or C(O)NR7R8 group;
R, R7 and R8 represent, independently of one another, one or more groups chosen from a hydrogen atom, a (C₁-C₆)alkyl, (C₃-C₇) cycloalkyl or (C₃-C₇) cycloalkyl (C₁-C₆)alkyl group, an aryl or aryl(C₁-C₆)alkylene group, or else R7 and R8 may form, with the atom which bears therm, a saturated, partially unsaturated or unsaturated ring containing from 5 to 7 carbon atoms and optionally containing; in addition, a heteroatom chosen from O, N or S(O)_{m;}
W represents a methylene or C(O) group;
m represents an integer which may take the values 0, 1 or 2;
n represents an integer which may take the values 1, 2, 3, 4, 5 or 6;
p represents an integer which may take the values 2 or 3;
the carbon bearing the benzyl group substituted by R2 is of S absolute configuration; and
the carbon bearing the hydroxyl group is of R absolute configuration, in the form of a base or addition salt with an acid.

2. Compound of formula (I) according to Claim 1, **characterized in that** W represents a methylene group, in the form of a base or addition salt with an acid.

3. Compound of formula (I) according to either of Claims 1 and 2, **characterized in that** W represents a C(O) group, in the form of a base or addition salt with an acid.

4. Compound of formula (I) according to one of Claims 1 to 3, **characterized in that** p represents 2, in the form of a base or addition salt with an acid.

5. Compound of formula (I) according to one of Claims 1 to 3, **characterized in that** p represents 3, in the form of a base or addition salt with an acid.

6. Compound of formula (I) according to one of Claims 1 to 5, **characterized in that** R6 represents a group chosen from a hydrogen atom, a COOR group or a C(O)NR7R8 group, in the form of a base or addition salt with an acid.

7. Compound of formula (I) according to one of Claims 1 to 6, **characterized in that** R6 represents a group chosen from a hydrogen atom, a COOH or COOMe group or a C(O)N(Et)₂ group, in the form of a base or addition salt with an acid.

8. Compound of formula (I) according to one of Claims 1 to 7, **characterized in that**:
W represents a methylene or C(O) group;
p represents 2 or 3;
R1 represents a hydrogen atom, a (C₁-C₁₀)alkyl, COOR or S(O)ₘR group, the (C₁-C₁₀)alkyl group being optionally substituted with one or more (C₁-C₆)alkyl groups;
R2 represents one or more groups chosen from a hydrogen atom or a halogen atom;
R4 and R5 represent a hydrogen atom or form, with the carbon atom which bears them, a cyclopropyl group;
R6 represents a group chosen from a hydrogen atom, a COOR group or a C(O)NR7R8 group; and
R, R7 and R8 represent, independently of one another, a hydrogen atom or one or more (C₁-C₆)alkyl groups, in the form of a base or addition salt with an acid.

9. Compound of formula (I) according to one of Claims 1 to 8, **characterized in that** it is chosen from:
• 2-*tert*-butyl 8-methyl 6-{[(1*S*,2*R*)-1-benzyl-2-hydroxy-3-({1-[3-(trifluoromethyl)phenyl]cyclopropyl}amino)propyl]carbamoyl}-3,4-dihydropyrrolo-[1,2-*a*]pyrazine-2,8(1*H*)-dicarboxylate;
• 6-{[(1*S*,2*R*)-1-benzyl-2-hydroxy-3-({1-[3-(trifluoromethyl)phenyl]cyclopropyl}amino)propyl]carbamoyl}-2-(*tert*-butoxycarbonyl)-1,2,3,4-tetrahydropyrrolo[1,2-*a*]pyrazine-8-carboxylic acid;
• 6-{[(1S,2R)-1-benzyl-2-hydroxy-3-({1-[3-(trifluoromethyl)phenyl]cyclopropyl}amino)propyl]carbamoyl}-1,2,3,4-tetrahydropyrrolo[1,2-*a*]pyrazine-8-carboxylic acid hydrochloride (2:1);
• *tert*-butyl 6-{[(1*S*,2*R*)-1-benzyl-2-hydroxy-3-({1-[3-(trifluoromethyl)phenyl]cyclopropyl}amino)propyl]-carbamoyl}-8-(diethylcarbamoyl)-3,4-dihydropyrrolo-[1,2-*a*]pyrazine-2(1*H*)carboxylate;
• 6-{(1S,2R)-1-benzyl-2-hydroxy-3-[1-(3-trifluoromethylphenyl)cyclopropylamino]propyl}-*N*-8,*N*-8-diethyl-1,2,3,4-tetrahydropyrrolo[1,2-a]pyrazine-6,8-dicarboxamide and its hydrochloride (2:1);
• 6-{(1S,2R)-1-benzyl-2-hydroxy-3-[1-(3-trifluoromethylphenyl)cyclopropylamino]propyl}-*N*-8,*N*-8-diethyl-2-(methylsulfonyl)-1,2,3,4-tetrahydropyrrolo-[1,2-*a*]pyrazine-6,8-dicarboxamide;
• *N*-[(1*S*,2*R*)-1-(3,5-difluorobenzyl)-2-hydroxy-3-({1-[3-(trifluoromethyl)phenyl]cyclopropyl}amino)propyl]-1-oxo-2-(1-propylbutyl)-1,2,3,4-tetrahydropyrrolo[1,2-*a*]pyrazine-6-carboxamide and its hydrochloride (1:1);
• *N*-[(1*S*,2*R*)-1-benzyl-2-hydroxy-3-({1-[3-(trifluoromethyl)phenyl]cyclopropyl}amino)propyl]-1-oxo-2-(1-propylbutyl)-1,2,3,4-tetrahydropyrrolo[1,2-*a*]pyrazine-6-carboxamide and its hydrochloride (1:1);
• *N*-[(1*S*,2*R*)-1-benzyl-2-hydroxy-3-{[3-(trifluoromethyl)benzyl]amino}propyl]-1-oxo-2-(1-propylbutyl)-1,2,3,4-tetrahydropyrrolo[1,2-*a*]pyrazine-6-carboxamide and its hydrochloride (1:1);
• *N*-[(1*S*,2*R*)-1-benzyl-2-hydroxy-3-{[3-(trifluoromethyl)benzyl]amino}propyl]-2-(1-ethylpropyl)-1-oxo-1,2,3,4-tetrahydropyrrolo[1,2-*a*]pyrazine-6-carboxamide and its hydrochloride (1:1); and
• *N*-[(1*S*,2*R*)-1-benzyl-2-hydroxy-3-{[3-(trifluoromethyl)benzyl]amino}propyl]-1-oxo-2-(1-propylbutyl)-2,3,4,5-tetrahydro-1H-pyrrolo[1,2-a][1,4]diazepine-7-carboxamide and its hydrochloride (1:1).

10. Compound of formula (IIIa) 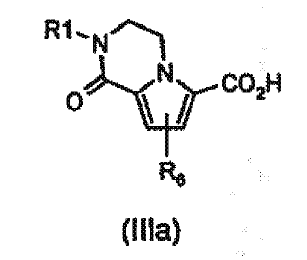 in which R1 and R6 are as defined in the general formula (I) according to Claim 1.

11. Compound of formula (IIIb) 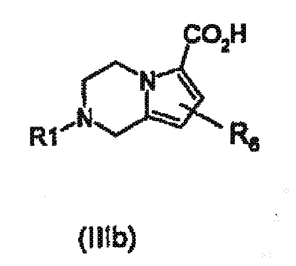 in which R1 and R6 are as defined in the general formula (I) according to Claim 1.

12. Compound according to Claim 11, **characterized in that** the compound of formula (IIIb) is 2-(*tert-*butoxycarbonyl)-8-(methoxycarbonyl)-1,2,3,4-tetrahydropyrrolo[1,2-*a*]pyrazine-6-carboxylic acid.

13. Compound of formula (IIIc) 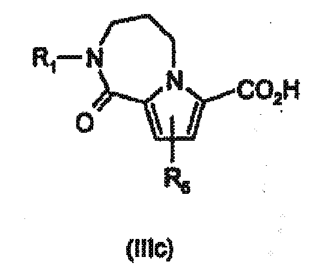 in which R1 and R6 are as defined in the general formula (I) according to Claim 1.

14. Medicament, **characterized in that** it comprises a compound of formula (I) according to any one of Claims 1 to 9, or an addition salt of this compound with a pharmaceutically acceptable acid.

15. Pharmaceutical composition, **characterized in that** it comprises a compound of formula (I) according to any one of Claims 1 to 9, or a pharmaceutically acceptable salt, and also at least one pharmaceutically acceptable excipient.

16. Use of a compound of formula (I) according to any one of Claims 1 to 9 for the preparation of a medicament intended for treating and/or for preventing Alzheimer's disease, Parkinson's disease, Huntington's disease, Creutzfeldt-Jakob disease, Down's syndrome, dementia with Lewy bodies, senile dementia, frontotemporal dementia, cerebral and systemic amyloidosis, mild cognitive impairments, cerebral amyloid angiopathy, primary and secondary memory disorders, amyotrophic literal sclerosis, multiple sclerosis, peripheral neuropathies, diabetic neuropathies, migraine, mood disorders, depression, anxiety, vascular disorders such as atherosclerosis, cerebrovascular ischemia, tumours and cell proliferation disorders.

17. Use of a compound of formula (I) according to any one of Claims 1 to 9 for the preparation of a medicament intended for treating and/or for preventing Alzheimer's disease, Parkinson's disease, Down's syndrome, dementia with Lewy bodies, senile dementia, frontotemporal dementia, cerebral and systemic amyloidosis, mild cognitive impairments, cerebral amyloid angiopathy, primary and secondary memory disorders and cerebrovascular ischemia.

18. Compound of formula (I) according to any one of Claims 1 to 9, in the form of a base, for preventing or treating Alzheimer's disease, Parkinson's disease, Huntington's disease, Creutzfeldt-Jakob disease, Down's syndrome, dementia with Lewy bodies, senile dementia, frontotemporal dementia, cerebral and systemic amyloidosis, mild cognitive impairments, cerebral amyloid angiopathy, primary and secondary memory disorders, amyotrophic lateral sclerosis, multiple sclerosis, peripheral neuropathies, diabetic neuropathies, migraine, mood disorders, depression, anxiety, vascular disorders such as atherosclerosis, cerebrovascular ischemia, tumours and cell proliferation disorders.

19. Compound of formula (I) according to any one of Claims 1 to 9, in the form of a base, for the preparation of a medicament intended for treating and/or for preventing Alzheimer's disease, Parkinson's disease, Down's syndrome, dementia with Lewy bodies, senile dementia, frontotemporal dementia, cerebral and systemic amyloidosis, mild cognitive impairments, cerebral amyloid angiopathy, primary and secondary memory disorders and cerebrovascular ischemia.

## Patentansprüche

1. Verbindung der Formel (I) 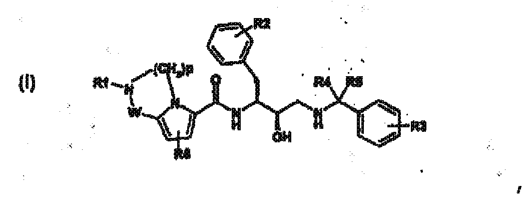 in der:
R1 ein Wasserstoffatom, eine (C₁-C₁₀) Alkyl-, (C₃-C₇)Cycloalkyl-, (CH₂)ₙ- (C₁-C₆)Alkenyl-, (CH₂)ₙ- (C₁-C₆)Alkinyl-, (C₁-C₆)Alkyl-Z-(C₁-C₆)alkylgruppe bedeutet, in der Z ein Heteroatom ausgewählt aus der Gruppe O, N und S(O)ₘ darstellt, oder R1 eine COOR-, S(O)ₘR-, Aryl- oder Aralkylgruppe bedeutet; wobei die (C₁-C₁₀)Alkyl-, (C₃-C₇) Cycloalkyl-, (CH₂)ₙ- (C₁-C₆)Alkenyl-, (CH₂) (C₁-C₆)Alkinyl-, (C₁-C₆)Alkyl-Z-(C₁-C₆)alkyl-, Aryl- oder Aralkylgruppe gegebenenfalls durch eine oder mehrere Gruppen ausgewählt aus der Reihe Halogenatom, (C₁-C₆)Alkyl-, (C₃-C₇)Cycloalkyl-, Halogen-(C₁-C₆)alkyl-, (C₁-C₆)alkoxy-, Halogen-(C₁-C₆)alkoxy, NR7R8-, Nitro-, Cyano-, OR-, COOR-, C(O)NR7R8-, S(O)ₘNR7R8-Gruppe substituiert ist,
R2 eine oder mehrere Gruppen aus der Reihe Wasserstoffatom, Halogenatom, (C₁-C₆)Alkyl-, (C₃-C₇)Cycloalkyl-, (C₁-C₆)Alkenyl-, (C₁-C₆)Alkinyl- (C₁-C₆)Alkyl-Z-(C₁-C₆)alkylgruppe bedeutet, in der Z ein Heteroatom aus der Reihe O, N und S(O)ₘ bedeutet, oder R2 eine Halogen-(C₁-C₆)alkyl-, Halogen-(C₁-C₆)alkoxy-, Hydroxy-, (C₁-C₆)Alkoxy-, Nitro-, Cyano-, Aminogruppe, eine NR7R8-, COOR-, C(O)NR7R8-, O-C(O) (C₁-C₆)Alkyl-, S(O)ₘ-NR7R8-Gruppe, eine Arylgruppe bedeutet, wobei die arylgruppe gegebenenfalls durch eine oder mehrere Gruppen aus der Reihe Halogenatom, (C₁-C₆)Alkyl-, (C₃-C₇)Cycloalkyl-, Halogen-(C₁-C₆)alkyl-, (C₁-C₆)Alkoxy-, Halogen-(C₁-C₆)alkoxy-, NR7R8-, OR-, Nitro-, Cyano-, COOR-, C(O)NR7R8-, S(O)ₘNR7R8-Gruppe substituiert sein kann,
R3 eine Trifluormethylgruppe bedeutet,
R4 und R5 ein Wasserstoffatom bedeuten, oder R4 und R5 mit dem Kohlenstoffatom, das sie trägt, einen gesättigen Cyclus mit 3 bis 6 Kohlenstoffatomen, der gegebenenfalls 0 bis 1 Heteroatom aus der Reihe O, N oder S enthält, bilden,
R6 eine Gruppe aus der Reihe Wasserstoffatom, Halogenatom, (C₁-C₆)Alkyl-, (C₃-C₇) Cycloalkyl-, (C₃-C₇)Cycloalkyl-(C1-C₆)alkyl-, Nitro-, Aminogruppe, eine NR7R8-Gruppe; COOR, eine Arylgruppe, eine NR7-(SO₂)R8-oder C(O)NR7R8-Gruppe bedeutet,
R, R7 und R8 unabhängig voneinander eine oder mehrere Gruppen ausgewählt aus der Reihe Wasserstoffatom, (C₁-C₆)Alkyl-, (C₃-C₇)Cycloalkyl-, (C₃-C₇)Cycloalkyl-(C₁-C₆)alkylgruppe, eine Arylgruppe, eine Aryl-(C₁-C₆)alkylengruppe bedeuten, oder R7 und R8 mit dem Atom, das sie trägt, einen gesättigten, teilweise ungesättigten oder ungesättigten Cyclus, der 5 bis 7 Kohlenstoffatome enthält und der gegebenenfalls weiterhin ein Heteroatom aus der Reihe O, N oder S(O)ₘ enthält, bilden können,
W eine Methylen- oder C(O)-Gruppe bedeutet,
m eine ganze Zahl, die die Werte 0, 1 oder 2 annehmen kann, bedeutet,
n eine ganze Zahl, die die Werte 1, 2, 3, 4, 5 oder 6 annehmen kann, bedeutet,
p eine ganze Zahl, die die Werte 2 oder 3 annehmen kann, bedeutet,
wobei der Kohlenstoff, der die durch R2 substituierte Benzylgruppe trägt, die absolute S-Konfiguration aufweiset,
wobei der Kohlenstoff, der die Hydroxylgruppe trägt, die absolute R-Konfiguration aufweist, als Base oder als Säureadditionssalz.

2. Verbindung der Formel (I) nach Anspruch 1, **dadurch gekennzeichnet, dass** W eine Methylengruppe bedeutet, als Base oder als Säureadditionssalz.

3. Verbindung der Formel (I) nach einem der Ansprüche 1 bis 2, **dadurch** gekenntzeichnet, dass W eine C(O)-Gruppe bedeutet, als Base oder als Säureadditionssalz.

4. Verbindung der Formel (I) nach einem der Ansprüche 1 bis 3, **dadurch gekennzeichnet, dass** p 2 bedeutet, als Base oder als Säureadditionssalz.

5. Verbindung der Formel (I) nach einem der Ansprüche 1 bis 3, **dadurch gekennzeichnet, dass** p 3 bedeutet, als Base oder als Säureadditionssalz.

6. Verbindung der Formel (I) nach einem der Ansprüche 1 bis 5, **dadurch gekennzeichnet, dass** R6 eine Gruppe ausgewählt aus einem Wasserstoffatom, einer COOR-Gruppe oder einer C(O)NR7R8-Gruppe bedeutet, als Base oder als Säureadditionssalz.

7. Verbindung der Formel (I) nach einem der Ansprüche 1 bis 6, **dadurch gekennzeichnet, dass** R6 eine Gruppe ausgewählt aus einem Wasserstoffatom, einer COOH-, COOMe- oder C(O)N(Et)₂-Gruppe bedeutet, als Base oder als Säureadditionssalz.

8. Verbindung der Formel (I) nach einem der Ansprüche 1 bis 7, **dadurch gekennzeichnet, dass**
W eine Methylengruppe oder C(O)-Gruppe bedeutet,
p 2 oder 3 bedeutet,
R1 ein Wasserstoffatom, eine (C₁-C₁₀)Alkyl-, COOR- oder S(O)ₘR-Gruppe bedeutet, wobei die (C₁-C₁₀)Alkylgruppe gegebenenfalls durch eine oder mehrere (C₁-C₆)Alkylgruppen substituiert ist,
R2 eine oder mehrere Gruppen aus der Reihe Wasserstoffatom oder Halogenatom bedeutet,
R4 und R5 ein Wasserstoffatom bedeuten oder mit dem Kohlenstoffatom, das sie trägt, eine Cyclopropylgruppe bilden,
R6 eine Gruppe ausgewählt aus einem Wasserstoffatom, einer COOR-Gruppe oder einer C(O)NR7R8-Gruppe bedeutet,
R, R7 und R8 unabhängig voneinander ein Wasserstoffatom oder eine oder mehrere (C₁-C₆)Alkygruppen bedeuten, als Base oder als Säureadditionssalz.

9. Verbindung der Formel (I) nach einem der Ansprüche 1 bis 8, **dadurch gekennzeichnet, dass** sie aus Folgendem ausgewählt ist:
• 2-tert-Butyl- und 8-Methyl-6-{[(1*S*,2*R*)-1-benzyl-2-hydroxy-3-({1-[3-(trifluormethyl)phenyl]cyclopropyl}amino)propyl]carbamoyl)-3,4-dihydropyrrolo[1,2-*a*]pyrazin-2,8(1H)-dicarboxylat
• 6-{[(1*S*,2*R*)-1-Benzyl-2-hydroxy-3-({1-[3-(trifluormethyl)phenyl]cyclopropyl}amino)propyl]-carbamoyl)-2-(tert.-butoxycarbonyl)-1,2,3,4-tetrahydropyrrolo[1,2-*a*]pyrazin-8-carbonsäure
• 6-{[(1*S*,2*R*)-1-Benzyl-2-hydroxy-3-({1-[3-(trifluormethyl)phenyl]cyclopropyl}amino)propyl]-carbamoyl)-1,2,3,4-tetrahydropyrrolo[1,2-*a*]pyrazin-8-carbonsäure-hydrochlorid (2:1)
• tert-Butyl-6-{[(1*S*,2*R*)-1-benzyl-2-hydroxy-3-({1-[3-(trifluormethyl)phenyl]cyclopropyl}amino)propyl]carbamoyl)-8-(diethylcarbamoyl)-3,4-dihydropyrrolo[1,2-*a*]pyrazin-2(1*H*)-carboxylat
• 6-{[(1*S*,2*R*)-1-Benzyl-2-hydroxy-3-[1-(3-trifluormethylphenyl)cyclopropylamino]propyl)-*N-*8,*N*-8-diethyl-1,2,3,4-tetrahydropyrrolo[1,2-*a*]pyrazin-6,8-dicarboxamid und sein Hydrochlorid (2:1)
• 6-{[(1*S*,2*R*)-1-Benzyl-2-hydroxy-3-[1-(3-trifluormethylphenyl)cyclopropylamino]propyl)-*N-*8,*N*-8-diethyl-2-(methylsulfonyl)-1,2,3,4-tetrahydropyrrolo[1,2-*a*]pyrazin-6,8-dicarboxamid
• *N*-[(1*S*,2*R*)-1-(3,5-Difluorbenzyl)-2-hydroxy-3-({1-[3-(trifluormethyl)phenyl]cyclopropyl}amino)propyl]-1-oxo-2-(1-propylbutyl)-1,2,3,4-tetrahydropyrrolo[1,2-*a*]pyrazin-6-carboxamid und sein Hydrochlorid (1:1)
• *N*-[(1*S*,2*R*)-1-Benzyl-2-hydroxy-3-({1-[3-(trifluormethyl)phenyl]cyclopropyl}amino)propyl]-1-oxo-2-(1-propylbutyl)-1,2,3,4-tetrahydropyrrolo[1,2-*a*]pyrazin-6-carboxamid und sein Hydrochlorid (1:1)
• *N*-[(1*S*,2*R*)-1-Benzyl-2-hydroxy-3-{[3-(trifluormethyl)benzyl]amino}propyl]-1-oxo-2-(1-propylbutyl)-1,2,3,4-tetrahydropyrrolo[1,2-a]pyrazin-6-carboxamid und sein Hydrochlorid (1:1)
• *N*-[(1*S*,2*R*)-1-Benzyl-2-hydroxy-3-{[3-(trifluormethyl)benzyl]amino}propyl]-2-(1-ethylpropyl)-1-oxo-1,2,3,4-tetrahydropyrrolo[1,2-a]pyrazin-6-carboxamid und sein Hydrochlorid (1:1)
• *N*-[(1*S*,2*R*)-1-Benzyl-2-hydroxy-3-{[3-(trifluoromethyl)benzyl]amino}propyl]-1-oxo-2-(1-propylbutyl)-2,3,4,5-tetrahydro-1H-pyrrolo[1,2-*a*] [1,4]diazepin-7-carboxamid und sein Hydrochlorid (1:1).

10. Verbindung der Formel (IIIa) 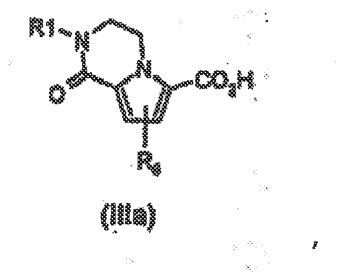 in der R1 und R6 wie in der allgemeinen Formel (I) nach Anspruch 1 definiert sind.

11. Verbindung der Formel (IIIb) 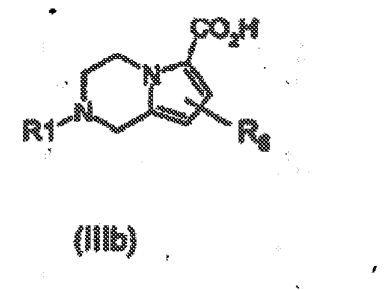 in der R1 und R6 wie in der allgemeinen Formel (I) nach Anspruch 1 definiert sind;

12. Verbindung nach Anspruch 11, **dadurch gekennzeichnet, dass** es sich bei der Verbindung der Formel (IIIb) um 2-(tert.-Butoxycarbonyl)-8-(methoxycarbonyl)-1,2,3,4-tetrahydropyrrolo[1,2-*a*]pyrazin-6-carbonsäure handelt.

13. Verbindung der Formel (IIIc) 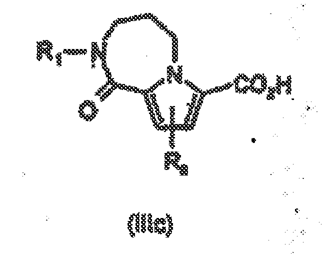 in der R1 und R6 wie in der allgemeinen Formel (I) nach Anspruch 1 definiert sind.

14. Arzneimittel, **dadurch gekennzeichnet, dass** es eine Vebindung der Formel (I) nach einem der Ansprüche 1 bis 9 oder ein Additionssalz dieser Verbindung mit einer pharmazeutisch unbedenklichen Säure umfasst.

15. Pharmazeutische Zusammensetzung, **dadurch gekennzeichnet, dass** sie eine Verbindung der Formel (I) nach einem der Ansprüche 1 bis 9 oder ein pharmazeutisch unbedenkliches Salz sowie mindestens einen pharmazeutisch unbedenklichen Grundstoff umfasst,

16. Verwendung einer Verbindung der Formel (I) nach einem der Ansprüche 1 bis 9 für die Herstellung eines Arzneimittels zur Behandlung und Vorbeugung von Morbus Alzheimer, Morbus Parkinson, Huntington-Chorea, Creutzfeld-Jacob-Krankheit, Down-Syndrom, Lewy-Körperchen-Demenz, Altersdemenz, frontotemporaler Demenz, cerebraler und systemischer Amyloidose, leichten kognitiven Beschwerden, cerebraler Amyloidangiopathie, primären und sekundären Gedächtnisbeschwerden, amyotropher Lateralsklerose, multipler Sklerose, peripheren Neuropathien, Diabetes-Neuropathien, Migräne, Stimmungaproblemen, Depression, Angstzuständen, Gefäßerkrankungen wie Atherosklerose, cerebrovaskulärer Ischämie, Tumoren und Zellproliferationserkrankungen.

17. Verwendung einer Verbindung der Formel (I) nach einem der Ansprüche 1 bis 9 für die Herstellung eines Medikaments zur Behandlung und Vorbeugung von Morbus Alzheimer, Morbus Parkinson, Down-Syndrom, Lewy-Körperchen-Demenz, Altersdemenz, frontotemporaler Demenz, cerebraler und systemischer Amyloidose, leichten kognitiven Störungen, amyloider Cerebralangibpathie, primären und sekundären Gedächtnisstörungen, cerebrovaskulärer Ischämie.

18. Verbindung der Formel (I) nach einem der Ansprüche 1 bis 9 als Base, zur Vorbeugung oder Behandlung von Morbus Alzheimer, Morbus Parkinson, Huntington-Chorea, Creützfeld-Jacob-Krankheit, Down-Syndrom, Lewy-Körperchen-Demenz, Altersdemenz, frontotemporaler Demenz, cerebraler und systemischer Amyloidose, leichten kognitiven Beschwerden, cerebraler Amylöidangiopathie, primären und sekundären Gedächtnisbeschwerden, amyotropher Lateralsklerose, multipler Sklerose, peripheren Neuropathien, Diabetes-Neuropathien, Migräne, Stimmungsproblemen, Depression, Angstzuständen, Gefäßerkrankungen wie Atherosklerose, cerebrovaskulärer Ischämie, Tumoren und Zellproliferationserkrankungen.

19. Verbindung der Formel (I) nach einem der Ansprüche 1 bis 9 als Base, zur Vorbeugung oder Behandlung von Morbus Alzheimer, Morbus Parkinson, Down-Syndrom, Lewy-Körperchen-Demenz, Altersdemenz, frontotemporaler Demenz, cerebraler und systemischer Amyloidose, leichten kognitiven Störungen, amyloider Cerebralangiopathie, primären und sekundären Gedächtnisstörungen, cerebrovaskulärer Ischämie.
